# EUROPEAN PATENT APPLICATION

(11) **EP 4 019 634 A2**
(43) Date of publication of application: **29.06.2022**
(21) Application number: 20852474.4
(22) Date of filing: 12.08.2020
(51) Int. Cl.: C12N 15/113, A61K 48/00, A61P 35/00

(54) **COMPOUNDS AND METHODS FOR TREATING CANCER**

(30) Priority: 13.08.2019 ES 201930743
(71) Applicant: Universidade de Santiago de Compostela, 15782 Santiago de Compostela, La Coruña (ES); Servizo Galego De Saúde, 15703 Santiago de Compostela, A Coruna (ES)
(72) Inventor: ÁLVAREZ VILLAMARÍN, Clara, 15782 Santiago de Compostela, A Coruña (ES); SOUSA RODRIGUES, Joana, 15782 Santiago de Compostela, A Coruña (ES); CAMESELLE TEIJEIRO, José Manuel, 15707 Santiago de Compostela, A Coruña (ES)
(74) Representative: ABG Intellectual Property Law, S.L.
(86) International application number: PCT/ES2020/070512
(87) International publication number: WO 2021/028610

(57) **Abstract**

The invention relates to compounds and compositions capable of silencing, in a specific manner, the mRNA of isoform beta of the PIAS2 protein, as well as to the use thereof for the treatment of cancer, and particularly anaplastic carcinoma of the thyroid.

SEQUENCE LISTING

<110>  SERVIZO GALEGO DE SAUDE (SERGAS)
       UNIVERSIDADE DE SANTIAGO DE COMPOSTELA

<120>  COMPUESTOS Y MÉTODOS PARA EL TRATAMIENTO DEL CANCER

<130>  P18164ES00

<150>  P201930743
<151>  2019-08-13

<160>  100

<170>  PatentIn version 3.5

<210>  1
<211>  21
<212>  RNA
<213>  Homo sapiens

<400>  1
aauaauuugg uauuuauuga a                                                21


<210>  2
<211>  21
<212>  RNA
<213>  Homo sapiens

<400>  2
aacaagaacc aaaugacaug c                                                21


<210>  3
<211>  21
<212>  RNA
<213>  Homo sapiens

<400>  3
aauuugguau uuauugaaag u                                                21


<210>  4
<211>  21
<212>  RNA
<213>  Homo sapiens

<400>  4
aaccauaacu guaucuuccu g                                                21


<210>  5
<211>  21
<212>  RNA
<213>  Homo sapiens

<400>  5
aauggaugga gucuacaaau c                                                21


<210>  6
<211>  19

<212>  RNA
<213>  Artificial Sequence

<220>
<223>  hebra antisentido dsRNAi de señalización de  PIAS2b

<400>  6
uucaauaaau accaaauua                                                    19


<210>  7
<211>  19
<212>  RNA
<213>  Artificial Sequence

<220>
<223>  hebra antisentido dsRNAi de señalización de  PIAS2b

<400>  7
gcaugucauu ugguucuug                                                    19


<210>  8
<211>  19
<212>  RNA
<213>  Artificial Sequence

<220>
<223>  hebra antisentido dsRNAi de señalización de PIAS2b

<400>  8
acuuucaaua aauaccaaa                                                    19


<210>  9
<211>  19
<212>  RNA
<213>  Artificial Sequence

<220>
<223>  hebra antisentido dsRNAi de señalización de  PIAS2b

<400>  9
caggaagaua caguuaugg                                                    19


<210>  10
<211>  19
<212>  RNA
<213>  Artificial Sequence

<220>
<223>  hebra antisentido dsRNAi de señalización de PIAS2b

<400>  10
gauuuguaga cuccaucca                                                    19


<210>  11
<211>  19
<212>  RNA
<213>  Artificial Sequence

<220>
<223>  hebra sentido dsRNAide señalización de PIAS2b

<400>  11
uaauuuggua uuuauugaa                                                    19


<210>  12
<211>  19
<212>  RNA
<213>  Artificial Sequence

<220>
<223>  hebra sentido dsRNAide señalización de PIAS2b

<400>  12
caagaaccaa augacaugc                                                    19


<210>  13
<211>  19
<212>  RNA
<213>  Artificial Sequence

<220>
<223>  hebra sentido dsRNAide señalización de PIAS2b

<400>  13
uuugguauuu auugaaagu                                                    19


<210>  14
<211>  19
<212>  RNA
<213>  Artificial Sequence

<220>
<223>  hebra sentido dsRNAide señalización de PIAS2b

<400>  14
ccauaacugu aucuuccug                                                    19


<210>  15
<211>  19
<212>  RNA
<213>  Artificial Sequence

<220>
<223>  hebra sentido dsRNAide señalización de PIAS2b

<400>  15
uggauggagu cuacaaauc                                                    19


<210>  16
<211>  19
<212>  DNA
<213>  Artificial Sequence

<220>
<223>  DNA hebra antisentido dsRNAi de señalización de PIAS2b

<400>  16
ttcaataaat accaaatta                                                    19


<210>  17
<211>  19
<212>  DNA
<213>  Artificial Sequence

<220>
<223>  DNA hebra antisentido dsRNAi de señalización de PIAS2b

<400>  17
gcatgtcatt tggttcttg                                                    19


<210>  18
<211>  19
<212>  DNA
<213>  Artificial Sequence

<220>
<223>  DNA hebra antisentido dsRNAi de señalización de PIAS2b

<400>  18
actttcaata aataccaaa                                                    19


<210>  19
<211>  19
<212>  DNA
<213>  Artificial Sequence

<220>
<223>  DNA hebra antisentido dsRNAi de señalización de PIAS2b

<400>  19
caggaagata cagttatgg                                                    19


<210>  20
<211>  19
<212>  DNA
<213>  Artificial Sequence

<220>
<223>  DNA hebra antisentido dsRNAi de señalización de PIAS2b

<400>  20
gatttgtaga ctccatcca                                                    19


<210>  21
<211>  19
<212>  DNA
<213>  Artificial Sequence

<220>
<223>  DNA hebra sentido dsRNAide señalización de PIAS2b

<400>  21
taatttggta tttattgaa                                                    19

<210>  22
<211>  19
<212>  DNA
<213>  Artificial Sequence

<220>
<223>  DNA hebra sentido dsRNAide señalización de PIAS2b

<400>  22
caagaaccaa atgacatgc                                                    19


<210>  23
<211>  19
<212>  DNA
<213>  Artificial Sequence

<220>
<223>  DNA hebra sentido dsRNAide señalización de PIAS2b

<400>  23
tttggtattt attgaaagt                                                    19


<210>  24
<211>  19
<212>  DNA
<213>  Artificial Sequence

<220>
<223>  DNA hebra sentido dsRNAide señalización de PIAS2b

<400>  24
ccataactgt atcttcctg                                                    19


<210>  25
<211>  19
<212>  DNA
<213>  Artificial Sequence

<220>
<223>  DNA hebra sentido dsRNAide señalización de PIAS2b

<400>  25
tggatggagt ctacaaatc                                                    19


<210>  26
<211>  21
<212>  RNA
<213>  Artificial Sequence

<220>
<223>  hebra antisentido dsRNAide señalización de PIAS2b

<400>  26
uucaauaaau accaaauuau u                                                 21


<210>  27
<211>  21

<212>  RNA
<213>  Artificial Sequence

<220>
<223>  hebra antisentido dsRNAide señalización de PIAS2b

<400>  27
gcaugucauu ugguucuugu u                                                21


<210>  28
<211>  21
<212>  RNA
<213>  Artificial Sequence

<220>
<223>  hebra antisentido dsRNAide señalización de PIAS2b

<400>  28
acuuucaaua aauaccaaau u                                                21


<210>  29
<211>  21
<212>  RNA
<213>  Artificial Sequence

<220>
<223>  hebra antisentido dsRNAide señalización de PIAS2b

<400>  29
caggaagaua caguuauggu u                                                21


<210>  30
<211>  21
<212>  RNA
<213>  Artificial Sequence

<220>
<223>  hebra antisentido dsRNAide señalización de PIAS2b

<400>  30
gauuuguaga cuccauccau u                                                21


<210>  31
<211>  21
<212>  RNA
<213>  Artificial Sequence

<220>
<223>  hebra sentido dsRNAide señalización de PIAS2b

<400>  31
uaauuuggua uuuauugaau u                                                21


<210>  32
<211>  21
<212>  RNA
<213>  Artificial Sequence

<220>
<223>  hebra sentido dsRNAide señalización de PIAS2b

<400>  32
caagaaccaa augacaugcu u                                                21


<210>  33
<211>  21
<212>  RNA
<213>  Artificial Sequence

<220>
<223>  hebra sentido dsRNAide señalización de PIAS2b

<400>  33
uuugguauuu auugaaaguu u                                                21


<210>  34
<211>  21
<212>  RNA
<213>  Artificial Sequence

<220>
<223>  hebra sentido dsRNAide señalización de PIAS2b

<400>  34
ccauaacugu aucuuccugu u                                                21


<210>  35
<211>  21
<212>  RNA
<213>  Artificial Sequence

<220>
<223>  hebra sentido dsRNAide señalización de PIAS2b

<400>  35
uggauggagu cuacaaaucu u                                                21


<210>  36
<211>  21
<212>  DNA
<213>  Artificial Sequence

<220>
<223>  DNA hebra antisentido dsRNAide señalización de PIAS2b

<400>  36
ttcaataaat accaaattat t                                                21


<210>  37
<211>  21
<212>  DNA
<213>  Artificial Sequence

<220>
<223>  DNA hebra antisentido dsRNAide señalización de PIAS2b

<400>  37
gcatgtcatt tggttcttgt t                                                21

<210>  38
<211>  21
<212>  DNA
<213>  Artificial Sequence

<220>
<223>  DNA hebra antisentido dsRNAide señalización de PIAS2b

<400>  38
actttcaata aataccaaat t                                                21

<210>  39
<211>  21
<212>  DNA
<213>  Artificial Sequence

<220>
<223>  DNA hebra antisentido dsRNAide señalización de PIAS2b

<400>  39
caggaagata cagttatggt t                                                21

<210>  40
<211>  21
<212>  DNA
<213>  Artificial Sequence

<220>
<223>  DNA hebra antisentido dsRNAide señalización de PIAS2b

<400>  40
gatttgtaga ctccatccat t                                                21

<210>  41
<211>  21
<212>  DNA
<213>  Artificial Sequence

<220>
<223>  DNA hebra sentido dsRNAide señalización de PIAS2b

<400>  41
taatttggta tttattgaat t                                                21

<210>  42
<211>  21
<212>  DNA
<213>  Artificial Sequence

<220>
<223>  DNA sense strans dsRNAi targeted to PIAS2b

<400>  42
caagaaccaa atgacatgct t                                                21

<210>  43
<211>  21
<212>  DNA
<213>  Artificial Sequence

<220>
<223>  DNA hebra antisentido dsRNAide señalización de PIAS2b

<400>  43
tttggtattt attgaaagtt t                                                21


<210>  44
<211>  21
<212>  DNA
<213>  Artificial Sequence

<220>
<223>  DNA hebra antisentido dsRNAide señalización de PIAS2b

<400>  44
ccataactgt atcttcctgt t                                                21


<210>  45
<211>  21
<212>  DNA
<213>  Artificial Sequence

<220>
<223>  DNA hebra antisentido dsRNAide señalización de PIAS2b

<400>  45
tggatggagt ctacaaatct t                                                21


<210>  46
<211>  21
<212>  DNA
<213>  Artificial Sequence

<220>
<223>  Control negativo

<400>  46
aagcgcgata gcgcgaatat a                                                21


<210>  47
<211>  19
<212>  RNA
<213>  Artificial Sequence

<220>
<223>  control negativo hebra antisentido dsRNAi

<400>  47
uauauucgcg cuaucgcgc                                                   19


<210>  48
<211>  19

<212>  RNA
<213>  Artificial Sequence

<220>
<223>  control negativo hebra sentido dsRNAi

<400>  48
gcgcgauagc gcgaauaua                                                    19


<210>  49
<211>  19
<212>  DNA
<213>  Artificial Sequence

<220>
<223>  c

<400>  49
tatattcgcg ctatcgcgc                                                    19


<210>  50
<211>  19
<212>  DNA
<213>  Artificial Sequence

<220>
<223>  DNA control negativo hebra sentido dsRNAi

<400>  50
gcgcgatagc gcgaatata                                                    19


<210>  51
<211>  21
<212>  RNA
<213>  Artificial Sequence

<220>
<223>  control negativo hebra antisentido dsRNAi

<400>  51
uauauucgcg cuaucgcgcu u                                                 21


<210>  52
<211>  21
<212>  RNA
<213>  Artificial Sequence

<220>
<223>  control negativo hebra sentido dsRNAi

<400>  52
gcgcgauagc gcgaauauau u                                                 21


<210>  53
<211>  21
<212>  DNA
<213>  Artificial Sequence

<220>
<223>  DNA control negativo hebra antisentido dsRNAi

<400>  53
tatattcgcg ctatcgcgct t                                                21


<210>  54
<211>  21
<212>  DNA
<213>  Artificial Sequence

<220>
<223>  DNA control negativo hebra sentido dsRNAi

<400>  54
gcgcgatagc gcgaatatat t                                                21


<210>  55
<211>  29
<212>  DNA
<213>  Artificial Sequence

<220>
<223>  Cebador

<400>  55
aataatttgg tatttattga acctgtctc                                        29


<210>  56
<211>  29
<212>  DNA
<213>  Artificial Sequence

<220>
<223>  Cebador

<400>  56
aattcaataa ataccaaatt acctgtctc                                        29


<210>  57
<211>  29
<212>  DNA
<213>  Artificial Sequence

<220>
<223>  Cebador

<400>  57
aacaagaacc aaatgacatg ccctgtctc                                        29


<210>  58
<211>  29
<212>  DNA
<213>  Artificial Sequence

<220>
<223>  Cebador

<400>  58
aagcatgtca tttggttctt gcctgtctc                                          29

<210>  59
<211>  29
<212>  DNA
<213>  Artificial Sequence

<220>
<223>  Cebador

<400>  59
aatttggtat ttattgaaag tcctgtctc                                          29

<210>  60
<211>  29
<212>  DNA
<213>  Artificial Sequence

<220>
<223>  Cebador

<400>  60
aaactttcaa taaataccaa acctgtctc                                          29

<210>  61
<211>  29
<212>  DNA
<213>  Artificial Sequence

<220>
<223>  Cebador

<400>  61
aaccataact gtatcttcct gcctgtctc                                          29

<210>  62
<211>  29
<212>  DNA
<213>  Artificial Sequence

<220>
<223>  Cebador

<400>  62
aacaggaaga tacagttatg gcctgtctc                                          29

<210>  63
<211>  29
<212>  DNA
<213>  Artificial Sequence

<220>
<223>  Cebador

<400>  63
aatggatgga gtctacaaat ccctgtctc                                          29

<210>  64
<211>  29
<212>  DNA
<213>  Artificial Sequence

<220>
<223>  Cebador

<400>  64
aagatttgta gactccatcc acctgtctc                                          29


<210>  65
<211>  29
<212>  DNA
<213>  Artificial Sequence

<220>
<223>  Cebador

<400>  65
aagcgcgata gcgcgaatat acctgtctc                                          29


<210>  66
<211>  29
<212>  DNA
<213>  Artificial Sequence

<220>
<223>  Cebador

<400>  66
aatatattcg cgctatcgcg ccctgtctc                                          29


<210>  67
<211>  567
<212>  DNA
<213>  Homo sapiens PIAS2b-UTR

<400>  67
tcagatttgc caggtttgga ttttctttcc cttattccag ttgatcccca gtactgtcct        60

cctatgtttt tggatagtct cacctcaccc ttaacagcaa gcagtacgtc tgtcaccacc       120

accagctccc atgaaagcag tactcatgtt agttcatcca gcagcaggag tgagacaggg       180

gtcataacca gcagtggaag taacattcct gacatcatct cattggacta aaggaggact       240

cacttgattc tgggaatcat tcatcagaac tgctttttct tggatctcta gtctgtggaa       300

acgttctttt tttttttttt ttaataattt ggtatttatt gaaagtcaga tggattcttt       360

tgctttctga ggggtgaaca cagaagactg caacaagaac caaatgacat gcaaggattt       420

ttcttaatta tgctgtactc tcagcatcag atacattcaa ccataactgt atcttcctga       480

gagatggatt tcactttcat acgttaatgg atggagtcta caaatcagtg aaaaaagttt       540

ttgttaaaat aaagagcaat aaaatta                                           567

<210>  68
<211>  28
<212>  PRT
<213>  Artificial Sequence

<220>
<223>  Péptido de vimentina

<400>  68

Ser Leu Tyr Ser Ser Ser Pro Gly Gly Ala Tyr Val Thr Arg Ile Ser
1               5                   10                  15

Leu Pro Leu Pro Asn Phe Ser Ser Leu Asn Leu Arg
            20                  25

<210>  69
<211>  66
<212>  PRT
<213>  Artificial Sequence

<220>
<223>  Péptido de PIAS2b

<400>  69

Ala Asp Phe Glu Glu Leu Arg Asn Met Val Ser Ser Phe Arg His Asp
1               5                   10                  15

Leu Leu Met Arg Ala Leu His Leu Leu Lys Phe Phe Ile Phe Ala Leu
            20                  25                  30

Thr Pro Gln Gln Val Arg Glu Ile Cys Ile Ser Arg Met Arg Leu Thr
        35                  40                  45

Ile Pro Cys Arg Phe Gln Glu Asp Gly Ser Trp Cys Pro Met Arg Pro
    50                  55                  60

Lys Lys
65

<210>  70
<211>  18
<212>  DNA
<213>  Artificial Sequence

<220>
<223>  Cebador

<400>  70
gcccgaaacg ccgaatat                                                    18

<210>  71
<211>  23

<212>  DNA
<213>  Artificial Sequence

<220>
<223>  Cebador

<400>  71
ttcgtggctc tcttatcctc atg                                               23


<210>  72
<211>  22
<212>  DNA
<213>  Artificial Sequence

<220>
<223>  Cebador

<400>  72
tcccaagcgg tttgctgcgg ta                                                22


<210>  73
<211>  20
<212>  DNA
<213>  Artificial Sequence

<220>
<223>  Cebador

<400>  73
ttggctcaaa ccactgtgtc                                                   20


<210>  74
<211>  20
<212>  DNA
<213>  Artificial Sequence

<220>
<223>  Cebador

<400>  74
gggtctcgct atgttgccta                                                   20


<210>  75
<211>  20
<212>  DNA
<213>  Artificial Sequence

<220>
<223>  Cebador

<400>  75
caagctgggt gcggtggctt                                                   20


<210>  76
<211>  20
<212>  DNA
<213>  Artificial Sequence

<220>
<223>  Cebador

<400>  76
atttgaggct tgccttctca                                                    20


<210>  77
<211>  20
<212>  DNA
<213>  Artificial Sequence

<220>
<223>  Cebador

<400>  77
taccacctcc caaaatctgc                                                    20


<210>  78
<211>  23
<212>  DNA
<213>  Artificial Sequence

<220>
<223>  Cebador

<400>  78
tggggtacgg tgtgactgga atg                                                23


<210>  79
<211>  20
<212>  DNA
<213>  Artificial Sequence

<220>
<223>  Cebador

<400>  79
cggttgatcc tgctgctatt                                                    20


<210>  80
<211>  23
<212>  DNA
<213>  Artificial Sequence

<220>
<223>  Cebador

<400>  80
aagatgttcc aagcttcagt tca                                                23


<210>  81
<211>  20
<212>  DNA
<213>  Artificial Sequence

<220>
<223>  Cebador

<400>  81
cggttgatcc tgctgctatt                                                   20


<210>  82
<211>  20
<212>  DNA
<213>  Artificial Sequence

<220>
<223>  Cebador

<400>  82
cctcaccctt aacagcaagc                                                   20


<210>  83
<211>  20
<212>  DNA
<213>  Artificial Sequence

<220>
<223>  cebador

<400>  83
gcttgctgtt aagggtgagg                                                   20


<210>  84
<211>  20
<212>  DNA
<213>  Artificial Sequence

<220>
<223>  cebador

<400>  84
gcggatttcg aagagttgag                                                   20


<210>  85
<211>  20
<212>  DNA
<213>  Artificial Sequence

<220>
<223>  cebador

<400>  85
agccgctctt caataaatgc                                                   20


<210>  86
<211>  18
<212>  DNA
<213>  Artificial Sequence

<220>
<223>  cebador

<400>  86
gcccgaaacg ccgaatat                                                     18

<210>  87
<211>  20
<212>  DNA
<213>  Artificial Sequence

<220>
<223>  Oligonucleótido

<400>  87
caagctgggt gcggtggctt                                                    20


<210>  88
<211>  23
<212>  DNA
<213>  Artificial Sequence

<220>
<223>  Oligonucleótido

<400>  88
tggggtacgg tgtgactgga atg                                                23


<210>  89
<211>  20
<212>  DNA
<213>  Artificial Sequence

<220>
<223>  cebador

<400>  89
ttggctcaaa ccactgtgtc                                                    20


<210>  90
<211>  20
<212>  DNA
<213>  Artificial Sequence

<220>
<223>  cebador

<400>  90
gggtctcgct atgttgccta                                                    20


<210>  91
<211>  20
<212>  DNA
<213>  Artificial Sequence

<220>
<223>  cebador

<400>  91
atttgaggct tgccttctca                                                    20


<210>  92
<211>  20

<212>  DNA
<213>  Artificial Sequence

<220>
<223>  cebador

<400>  92
taccacctcc caaaatctgc                                                    20


<210>  93
<211>  21
<212>  DNA
<213>  Homo sapiens

<400>  93
aataatttgg tatttattga a                                                  21


<210>  94
<211>  21
<212>  DNA
<213>  Homo sapiens

<400>  94
aacaagaacc aaatgacatg c                                                  21


<210>  95
<211>  21
<212>  DNA
<213>  Homo sapiens

<400>  95
aatttggtat ttattgaaag t                                                  21


<210>  96
<211>  21
<212>  DNA
<213>  Homo sapiens

<400>  96
aaccataact gtatcttcct g                                                  21


<210>  97
<211>  21
<212>  DNA
<213>  Homo sapiens

<400>  97
aatggatgga gtctacaaat c                                                  21


<210>  98
<211>  21
<212>  DNA
<213>  Homo sapiens

<400>  98
aagcgcgata gcgcgaatat a                                                  21

<210>  99
<211>  600
<212>  DNA
<213>  Artificial

<220>
<223>  Exones 13 a 14 del ARNm de PIAS2b

<400>  99
ggttctcatg tatcagccat cttctgtaag ggtgcccagt gtgacttcgg ttgatcctgc      60

tgctattccg ccttcattaa cagactactc agtaccattc caccatacgc caatatcaag     120

catgtcatca gatttgccag gtttggattt tctttccctt attccagttg atccccagta     180

ctgtcctcct atgtttttgg atagtctcac ctcaccctta acagcaagca gtacgtctgt     240

caccaccacc agctcccatg aaagcagtac tcatgttagt tcatccagca gcaggagtga     300

gacaggggtc ataaccagca gtggaagtaa cattcctgac atcatctcat tggactaaag     360

gaggactcac ttgattctgg gaatcattca tcagaactgc tttttcttgg atctctagtc     420

tgtggaaacg ttcttttttt ttttttttta ataatttggt atttattgaa agtcagatgg     480

attcttttgc tttctgaggg gtgaacacag aagactgcaa caagaaccaa atgacatgca     540

aggatttttc ttaattatgc tgtactctca gcatcagata cattcaacca taactgtatc     600


<210>  100
<211>  211
<212>  DNA
<213>  Artificial

<220>
<223>  Exones 12 a 14 del ARNm de PIAS2a

<400>  100
ggttctcatg tatcagccat cttctgtaag ggtgcccagt gtgacttcgg ttgatcctgc      60

tgctattccg ccttcattaa cagactactc agtaccattc caccatacgc caatatcaag     120

catgtcatca gatttgccag gagaacaaag aagaaatgat attaataatg aactgaagct     180

tggaacatct tctgatactg tgcaacagtg a                                    211

## Description

### FIELD OF THE INVENTION

The present invention relates to novel pharmacological agents in advanced cancer therapies. More specifically, the invention relates to inhibition of the expression of a marker for inducing the death and inhibiting the proliferation of tumour cells in undifferentiated or poorly differentiated carcinomas of the thyroid gland.

### BACKGROUND OF THE INVENTION

Thyroid tumours are the most common malignant tumours of the endocrine organs with increasing incidence in recent years. Most malignant thyroid tumours, referred to as carcinomas, originate in follicular cells and are divided into three large groups: a) differentiated carcinoma, b) poorly differentiated carcinoma, and c) undifferentiated carcinoma, according to their histological and clinical characteristics.

Differentiated follicular epithelial carcinomas of the thyroid gland have become one of the most common cancers among women. Therapy is effective, although no patient can be considered cured due to the possibility of recurrence, either as a persistence or else as a regrowth, until many years later.

In terms of undifferentiated/anaplastic carcinomas, recurring poorly differentiated carcinomas and differentiated carcinomas are part of the group of advanced carcinomas. Undifferentiated/anaplastic carcinomas are considered an orphan disease because they constitute a very small number of cases, with a fatal prognosis within a few months. New treatments are based on compassionate individual use of treatments identified for other cancers in patients who have received several highly aggressive types of therapy within a short time, without there being any controlled clinical trial in view of the scarcity of living patients.

Therefore, there is a need in the field to provide more effective novel treatments for treating follicular epithelial carcinomas of the thyroid gland, particularly undifferentiated/anaplastic carcinomas.

### SUMMARY OF THE INVENTION

The present invention is based on the finding that inhibition of the expression of the PIAS2β protein results in specific inhibition of the proliferation of and the induction of death of cells of undifferentiated or poorly differentiated carcinoma of the thyroid gland. Therefore, inhibition of PIAS2β gives rise to a reduction in growth of carcinomas of this type. Furthermore, the inventors have surprisingly observed that the PIAS2β protein is less expressed in cells of undifferentiated or poorly differentiated carcinoma of the thyroid gland than in the cells of normal tissue or in the cells of differentiated papillary carcinoma of the thyroid.

Therefore, in a first aspect, the invention relates to a nucleic acid comprising an antisense nucleotide sequence complementary to a target region of the PIAS2β mRNA, wherein the binding of the nucleic acid to its target sequence causes inhibition of the expression of the PIAS2β mRNA.

In a second aspect, the invention relates to a vector comprising a nucleic acid encoding the antisense sequence of the nucleic acid of the first aspect the invention.

In a third aspect, the invention relates to a host cell comprising the nucleic acid of the first aspect of the invention or the vector of the second aspect of the invention.

In a fourth aspect, the invention relates to a pharmaceutical composition comprising the nucleic acid of the first aspect of the invention, or the vector of the second aspect of the invention, and a pharmaceutically acceptable excipient of the fourth aspect of the invention.

In a fifth aspect, the invention relates to an inhibitor of the PIAS2β protein for use in the treatment of cancer in a patient.

In a sixth aspect, the invention relates to a polymerase chain reaction (PCR) amplification method for detecting, in a differential manner, the nucleic acid sequence of isoform β or of isoform α of the PIAS2 gene in a sample, said process comprising:
(a) providing the PCR assay containing said sample with a labelled oligonucleotide containing a sequence complementary to a region of the target nucleic acid, wherein said labelled oligonucleotide hybridises with the target nucleic acid sequence, binding thereto through the primers of step (b), and wherein said labelled oligonucleotide has the sequence SEQ ID NO: 87 when the detection of the nucleic acid sequence of isoform α of the PIAS2 gene is desired or SEQ ID NO: 88 when the detection of the nucleic acid sequence of isoform β of the PIAS2 gene is desired;
(b) providing a pair of primers, the first primer containing a sequence complementary to a region of a chain of the target nucleic acid sequence and initiates the synthesis of an extension product and a second primer containing a sequence complementary to the other chain of the target nucleic acid sequence or complementary to a region in the extension product of the first primer and initiates the synthesis of a complementary DNA strand; and wherein each primer is selected so that it hybridises with its complementary template upstream of any labelled oligonucleotide hybridised with the same nucleic acid chain, wherein the primer pair is formed by the primers of sequence SEQ ID NO: 89 and SEQ ID NO: 90 when the detection of the nucleic acid sequence of isoform α of the PIAS2 gene is desired or by the primers of sequence SEQ ID NO: 91 and SEQ ID NO: 92 when the detection of the nucleic acid sequence of isoform β of the PIAS2 gene is desired,
(c) amplifying the target nucleic acid sequence using a nucleic acid polymerase with 5' to 3' nuclease activity as a template-dependent polymerising agent under conditions permissive for PCR cycling steps of (i) hybridising the primers and the labelled oligonucleotide with a template nucleic acid sequence contained within the target sequence, and (ii) extending the primer, wherein said nucleic acid polymerase synthesises a primer extension product while the 5' to 3' nuclease activity of the nucleic acid polymerase simultaneously releases labelled fragments of the hybridised double chains comprising labelled oligonucleotides and their complementary template nucleic acid sequences, thereby creating detectable labelled fragments, and
(d) detecting and/or measuring the signal generated by the hydrolysis of the labelled oligonucleotide in order to determine the presence or absence of the target sequence in the sample.

In a seventh aspect, the invention relates to a kit for the differential detection of the nucleic acid sequence of isoform β or of isoform α of the PIAS2 gene in a sample comprising:
(a) at least one labelled oligonucleotide containing a sequence complementary to a region of the target nucleic acid, wherein said labelled oligonucleotide has the sequence SEQ ID NO: 87 when the detection of the nucleic acid sequence of isoform α of the PIAS2 gene is desired or SEQ ID NO: 88 when the detection of the nucleic acid sequence of isoform β of the PIAS2 gene is desired, and the 3' end of the labelled oligonucleotide being blocked to prevent extension by a nucleic acid polymerase having 5' to 3' activity, and the labelled oligonucleotide containing first and second markers, the first marker being separated from the second marker by a site susceptible to cleavage by a nuclease, and wherein the markers of the labelled oligonucleotide constitute a pair of interactive signal-generating markers located on the oligonucleotide to shield the generation of a detectable signal, and
(b) at least one pair of primers, formed by the primers of sequence SEQ ID NO: 89 and SEQ ID NO: 90 when the detection of the nucleic acid sequence of isoform α of the PIAS2 gene is desired or by the primers of sequence SEQ ID NO: 91 and SEQ ID NO: 92 when the detection of the nucleic acid sequence of isoform β of the PIAS2 gene is desired.

### BRIEF DESCRIPTION OF THE DRAWINGS

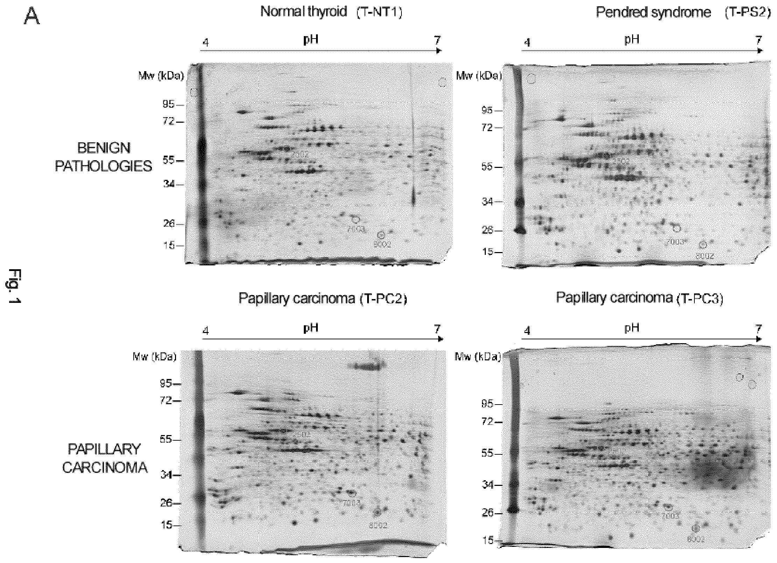

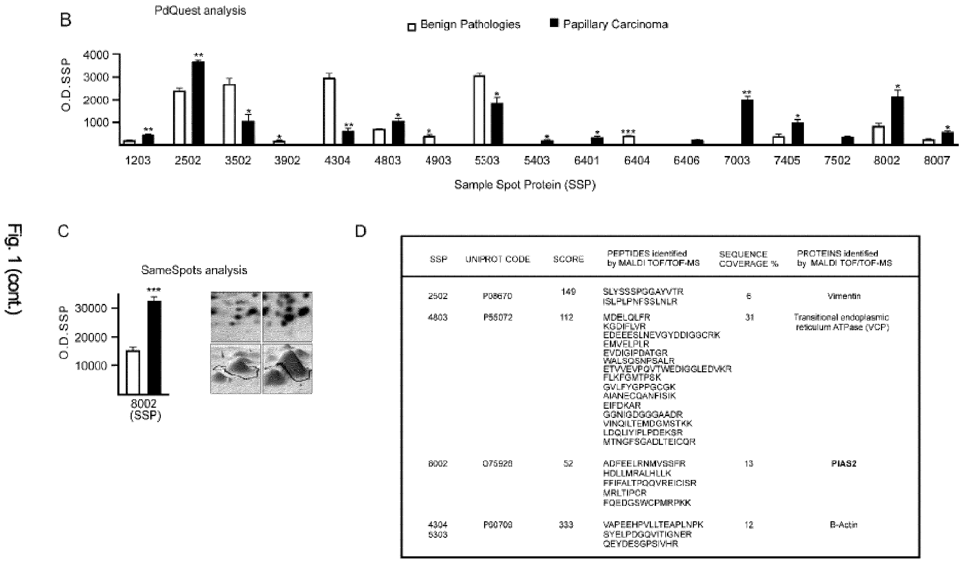

**Figure 1****.** 2D-proteomics differential expression study between normal/benign tissue and papillary thyroid carcinoma cultures. **A)** Two gels of the "benign" cultures and two gels of "papillary carcinoma" after the second dimension and silver staining are shown. **B)** Spots showing greater differences after PDQuest analysis. **C)** Repetition of the analysis with the SameSpots programme for spot 8002 is shown. **D)** Identification of three of the significantly increased spots and of two of the significantly decreased spots in papillary carcinomas. (Mann-Whitney; ^{∗}, p<0.05; ^{∗∗}, p<0.01; ^{∗∗∗}, p<0.001).

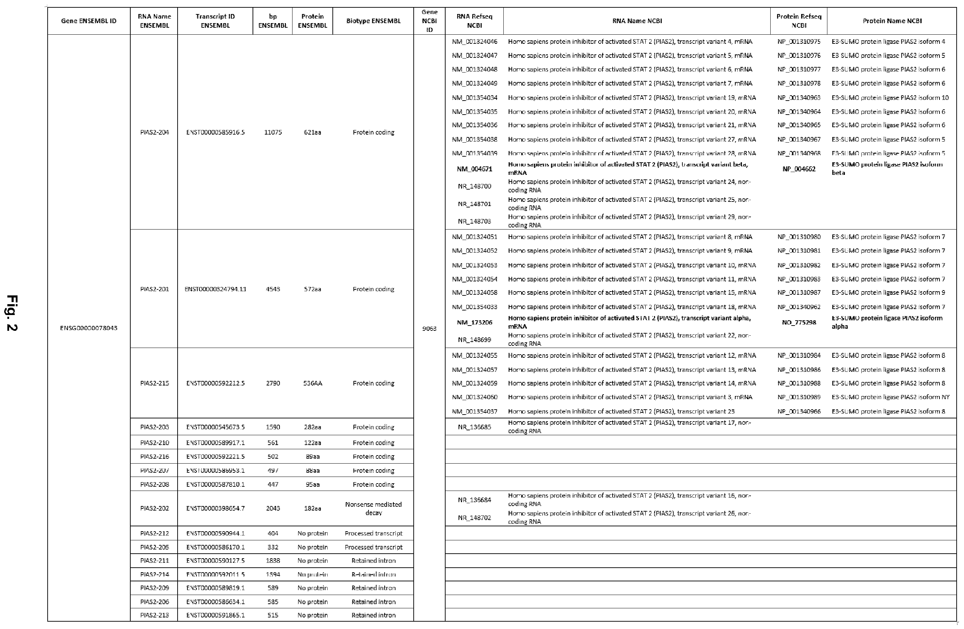

**Figure 2****.** Human PIAS2 gene: its mRNA and protein isoforms with equivalence of the codes of the two international consortiums, Ensembl and NCBI.

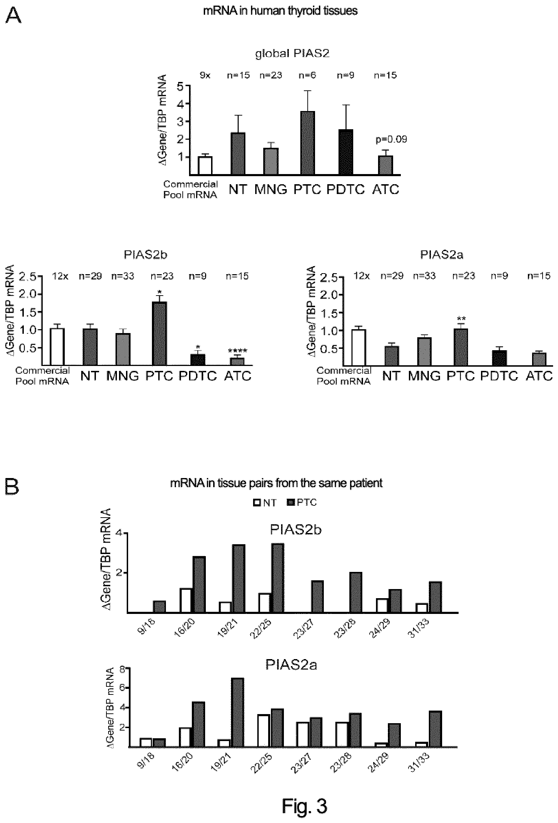

**Figure 3****.** PIAS2b and PIAS2a expression in tissues of normal human thyroid, of benign proliferation and cancer. **A)** Expression of PIAS2b and PIAS2a mRNA measured by means of quantitative PCR, TaqMan qRT-PCR. **B)** Comparison of the detected levels per tissue pairs originating from the same patient, PTC cancer vs. the corresponding normal tissue. (NT, normal thyroid; MNG, multinodular goitre; PTC, papillary carcinoma; PDTC, poorly differentiated carcinoma; ATC, anaplastic carcinoma). (Kruskal-Wallis; ^{∗}, p<0.05; ^{∗∗}, p<0.01; ^{∗∗∗∗}, p<0.000).

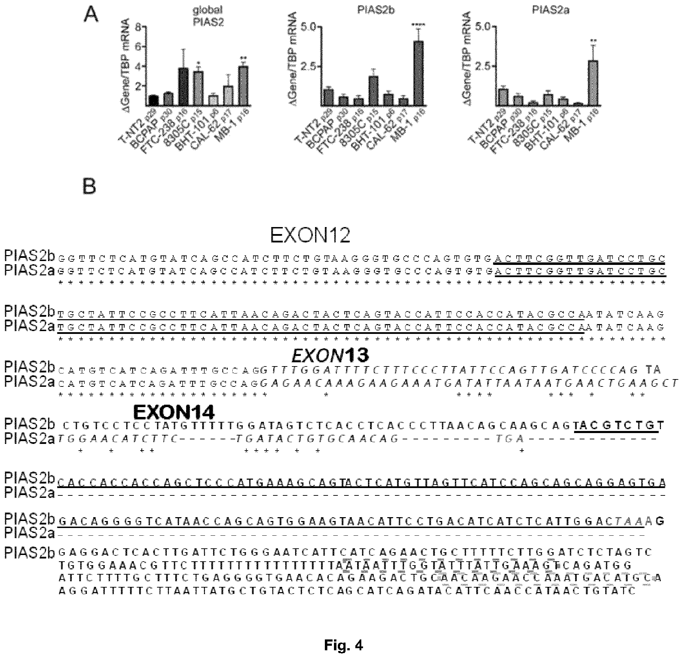

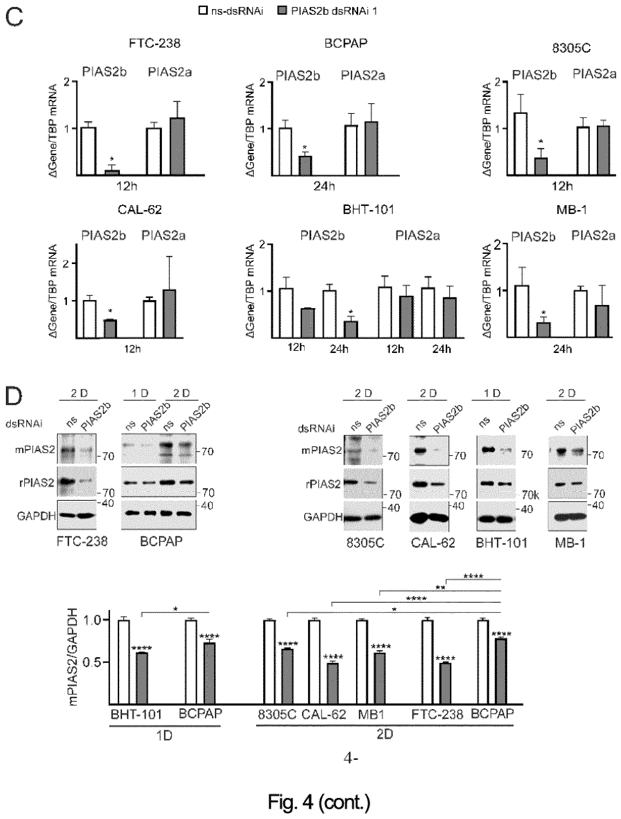

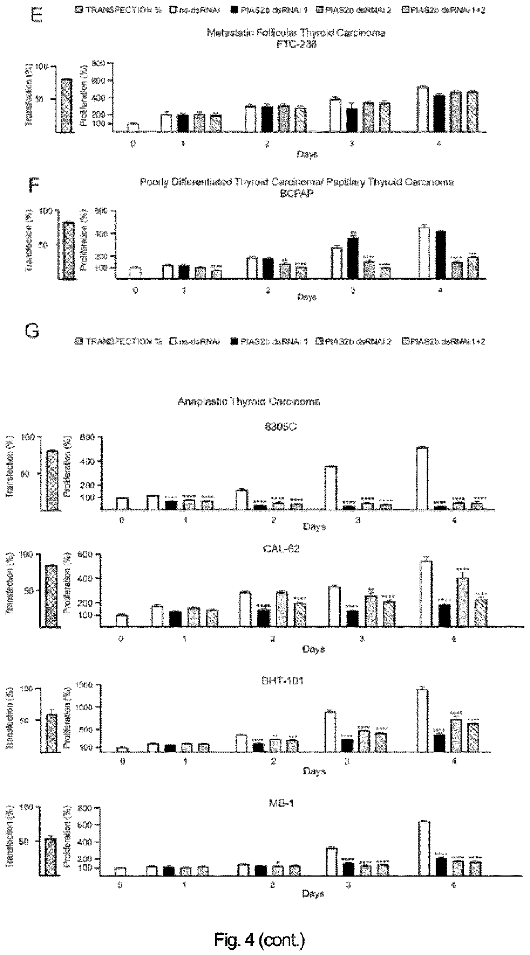

**Figure 4****. PIAS2b dsRNAi inhibits the proliferation of anaplastic thyroid carcinoma lines. A)** Expression of global PIAS2 mRNA and of each of the isoforms of the protein in cancer lines in comparison with a culture of normal cells which grows indefinitely (T-NT2). BCPAP and FTC-238 originate from poorly differentiated papillary or follicular carcinomas (PDTC), respectively; 8305C, CAL-62, BHT-101, MB-1 originate from anaplastic carcinomas of different continents and genetic background. **B)** Sequence of exons 12, 13, and 14 of the PIAS2 gene indicating the differences between the two isoforms, PIAS2a and PIAS2b. The target dsRNAi sequences 1 and dsRNAi 2 from 5' to 3' are included in dashed line. Bases corresponding to the amino acids of the epitope of the mPIAS2 antibody (in a dark colour in exon 12) and of the rPIAS2 antibody (in a light colour in exon 14) are shown underlined. **C-D)** PIAS2 dsRNAi 1 supresses the expression , of mRNA measured by qRT-PCR **(C)** and protein **(D),** specifically of isoform beta, in all the cell lines. The same result was obtained with two different antibodies: mPIAS2, mouse anti-human PIAS2 antibody, and rPIAS2, rabbit anti-human PIAS2b antibody. Bottom: quantification of the p75 kDa PIAS2 protein with respect to the GAPDH loading control in Western blots. **E-G)** Effect of PIAS2b dsRNAi 1, PIAS2b dsRNAi 2, or the sum of both on FTC-238 **(E),** BCPAP **(F),** or the four anaplastic carcinoma lines **(G).** Transfection efficiency was greater than 50% in all the lines. (A: Kruskal-Wallis; C: Mann-Whitney; D: ANOVA; E-G: Two-way ANOVA with repeated measurements. ^{∗}, p<0.05; ^{∗∗}, p<0.01; ^{∗∗∗}, p<0.001; ^{∗∗∗∗}, p<0.000).

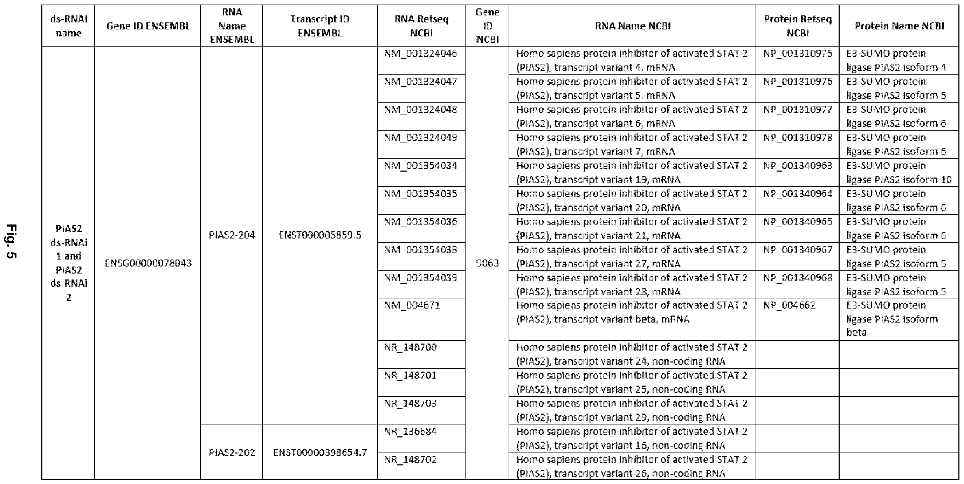

**Figure 5****.** PIAS2b dsRNAi: recognized PIAS2 isoforms. Both sequence 1 and sequence 2 recognize a single protein isoform PIAS2-204 (PIAS2b) and a non-coding isoform PIAS2-202 based on Ensembl codes. Equivalence with NCBI codes is shown.

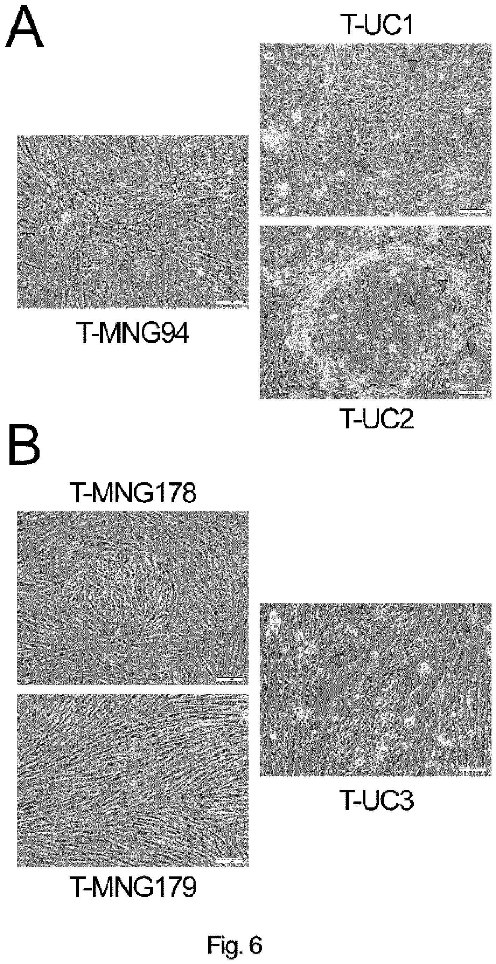

**Figure 6****.** Primary cultures of benign tissue (T-MNG) and of anaplastic carcinoma tissue (T-UC) established from independent fragments of surgical residues from two patients operated for this disease. **A)** Two anaplastic cultures, T-UC1 and T-UC2, and one benign tissue culture, T-MNG94, were established from this patient. **B)** Two benign cultures (T-MNG178 and T-MNG179) and one anaplastic culture (T-UC3) were established from this patient.

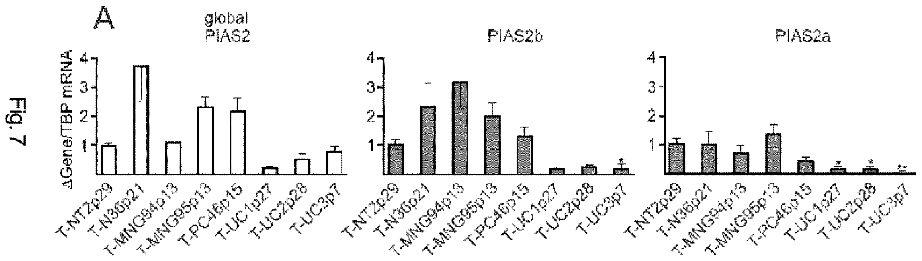

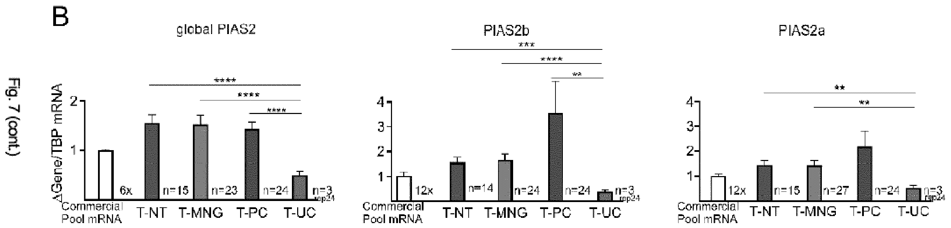

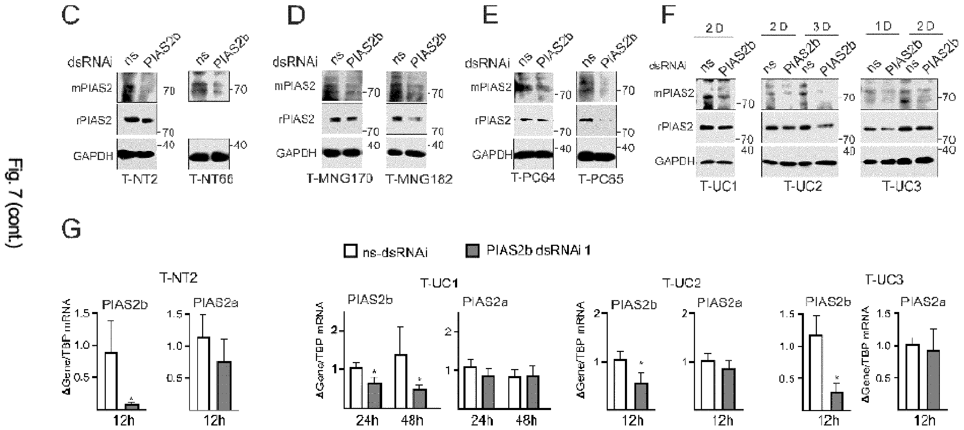

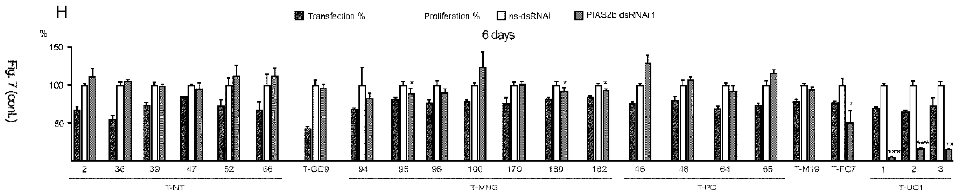

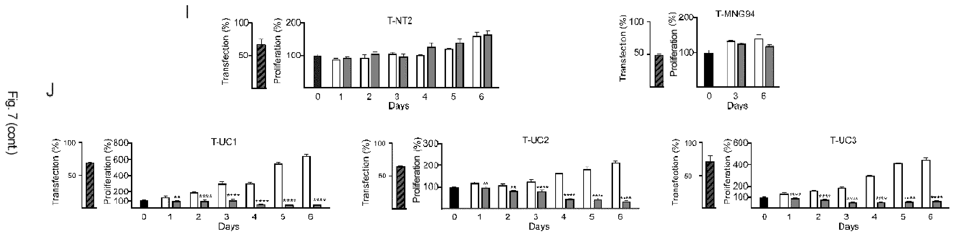

**Figure 7****.** PIAS2b dsRNAi 1 inhibits the growth of primary anaplastic cultures from patients, but not their normal, goitre, or differentiated carcinoma cultures. **A)** Expression of total PIAS2 mRNA and its isoforms in individual cultures of a different pathological origin. Culture passage (splitting of the cells obtained into two new empty plates) from passage 1 (p1), in which the culture is established, is indicated. T-NT, normal thyroid culture; T-MNG, multinodular goitre culture; T-PC, papillary carcinoma culture, T-UC, anaplastic carcinoma culture. **B)** Quantification of the expression of PIAS2 mRNA for groups of cultures of the same pathological origin. The three assays are shown: global PIAS2 which measures all the coding isoforms; PIAS2b which measures coding isoform PIAS-204 (PIAS2b), and PIAS2a which measures coding isoform PIAS-201 (PIAS2a). It is observed that anaplastic cultures (T-UC) express significantly little PIAS2, whereas papillary carcinoma cultures (T-PC) express more PIAS2, as their tissues of origin do, as demonstrated in Figure 3. This result is particularly noticeable when measuring isoform PIAS2b. **C-F)** PIAS2b dsRNAi 1 supresses expression of the PIAS2b protein in cultures derived from T-NT **(C),** T-MNG **(D),** differentiated papillary carcinoma T-PC **(E),** and T-UC **(F). G)** PIAS2b dsRNAi 1 specifically supresses expression of PIAS2b mRNA, but not of PIAS2a mRNA in T-NT and T-UC. **H)** Effect of PIAS2b dsRNAi 1 with respect to the control ns-dsRNAi on the proliferation of cultures of different pathological origin, measured six days after transfection. **I-J)** Effect of PIAS2b dsRNAi 1 on the proliferation over time of cultures of different origin. **I)** Normal thyroid culture T-NT and T-MNG94. **J)** Cultures T-UC1 and T-UC2, and in T-UC3 (T-MNG94 originates from the same patient as T-UC1 and T-UC2) (A-B: Kruskal-Wallis; F-G-H-I: Mann-Whitney; ^{∗}, p<0.05; ^{∗∗}, p<0.01; ^{∗∗∗}, p<0.001; ^{∗∗∗∗}, p<0.000)

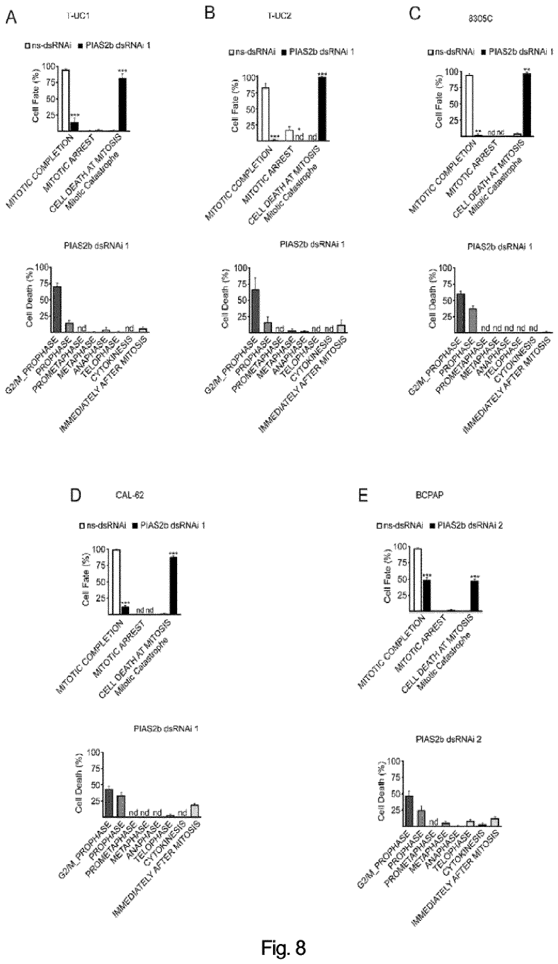

**Figure 8****.** PIAS2b dsRNAi exhibits a cytotoxic anti-tumour effect which causes mitotic catastrophe in anaplastic and poorly differentiated cell lines and cultures. The cells were recorded over time by means of video-microscopy for three days after treatment with PIAS2b dsRNAi (black bars) or ns-dsRNAi (white bars). T-UC1, T-UC2, 8305-C, and CAL-62 were treated with PIAS2b dsRNAi 1; BCPAP was treated with PIAS2b dsRNAi 2. **A-E Top part)** Quantification of the number of cells which successfully finished mitosis (Mitotic Completion), cells which are arrested in mitosis (Mitotic Arrest), and cells which die during the mitosis process (Cell death at Mitosis or Mitotic Catastrophe). **A-E Bottom part)** Classification of the phase of mitosis during which cells treated with PIAS2b dsRNAi enter into mitotic catastrophe.

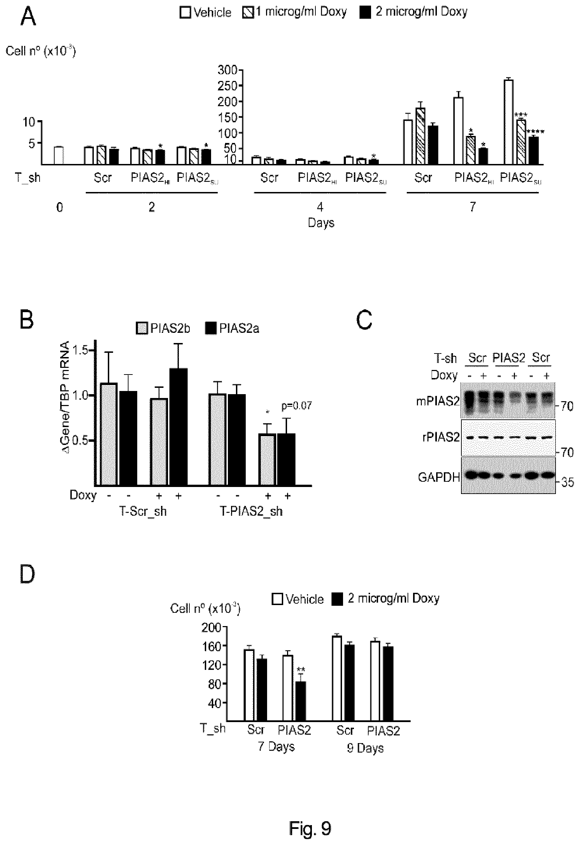

**Figure 9****.** Expression of shRNA with respect to PIAS2 (which supresses both isoforms) in CAL-62 cells reduces proliferation by preventing cell division, but the effect is moderate and lasts very little over time. **A)** Populations infected with lentivirus and selected for their RFP fluorescence intensity, expressing the control sequence T-Scr-shRNA, and two populations expressing the sequence T-PIAS2-shRNA, and with increasing intensity for RFP in the sorter: T-PIAS2_{HI_}sh and T-PIAS2_{SU_}sh. The cells were treated with a vehicle and two concentrations of doxycycline (Doxy) which induce the shRNA. **B)** RT-PCR of the expression of the two PIAS2 coding isoforms of the PIAS2b and PIAS2a proteins in the absence or presence of 2 microg/ml of doxycycline in the control population T-Scr_sh and the population T-PIAS2_{HI_}sh. **C)** Western blot for p75 PIAS2b with the two antibodies, mPIAS2 and rPIAS2, in the presence or absence of doxycycline. **D)** Time-response experiment at 7 and 9 days. (A: ANOVA; B: T-test. E: Mann-Whitney. ^{∗}, p<0.05; ^{∗∗}, p<0.01; ^{∗∗∗}, p<0.001; ^{∗∗∗∗}, p<0.000)

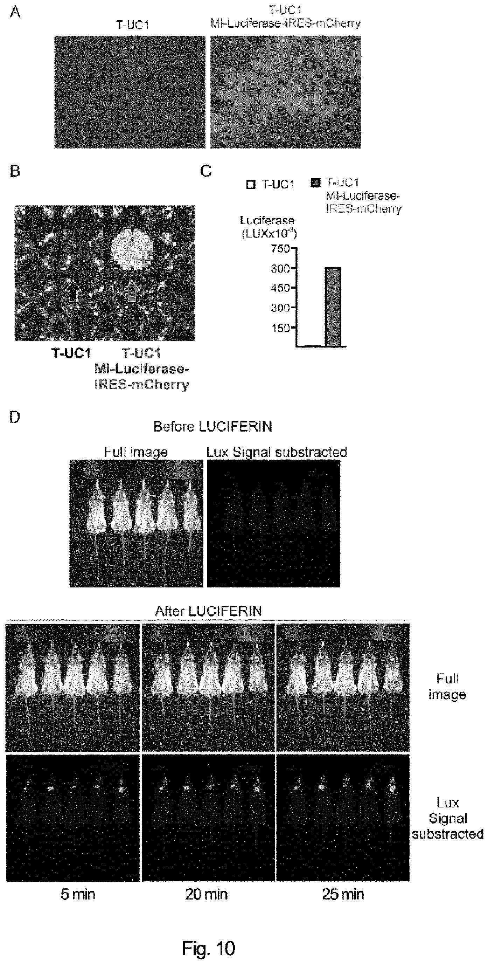

**Figure 10****.** *In vivo* model of anaplastic thyroid carcinoma with orthotopic patient-derived xenograft (oPDX). **A)** Cells infected without virus and with the virus expressing mCherry under optical fluorescence/phase contrast microscope (20x magnification). **B)** The same wells photographed after the addition of the substrate luciferin photographed directly in the IVIS, where the animals with the xenograft will subsequently be recorded. C) Luminometer light emission quantification. **D)** Five mice with oPDX of 2 weeks of evolution, which are recorded in the IVIS before (Before) and after (After) intraperitoneal injection of luciferin and followed-up over time. The full image (Full image) and the specific light signal emitted by the oPDX cancer in the neck of the animals (Lux signal substracted) are observed.

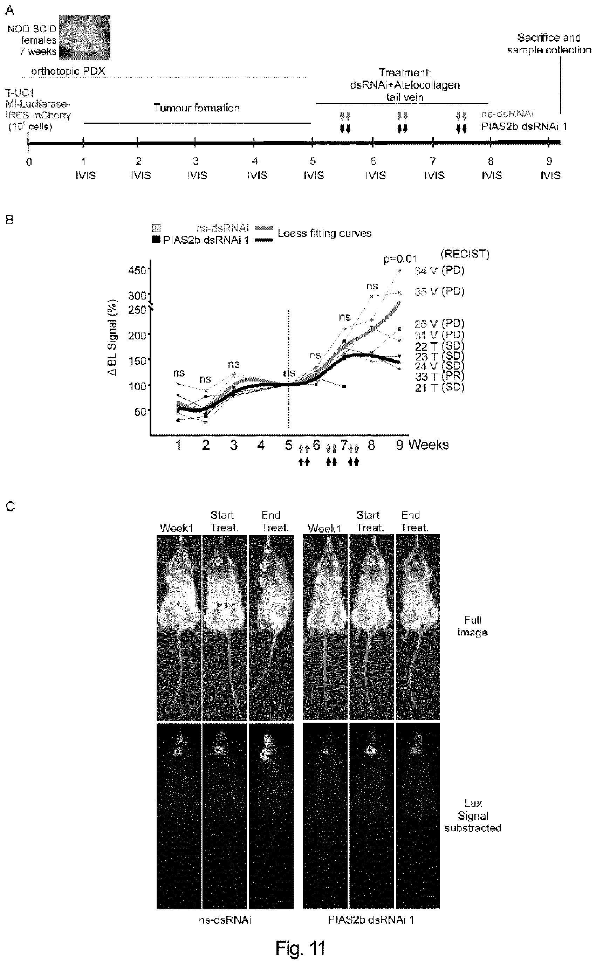

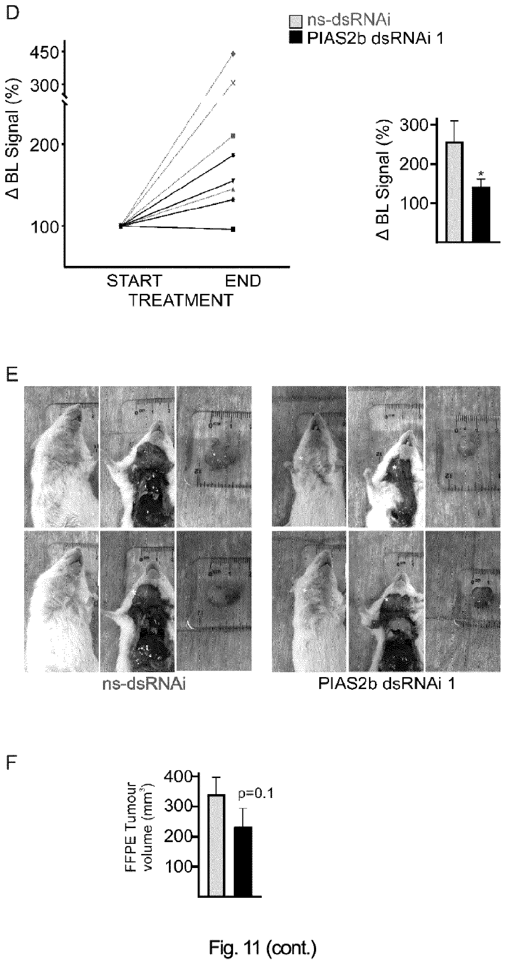

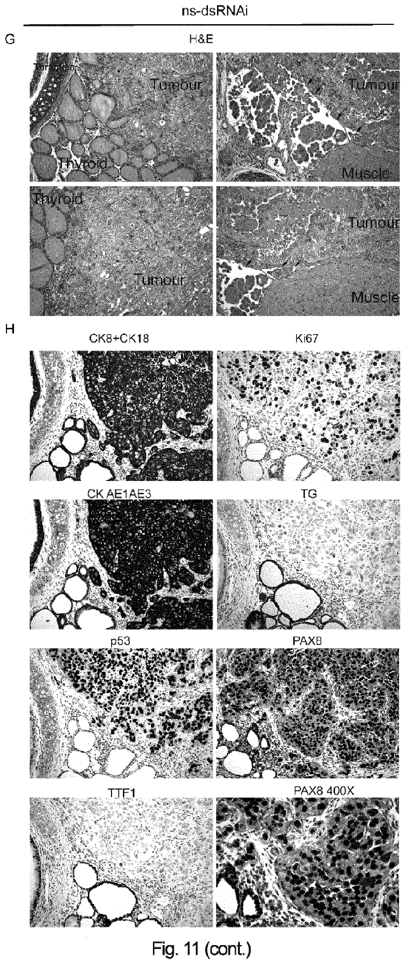

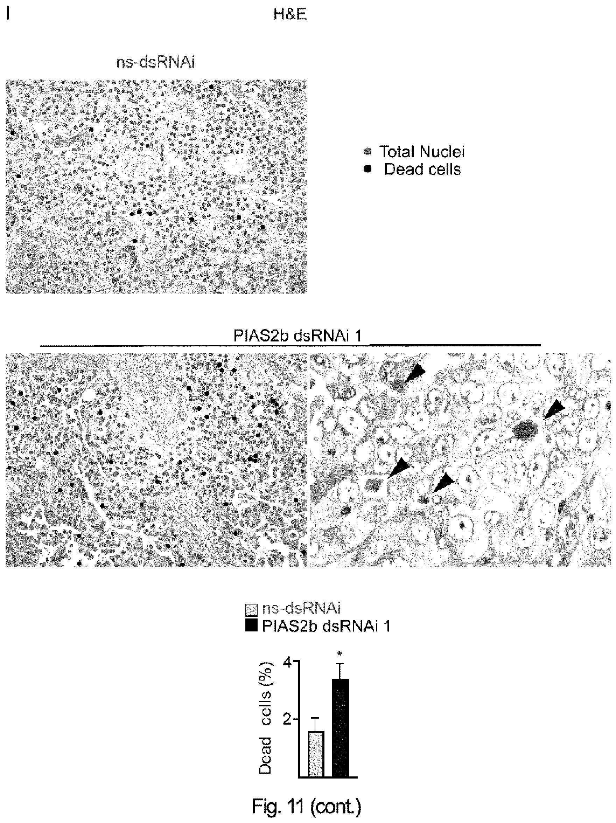

**Figure 11****.** PIAS2 dsRNAi 1 has *in vivo* anti-cancer action. **A)** Diagram of the experiment with the generation of oPDX mice and treatment with PIAS2b dsRNAi 1 (black) or ns-dsRNAi (grey) once/week for three weeks. Follow-up was performed with IVIS once/week. **B)** Individual evolution curves fitted to 100% at week 5, which is the moment when treatment started. Final evaluation of each individual response according to the RECIST code (PD= Progressive disease; SD= Stable Disease; PR= Partial response). In grey, thin dashed lines, animals treated with ns-dsRNAi 1; in black, thin solid lines, animals treated with PIAS2b dsRNAi 1. Statistical analysis demonstrated a significant difference at the end of treatment. In thick, mathematical fitting line LOWESS for data populations that demonstrates growth of oPDX cancers in animals treated with ns-dsRNAi (grey) with respect to stabilization or reduction of growth in those treated with dsRNAi 1 (black). The LOWESS curve coincides with the RECIST evaluation. **C)** Sample from two mice treated with ns-dsRNAi and PIAS2b dsRNAi 1 and the luminescence signal at the start and end of treatments. **D)** Initial and final intensity analysis which demonstrates a statistically significant difference between the two treatments. **E)** Dissection of the carcinomas at the end-point. Differences are observed with the naked eye. **F)** The tumours were fixed, sectioned, and their three-dimensional volume calculated from the sections. The difference in volume of the fixed tissue, calculated from measurements in the sections, has a p=0.1. **G)** Hematoxylin-eosin staining of the carcinomas showing invasion of the healthy thyroid and adjacent structures such as muscle and vessels. It is an image similar to what this carcinoma does in a patient. **H)** The immunohistochemistry performed under the same conditions as for human pathology shows an overall positivity for cytokeratins (A1A3) and for CK8/CK18, as expected in an anaplastic carcinoma. The carcinoma is negative for thyroid differentiation markers (thyroglobulin, TG; NKX2.1, TTF1) but highly positive for Ki67, p53, and PAX8. **I)** Systematic counting of dead or transitional carcinoma cells (black dots and arrows) with respect to total cells (grey dots). Quantification showed significant differences in the treated carcinomas. (B: ANOVA with repeated measurements; D: Mann-Whitney; F-I: T-test. ^{∗}, p<0.05)

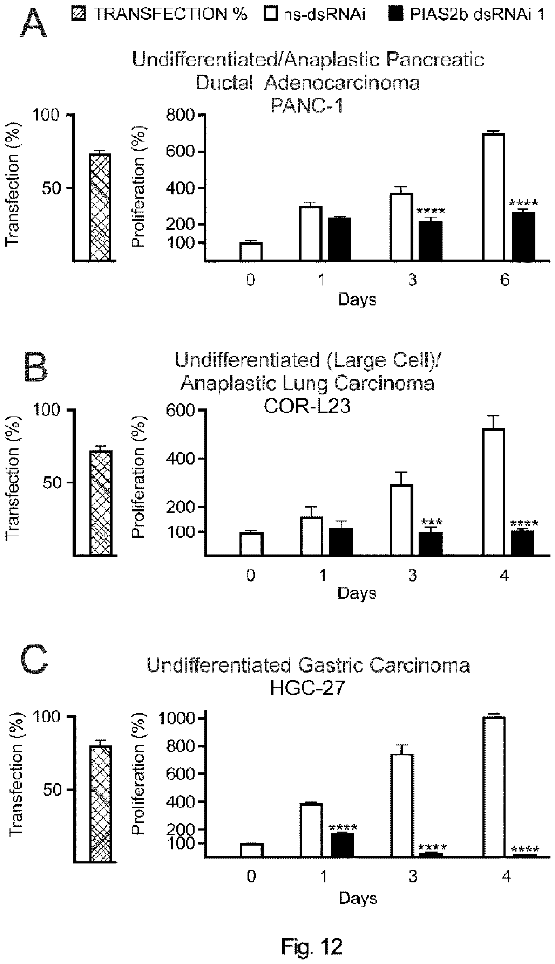

**Figure 12****.** PIAS2 dsRNAi 1 inhibits the growth of three types of non-thyroid carcinoma with the same characteristics as anaplastic thyroid carcinoma. A) Effect of PIAS2 dsRNAi 1 or its control ns-dsRNAi on the growth over days of the cells of undifferentiated/anaplastic pancreatic carcinoma PANC-1. B) Effect of PIAS2 dsRNAi 1 or its control ns-dsRNAi on the growth over days of the cells of undifferentiated/anaplastic giant-cell lung carcinoma COR-L23. These cells grow so fast that after three days in the same well the controls are already floating, exceeding the growth surface. C) Effect of PIAS2 dsRNAi 1 or its control ns-dsRNAi on the growth over days of the cells of undifferentiated gastric carcinoma HGC-27.

### DETAILED DESCRIPTION OF THE INVENTION

The inventors have identified that inhibition of the expression of PIAS2β in cells of anaplastic thyroid carcinoma and in cells of poorly differentiated thyroid carcinoma exhibits a cytotoxic effect on said cells and inhibits their proliferation. Furthermore, the administration of an inhibitor of the expression of PIAS2β to a mouse model of anaplastic carcinoma reduces tumour growth.

### 1- Nucleic acid of the invention

In a first aspect, the invention relates to a nucleic acid comprising an antisense nucleotide sequence complementary to a target region of the PIAS2β mRNA, wherein the binding of the nucleic acid to its target sequence causes inhibition of the expression of the PIAS2β mRNA.

The expression "nucleotide sequence", "nucleic acid", "polynucleotide" are used interchangeably in the present invention to refer to the polymeric form of the phosphate ester of ribonucleosides (adenosine, guanosine, uridine, or cytidine; "RNA molecules") or deoxyribonucleosides (deoxyadenosine, deoxyguanosine, deoxythymidine, or deoxycytidine; "DNA molecules") or any phosphoester analogue thereof, such as phosphorothioates and thioesters, in the form of a single strand or a double strand. Helices formed by DNA-DNA, DNA-RNA, and RNA-RNA are therefore possible. The term "nucleic acid sequences" or "nucleic acid", and particularly DNA or RNA molecule, refers only to the primary or secondary structure of the molecule and is not limited to any particular type of tertiary structure. This term therefore encompasses single-stranded or double-stranded DNA or RNA, as seen in linear or circular DNA molecules, plasmids DNA supercoil, and chromosomes, or interference RNA molecules.

The expression "nucleic acid comprising a sequence" refers to a nucleic acid molecule comprising or consisting of a specific nucleotide sequence, as defined immediately above. Said nucleic acid can be single-stranded or double-stranded.

As used in the present invention, "nucleic acid comprising an antisense nucleotide sequence" or "antisense nucleic acid" refers to a nucleic acid comprising, substantially comprising, or consisting of an antisense sequence which is single-stranded or double-stranded. In the case where it is single-stranded, the nucleic acid consists of a single chain nucleotide comprising, substantially comprising, or consisting of the antisense nucleotide sequence, and it is preferably referred to as "antisense oligonucleotide". In the case where the nucleic acid is double-stranded, in addition to comprising a first nucleotide chain comprising, substantially comprising, or consisting of an antisense sequence, it comprises a second chain complementary to the first chain. In a preferred embodiment, said second chain comprises a sense sequence complementary to the antisense sequence comprised in the first nucleic acid chain.

The nucleic acids of the invention comprising the antisense sequence may consist of 5, 10, 15, 20, 25, 30, 35, 40, 45, 50 or more nucleotides in length.

As used in the present invention, "antisense nucleotide sequence", "antisense nucleotidic sequence" or "antisense sequence" refers to a nucleic acid sequence which hybridises with, or is complementary to, a specific single-stranded target sequence (RNA or DNA) in the 3'-5' direction. In this case, the antisense sequence is complementary to a region of the mRNA encoding PIAS2β, which comprises or consists of the target sequence, or a functionally equivalent variant of said region or sequence (target sequence). The antisense sequence can be complementary to a complete coding region or to a region thereof of a specific mRNA, or to an untranslated 5' or 3' region thereof, or a region including the coding region and a sequence of the untranslated 5' or 3' region thereof. In a particular embodiment, the antisense sequence is complementary to the target sequence in at least 30%, 35%, 40%, 45%, 50%, 55; 60%, 65%, 70%, 75%, 80%, 85%, 90%, 95%, 7%, 99%, 99,9%, 99,99%, 100% of the nucleotides thereof. The methods used for calculating the percentage of complementarity between sequences are described in the definition of "complementary sequence".

In a particular embodiment, the percentage of complementarity between the two sequences is calculated with respect to the entire antisense sequence. In a particular embodiment, it is calculated with respect to the entire target sequence. In another particular embodiment, it is calculated with respect to the entire antisense sequence or the target sequence.

The nucleic acids of the invention can be obtained by chemical synthesis or by enzymatic binding reactions widely known to one skilled in the art. The capacity to derive an antisense nucleotide sequence, based on a cDNA sequence corresponding to the mRNA encoding a specific protein is described, for example, in Stein and Cohen, Cancer Res. 48:2659, (1988) and van der Krol et al., BioTechniques 6:958, (1988).

An antisense nucleic acid, an antisense oligonucleotide, or an antisense sequence may further contain modified nucleotides which increase its biological stability or the stability of double-stranded RNA-RNA or DNA-RNA complexes formed between the antisense nucleotidic sequence and the sequence of target nucleic acids, or between the antisense sequence of the nucleic acid and the sense sequence in the case of double-stranded nucleic acids, such as phosphorothioate derivatives, peptide nucleic acids, and acridine-substituted oligonucleotides. Modified nucleic acids which can be used for the preparation of antisense nucleic acid include 5-fluorouracil, 5-bromouracil, 5-chlorouracil, 5-iodo uracil, hypoxanthine, xanthine, 4-acetyl-cytosine, 5-(carboxyhydroxylmethyl)uracil, 5-carboxymethylaminomethyl -2-thiouridine, uracil 5-carboxymethyl-aminomethyl, dihydrouracil, beta-D-galactosylqueosine, inosine, N6-isopentenyladenine, 1-methylguanine, 1-methylinosine, 2,2-dimethylguanine, 2-methyladenine, 2-methylguanine, 3-methylcytosine, 5-methylcytosine, N6-adenine, 7-methylguanine, 5-methylaminomethyluracil, 5-methoxyaminomethyl-2-thiouracil, beta-D-mannosylqueosine, 5'-methoxycarboxymethyluracil, 5-methoxyuracil, 2-methylthio-N6-isopentenyladenine, uracil 5-oxyacetic acid, pseudouracil, queosine, 2 - thiocytosine, 5-methyl-2-thiouracil, 2-thiouracil, 4-thiouracil, 5-methyluracil, uracil-5-oxyacetic acid methyl ester, 5-methyl-2-thiouracil, 3-(3-amino-3-N-2-carboxypropyl)uracil, and 2,6-diaminopurine. Alternatively, the antisense nucleic acid can be produced biologically using an expression vector in which the directed antisense nucleic acid has been cloned.

Alternatively, the nucleic acid may comprise in any of the chains comprising the antisense and/or sense sequence thereof, one or more of the following modifications in one or more residues of the nucleic acid backbone: sugar modifications at 2' such as 2'-O-methyl (2'-OMe), 2'-O-methoxyethyl (2'-MOE), 2'-O-methoxyethoxy, 2'-fluoro (2'-F), 2'-allyl, 2'-O-[2-(methylamino)-2-oxoethyl], 2'-O-(N-methylcarbamate); sugar modifications at 4' including 4'-thio, 4'-CH2-O-2' bridge, 4-(CH2)2-O-2' bridge; closed nucleic acid (LNA, locked nucleic acid); peptide nucleic acid (PNA); intercalating nucleic acid (INA); twisted intercalating nucleic acid (TINA); hexitol nucleic acids (HNA); arabinonucleic acid (ANA); cyclohexane nucleic acids (CNA); cyclohexenyl nucleic acid (CeNA); threosyl nucleic acid (TNA); morpholino oligonucleotides; gapmers; mixmers; incorporation of arginine-rich peptides; addition of 5'-phosphate to synthetic RNAs; RNA aptamers (Que-Gewirth NS, Gene Ther. 2007 Feb;14(4):283-91.); RNA aptamers regulated with antidotes on the subject of the specific RNA aptamer (Oney S., Oligonucleotides. 2007 Fall; 17(3):265-74), or any combination thereof.

Modifications of one or more nucleoside linkages of the nucleic acids may comprise one or more of the following: phosphorothioate, phosphoramidate, phosphorodiamidate, phosphorodithioate, phosphoroselenoate, phosphorodiselenoate, phosphoroanilothioate, and phosphoranilidate, or any combination thereof.

As used in the present invention, "a sense nucleotide sequence" refers to a sequence capable of hybridising with, or is complementary to, the antisense sequence. In a preferred embodiment, the sense sequence is that one whose sequence is complementary to the antisense sequence in all the nucleotides thereof, i.e., in which all the nucleotides are complementary to all the nucleotides of all or part of the antisense sequence. Like the antisense sequence, the sense sequence can be made up of RNA or DNA. It may consist of 5, 10, 15, 20, 25, 30, 35, 40, 45, 50, or more nucleotides in length. In a particular embodiment, it is made up of the same number of nucleotides as the antisense sequence. In another particular embodiment, it is made up of 1, 2, 3, 4, 5, 6, 7, 8, 9, 10, 15, 20, 25, or more nucleotides than the antisense sequence. In another particular embodiment, it contains 1, 2, 3, 4, 5, 6, 7, 8, 9, 10, 15, 20, 25, or more, nucleotides less than the antisense sequence.

The term "complementary" includes within its scope nucleic acid sequences which are "completely complementary", "substantially complementary", or "partially complementary". As used herein, the term "completely complementary" indicates that 100 percent of the nucleotides in a polynucleotide can participate in base pairing with another polynucleotide. As used herein, the term "substantially complementary" indicates that at least about 65 percent, 70 percent, 75 percent, 80 percent, 85 percent, 90 percent, 95 percent, 96 percent, 97 percent, 98 percent, or 99 percent of the nucleotides in a particular polynucleotide can participate in base pairing with another polynucleotide. As used herein, the term "partially complementary" indicates that at least about 50 percent, 55 percent, or 60 percent of the nucleotides in a particular polynucleotide can participate in base pairing with another polynucleotide

The particular and preferred embodiments indicated in the definition of "a sequence capable of hybridising" with a second sequence, or "a sequence complementary" to a second sequence, apply to the sense sequence, in which the first sequence is the sense sequence and the second sequence is the antisense sequence.

As used in the present invention, "a sequence capable of hybridising" with a second sequence, or "a sequence complementary" to a second sequence, refers to a sequence, preferably named as the first sequence, comprising a sufficient number of nucleotides complementary to the nucleotides of the second sequence, such that they form a double-stranded nucleotide sequence. A sequence which hybridizes with a second sequence is that one capable of hybridising with the second sequence under little restrictive, moderately restrictive, or highly restrictive conditions.

In a particular embodiment, typical highly restrictive hybridisation conditions include incubation in 6 X SSC (1 X SSC: NaCl 0.15 M, sodium citrate 0.015 M) and 40% formamide at 42 0C for 14 hours, followed by one or more washing cycles using 0.5 X SSC, 0.1% SDS at 600C. Alternatively, highly restrictive conditions include those comprising hybridisation at a temperature of about 50°-55° C in 6X SSC and a final washing at a temperature of 68° C in 1-3 X SSC. Moderately restrictive conditions comprise hybridisation at a temperature of about 50° C to 65° C in NaCl 0.2 or 0.3 M, followed by washing at about 50° C to 55° C in 0.2X SSC, 0.1% SDS (sodium dodecyl sulphate). Generally, unrestrictive hybridisation is a hybridisation performed at 40°C in 10x SSC, or in an equivalent solution.

Alternatively, methods for determining the capacity of a first sequence to hybridise with a second sequence have been described, among others, in Pearson W.R. (Curr Protoc Bioinformatics. 2013 Jun; 03: 10.1002/0471250953.bi0301s42. doi: 10.1002/0471250953.bi0301s42), Pervouchine, D.D. (RNA. 2014 Oct; 20(10): 1519-1531. doi: 10.1261/rna.045088.114), Xie et al. (BMC Biol. 2019; 17: 62. doi: 10.1186/s12915-019-0679-8), or Elliot et al. (Nat. Commun. 2019; 10: 3401). There are different algorithms which allow determining the melting temperature of the hybrid formed by a first sequence and a second sequence such as, for example, the Oligo Calc algorithm, accessible at http://biotools.nubic.northwestern.edu/OligoCalc.html.

In a preferred embodiment, the sequence capable of hybridising with a second sequence, preferably referred to as a first sequence, is that one whose sequence is complementary to the second sequence in all the nucleotides thereof, i.e., in which all the nucleotides are complementary to all the nucleotides of all or part of the second sequence. In a preferred embodiment, the sequence complementary to the second sequence, preferably named as first sequence, is complementary in at least 30%, 35%, 40%, 45%, 50%, 55; 60%, 65%, 70%, 75%, 80%,85%, 90%, 95%, 7%, 99%, 99.9%, 99.99%, 100% of the nucleotides thereof.

In a particular embodiment, the percentage of complementarity between the first sequence, or the complementary sequence, and the second sequence is calculated with respect to the entire sequence of the first sequence. In a particular embodiment, it is calculated with respect to the entire sequence of the second sequence. In another particular embodiment, it is calculated with respect to the entire sequence of the first sequence or of the second sequence.

In a particular embodiment, the methods for calculating the percentage of complementarity between two sequences, i.e., between a first sequence complementary to a second sequence, are known to one skilled in the art, taking into account that for an RNA sequence, uracils with complementary to adenines, and vice versa, and cytosines to guanines, and vice versa. When calculating complementarity for a DNA sequence, thymines are complementary to adenines, and vice versa, and cytosines to guanines, and vice versa. In this sense, the percentage of complementarity can be calculated such that the percentage of complementarity "X" of a first sequence with respect to a second sequence is 100x(Y/Z), where Y is the number of nucleotides which are complementary to those of the second sequence (visually identified, i.e., manually or using a specific algorithm), and Z is the total number of nucleotides, or the length, of the second sequence. In a particular embodiment, in the case where the length of the two sequences is different, it is calculated with respect to the length of the region of each of the sequences which shows complementarity.

As used in the present invention, the expression "target sequence" refers to a nucleotide sequence of interest. In the particular case of the present invention, the target sequence is a sequence of the PIAS2β mRNA. Target region is understood as the area in which the target sequence is located.

In the present invention, the expression "inhibition of the expression of the PIAS2β mRNA" refers to a reduction of the expression of the PIAS2β mRNA in a cell, i.e., a reduction in the translation of said mRNA. Said reduction can be due to an inhibition of the translation of said mRNA specifically or to a degradation of the PIAS2β mRNAs present in the cell, or even to a reduction in the synthesis of said mRNAs by the cell. Methods suitable for determining if inhibition of the expression of the PIAS2β mRNA has occurred include, without limitation, standard assays for determining mRNA expression levels such as qPCR, RT-PCR, RNA protection analysis, Northern blot analysis, RNA immunoblot, *in situ* hybridisation, microarray technology, tag-based methods such as serial analysis of gene expression (SAGE) including variants such as LongSAGE and SuperSAGE, microarrays, fluorescence *in situ* hybridisation (FISH), including variants such as Flow-FISH, qFISH and double-fusion FISH (D-FISH), and the like. Assays intended for determining the amount of the PIAS2β protein expressed in a cell as an indirect indication of the PIAS2β mRNA translation level are also included. Said assays include quantification of the protein present in a cell, group of cells, or cell extract, by means of conventional methods, for example, using antibodies capable of binding specifically to the proteins encoded by the PIAS2β gene (or to fragments thereof containing antigenic determinants) and subsequent quantification of the resulting antibody-antigen complexes. There is a wide range of well-known assays that can be used in the present invention, which use non-labelled antibodies (primary antibody) and labelled antibodies (secondary antibodies); these techniques include, among others, Western blots or Western transfer, ELISA (enzyme-linked immunosorbent assay), RIA (radioimmunoassay), competitive EIA (enzyme immunoassay), DAS-ELISA (double antibody sandwich ELISA), immunocytochemical and immunohistochemical techniques, techniques based on the use of biochips or protein microarrays including specific antibodies or assays based on colloidal precipitation in formats such as reagent strips. Other ways of detecting and quantifying the levels of the protein of interest include affinity chromatography techniques, ligand binding assays, etc.

In a particular embodiment, inhibition of the PIAS2β mRNA consists of reducing the amount of the PIAS2β mRNA by at least 5%, at least 10%, at least 25%, at least 50%, at least 75%, at least 90%, or by 100%, and all the ranges between 5% and 100% with respect to a reference value. In another particular embodiment, inhibition of the PIAS2β mRNA consists of reducing the translation of the PIAS2β mRNA by at least 5%, at least 10%, at least 25%, at least 50%, at least 75%, at least 90%, or by 100%, and all the ranges between 5% and 100% with respect to a reference value.

In a particular embodiment, the reference value is that one measured in the absence of the nucleic acid of the invention.

In a preferred embodiment, inhibition of the PIAS2β mRNA of the preceding embodiments refers to inhibition of the mRNA of a cell, group of cells, cell extract, tissue, tissue cell extract, preferably in which the cells or tissue are from the thyroid gland, more preferably from the follicular tissue of the thyroid gland, even more preferably from a tumour of the follicular tissue of the thyroid gland, still more preferably from a carcinoma of the follicular tissue of the thyroid gland, still further preferably from a carcinoma selected from the group consisting of an anaplastic carcinoma, poorly differentiated carcinoma, and a recurrent carcinoma of the thyroid gland. In an even more preferred embodiment, inhibition of the PIAS2β mRNA of the preceding embodiments refers to inhibition of the mRNA of a cell, group of cells, cell extract, tissue, tissue cell extract, preferably in which the cells or tissue are from an anaplastic or poorly differentiated carcinoma of the thyroid gland, preferably from an anaplastic carcinoma of the thyroid gland.

In a particular embodiment, inhibition of the PIAS2β mRNA of the preceding embodiments refers to inhibition of the PIAS2β mRNA extracted from the cell, group of cells, cell extract, tissue, tissue cell extract indicated in any of the preceding embodiments.

In a particular embodiment, the nucleic acid of the invention specifically inhibits the expression of PIAS2β. The expression "specifically inhibits the expression of the PIAS2β mRNA" or "specific inhibition of the expression of the PIAS2β mRNA" refers to the inhibition of the PIAS2β mRNA, as defined above and according to any of the particular and preferred embodiments indicated above, in which the expression of the PIAS2β mRNA is furthermore inhibited without inhibiting the expression of a protein other than PIAS2β, such as a PIAS2 isoform other than PIAS2β, such as PIAS2α, or of the mRNA encoding a protein other than PIAS2β, even a PIAS2 isoform other than PIAS2β, such as PIAS2α.

In the present invention, the term "PIAS2" refers to the different isoforms of the PIAS2 (protein inhibitor of activated STAT 2) protein which are involved in the sumoylation of different proteins, such as PARK7, MDM2, or NCOA2. PIAS2 isoforms referred to in the present invention are PIAS2β, PIAS2α, and PIAS2 isoform 8 which is also known as PIAS2 NY.

In the present invention, the term "PIAS2α", "PIAS2a", "PIAS2α protein", or "isoform PIAS2α" refers to isoform α of PIAS2 which is involved in the specific sumoylation of different proteins. The PIAS2α protein of the present invention has accession number O75928-2 in the Uniprot database, in the version available on 5 May 2009, and is encoded by the mRNA with accession number ENST00000324794.11 in version 97 of July 2019 of the ENSEMBL database (EMBL-EBI).

In the present invention, the expression "PIAS2α mRNA", "PIAS2a mRNA", "mRNA of the PIAS2α protein", or "mRNA encoding the PIAS2α protein" refers to the mRNA whose sequence encodes the PIAS2α protein of the invention, defined immediately above and whose translation gives rise to the PIAS2β protein. Said mRNA has accession number ENST00000324794.11 in version 97 of July 2019 of the ENSEMBL database (EMBL-EBI). This mRNA is formed by 13 exons.

In the present invention, the term "PIAS2β", "PIAS2b", "PIAS2β protein", or "isoform PIAS2β" refers to isoform β of the PIAS2 protein which is specifically involved in the sumoylation of different proteins, non-limiting examples of said proteins are the MDM2 protein or the NCOA2 protein. The PIAS2β protein of the present invention has accession number 075928-1 in the Uniprot database, in the version available on 5 May 2009, and is encoded by the mRNA with accession number ENST00000585916.6 in version 97 of July 2019 of the ENSEMBL database (EMBL-EBI).

In the present invention, the expression "PIAS2β mRNA", "PIAS2b mRNA", "mRNA of the PIAS2β protein", or "mRNA encoding the PIAS2β protein" refers to the mRNA whose sequence encodes the PIAS2β protein of the invention defined immediately above and the translation of which gives rise to the PIAS2β protein. Said mRNA has accession number ENST00000585916.6 in version 97 of July 2019 of the ENSEMBL database. This mRNA is formed by 14 exons.

In the present invention, the term "PIAS2 isoform 8" or "PIAS2 NY" refers to isoform 8 or NY of the PIAS2 protein, with accession number NP_001310988.1 in the version of 31 May 2019 of the NCBI database and encoded by the mRNA with accession number ENST00000592212.5 in version 97 of July 2019 of the ENSEMBL database (EMBL-EBI).

In the present invention, the expression "PIAS2 isoform 8 mRNA", "PIAS2 NY mRNA", "mRNA of the PIAS2 protein isoform 8", "mRNA of the PIAS2 protein NY", "mRNA encoding the PIAS2 protein isoform 8", or "mRNA encoding the PIAS2 protein NY", refers to the mRNA whose sequence encodes the PIAS2 protein isoform 8 or PIAS2 NY of the invention, defined immediately above and whose translation gives rise to the PIAS2 protein isoform 8 or PIAS2 NY. Said mRNA has accession number ENST00000592212.5 in version 97 of July 2019 of the ENSEMBL database (EMBL-EBI). This mRNA is formed by 12 exons.

In a particular embodiment, the target sequence of the nucleic acid of the invention is located in exon 14 of the PIAS2β mRNA.

In another particular embodiment of the invention, the target sequence of the nucleic acid of the invention is selected from the group consisting of sequences SEQ ID NO. 1-5 and functionally equivalent sequences. In a preferred embodiment, the target sequence of the nucleic acid of the invention is SEQ ID NO. 1 or a functionally equivalent sequence.

As used in the present invention, the expression "functionally equivalent sequence", "functionally equivalent sequences", or "functionally equivalent variant" refers to sequence/sequences resulting from the insertion, deletion, and/or substitution of one or more nucleotides in the reference sequences, i.e., in the sequences to which the sequence/sequences is/are functionally equivalent. In a particular embodiment, it results from the insertion, deletion, and/or substitution of at least 1, 2, 3, 4, 5, 6, 7, 8, 910, 12, 15, 20, 25 nucleotides in the reference sequences.

In a preferred embodiment, the functionally variant sequence has a sequence identity of at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, or at least 99% with the reference sequence.

In a preferred embodiment, the identity of the sequence is determined along the entire length of the reference sequence. In another embodiment, the sequence identity is determined in the entire length of the variant, preferably of the functionally equivalent variant.

The terms "identity", "sequence identity", "identical", or "percentage of identity" in the context of two or more nucleotide sequences refer to two or more sequences or subsequences which are the same or have a specific percentage of nucleotides which are the same, when compared and aligned (introducing spaces, if necessary) for a maximum correspondence, without considering conservative amino acid substitutions as part of the sequence identity. The percentage of identity can be measured using sequence comparison software or algorithms or by visual means. Various algorithms and software which can be used to obtain nucleotide sequence alignments are known in the art.

The percentage of sequence identity can be determined by comparing two optimally aligned sequences over a comparison window. The aligned sequences can be polynucleotide sequences or polypeptide sequences. For optimal alignment of the two sequences, the portion of the polynucleotide sequence in the comparison window may comprise insertions or deletions (i.e., gaps) in comparison with the reference sequence (which does not comprise insertions or deletions). The percentage of sequence identity is calculated by determining the number of positions in which identical nucleotide residues occur in both compared sequences to obtain the number of matched positions, then dividing the number of matched positions by the total number of positions in the comparison window and multiplying the result by 100 to obtain the percentage of sequence identity. In a particular embodiment, the sequence identity between two polypeptide sequences or two polynucleotide sequences can be determined, for example, using the programme which is developed by the National Center for Biotechnology Information, NCBI, Bethesda, Maryland, USA (U.S. National Library of Medicine 8600 Rockville Pike, Bethesda MD, 20894 USA) available free of charge for the scientific community at the web page (https://blast.ncbi.nlm.nih.gov/Blast.cgi?CMD=Web&PAGE_TYPE=BlastHome) and uses the algorithm known as Basic Local Alignment Search Tool (acronym BLAST, registered trademark of the U.S. National Library of Medicine). The BLAST algorithm has different indices indicating how identical or different a sequence is due either to its composition or else to the order of its elements. Among others, BLAST has a specific section "Nucleotide BLAST" (BLASTn) for short-chain nucleotides, another specific section "Protein BLAST" (BLASTp) for peptides and proteins. In a particular embodiment, the sequence identity between two polypeptide sequences can be determined using the BLAST programme (particularly BLASTp) or the Gap programme in WISCONSIN PACKAGE version 10.0-UNIX from Genetics Computer Group, Inc., based on the Needleman and Wunsch method (J. Mol. Biol. 48: 443-453, 1970) using the set of predetermined parameters for pairwise comparison (for the comparison of amino acid sequences: gap creation penalty = 8, gap extension penalty = 2; for the comparison of nucleotide sequences: gap creation penalty = 50; gap extension penalty = 3), or using the TBLASTN programme in the BLAST 2.2.1 software package (Altschul et al., Nucleic Acids Res. 25: 3389-3402), using the BLOSUM62 matrix (Henikoff and Henikoff, Proc. Natl. Acad. Sci. USA 89: 10915-10919, 1992) and the set of predetermined parameters for pairwise comparison (gap creation cost = 11, gap extension cost = 1).

Alternatively, the degree of identity between two sequences can be determined using the ENSEML algorithm as described in https://www.ensembl.org/info/docs/index.html and https://es.wikipedia.org/wiki/Ensembl

In another particular embodiment, the percentage of sequence identity between polynucleotides or nucleic acid sequences, and their corresponding functions can be determined, for example, using a variety of homology-based search algorithms which are available for comparing a sequence of interest, with a nucleotide sequence database, including for example, BLAST (particularly BLASTN), FASTA, CLUSTAL OMEGA, EMBOSS Needle, EMBOSS Stretcher, or ENSEMBL. The percentage of identity refers to the percentage of identically paired nucleotide residues existing along the portion of the sequences which is aligned by the BLAST algorithm. By using the BLAST programme, in a particular embodiment, the alignment yielding a higher value of sequence identity is chosen from among the different alignments provided by the programme. A percentage of sequence identity or a coincidence between the analysed sequences is considered "high if the percentage of sequence identity is of at least 70%; a percentage of sequence identity from 35% to 70% is considered average; and a percentage of sequence identity less than 35% is considered low. In a preferred embodiment, the sequence identity is determined along the entire length of the reference sequence. In another embodiment, the sequence identity is determined with respect to the entire length of the variant, preferably of the functionally equivalent sequence. In another particular embodiment, the sequence identity is determined with respect to the entire length of the reference sequence or of the functionally equivalent sequence.

In a particular embodiment, the definitions and particular embodiments indicated in the definition of "functionally equivalent sequence" and in "percentage of identity" apply to the sequences functionally equivalent to the target sequence of the PIAS2β mRNA, wherein the reference sequence is the target sequence of the PIAS2β mRNA, preferably sequences SEQ ID NO 1-5, more preferably the sequence SEQ ID NO 1. Methods which allow measuring the sequence identity of a sequence have been indicated above in the definition of "identity", "identical", or "percentage of identity".

Preferably, functionally equivalent target sequences are those whose sequence allows them to hybridise in highly restrictive conditions with the antisense nucleic acid of the invention. Typical highly restrictive hybridisation conditions have been indicated above in the definition of "sequences capable of hybridising".

In a particular embodiment, the functionally equivalent sequence maintains the function of the reference sequence, preferably the sequence to which it is functionally equivalent, by at least in 10%, 15%, 20%, 25%, 30%, 35%, 40%, 45%, 50%, 55%, 60%, 65%, 70%, 75%, 80%, 85%, 90%, 95%, 100%, 120%, 150%, 180%, 200%, 250%, 300%, 350%, 400%, or more.

In the case of the sequence functionally equivalent to the target sequence of the PIAS2β mRNA, preferably to a sequence selected from the group consisting of sequences SEQ ID NO. 1-5, the function that they maintain is the function of said target sequences. In a particular embodiment, the function of said sequences is to encode a region of the functional PIAS2β protein. Therefore, the function of the PIAS2β protein encoded by the mRNA which does not have said sequences, is affected. Therefore, methods suitable for determining whether a sequence maintains the function of the target sequences in the PIAS2β mRNA may consist of determining whether an mRNA which does not contain said functionally equivalent sequences maintains the sumoylation capacity of PIAS2β. Non-limiting examples of methods which allow determining whether a protein maintains the SUMOylation capacity of PIAS2β include determining the SUMOylation capacity of the MDM2, NCOA2, and/or MDA5 proteins, using widely known assays such as those described in El Motiam A. et al, FASEB Journal, 2019, 33:643-651).

In a preferred embodiment, the nucleic acid of the invention is selected from the group consisting of a double-stranded RNA interference nucleic acid, an antisense oligonucleotide, a gapmer, a PNA, an LNA, an INA, an HNA, a morpholino, a ribozyme, and an aptamer.

In a preferred embodiment, the nucleic acid of the invention is selected from the group consisting of a small interfering RNA (siRNA), a short hairpin RNA (shRNA), and a microRNA (miRNA).

In a preferred embodiment, the nucleic acid of the invention is a small interfering RNA.

As used in the present invention, "double-stranded RNA interference nucleic acid", "double-stranded nucleic acid" refers to a double-stranded nucleic acid, i.e., made up of two hybridising or complementary nucleic acids chains which induce the RNA interference pathway. In a preferred embodiment, the nucleic acid of the invention comprises a first chain comprising, substantially comprising, or consisting of a sense sequence and a second chain comprising, substantially comprising, or consisting of an antisense sequence. In a particular embodiment, the double-stranded interference nucleic acids of the present invention include the double-stranded nucleic acids which consist of or give rise to a small interfering RNA (siRNA), short hairpin RNA (shRNA), or a microRNA (miRNA). The particular embodiments indicated in the definition of "a sequence capable of hybridising" to a second sequence or "a complementary sequence" apply to the "double-stranded RNA interference nucleic acid", wherein the first sequence is that one comprising the sense sequence and the second sequence is that one comprising the antisense sequence.

The term small interfering RNA ("siRNA") refers to the duplex of small inhibitory RNAs which induce the RNA interference pathway. The length of these molecules may vary (generally 18-30 base pairs), and they contain variable degrees of complementarity to their target mRNA in the antisense chain. Some but not all siRNAs have unpaired bases overhanging from the 5' or 3' end of the sense strand and/or the antisense strand. The term "siRNA" includes a duplex of two separated chains. As used herein, siRNA molecules are not limited to RNA molecules, but also further include nucleic acids with one or more chemically modified nucleotides, such as morpholinos.

As used herein, the term "shRNA" or "short hairpin RNA" refers to a dsRNA in which the two chains are bound by a chain without interrupting the nucleotides between the 3' end of one strand and the 5' end of the other respective strand to form a duplex structure.

The term "microRNA" or "miRNA" refers to short, single-stranded RNA molecules, typically about 21-23 nucleotides in length, which are capable of regulating gene expression. miRNAs can be synthetic (i.e., recombinant) or natural. Natural miRNAs are encoded by genes which are transcribed from DNA and processed from primary transcripts ("pri-miRNA") into short stem-loop structures ("pre-miRNA") and finally into a mature miRNA. Mature miRNA molecules are partially complementary to one or more mRNA molecules and reduce gene expression through a process similar to RNA interference or by inhibiting mRNA translation.

The expression "antisense oligonucleotide" has been defined above in relation to the nucleic acid comprising an antisense sequence.

As used herein, the term "ribozyme" or "RNA enzyme", or "catalytic RNA" refers to a RNA molecule which catalyses a chemical reaction. Many natural ribozymes catalyse the hydrolysis of one or more of their own phosphodiester bonds or the hydrolysis of bonds in other RNAs; but they have also been observed to catalyse the aminotransferase activity of a ribosome, the ligase activity of a DNA ligase, and a number of other chemical reactions performed by conventional protein enzymes.

As used herein, an "aptamer" refers to a nucleic acid ligand which binds to more than one site in a target molecule, wherein the binding is not "complementary", i.e., not due to the formation of base pairs between a nucleic acid ligand and a target nucleic acid sequence. An aptamer which binds to any foreseeable target, including polypeptides, can be designed. Aptamers are useful for biotechnological and therapeutic applications as they offer molecular recognition properties which rival that of the biomolecule that is normally used, i.e., antibodies. In addition to selective recognition, aptamers exhibit advantages over antibodies because they can be manipulated entirely in a test tube and are readily produced by means of chemical synthesis, have desirable storage properties, and elicit little to no immunogenicity in therapeutic applications. Aptamers can be synthesised by means of repeated rounds *of in vitro* distribution, selection, and amplification, a methodology known in the state of the art as "SELEX" (systematic evolution of ligands by exponential enrichment) (Shamah et al, Acc. Chem. Res. 2008, 41 pp. 130-8). Alternatively, they can be synthesised, for example, by means of stepwise solid phase.

A locked nucleic acid (LNA), often referred to as inaccessible RNA, is a modified RNA nucleotide. The ribose group of a LNA nucleotide is modified with an extra bridge joining carbons 2' and 4' (02',C4'-methylene bridge). The bridge "closes" the ribose in the 3'-endo structural conformation which is often found in the A form of DNA or RNA. LNA nucleotides can be mixed with DNA or RNA bases in the nucleic acid when desired. Such oligomers are commercially available. The closed conformation of the ribose increases base stacking and backbone pre-organization. This significantly increases thermal stability (melting temperature) and the hybridisation affinity of modified nucleic acids with LNA, in addition to having improved capacities in discriminating poor pairings. These properties make aptamers extremely useful for antisense techniques. Furthermore, anti-miR LNA oligonucleotides have been tested in primates with encouraging results and a low toxicity.

A peptide nucleic acid (PNA) is an artificially synthesised DNA- or RNA-like polymer used in biological research and medical treatments. PNA is not known to occur naturally. DNA and RNA have a deoxyribose and ribose sugar backbone, respectively, whereas the backbone of PNA is a compound with repeating units of N-(2-aminoethyl)-glycine joined by peptide bonds. The different purine and pyrimidine bases are joined to the backbone by methylene-carbonyl bonds. PNAs are depicted as peptides, with the N-terminal in the first position (left) and the C-terminal on the right. Given that the backbone of PNA does not contains charged phosphate groups, binding between PNA/DNA strands is stronger than between DNA/DNA strands due to the lack of electrostatic repulsion. The mixed base molecules of PNA are true DNA molecule mimics in terms of base pair recognition. PNA/PNA binding is stronger than PNA/DNA binding.

An intercalating nucleic acid (INA) is a modified nucleic acid analogue comprising normal deoxyribonucleotides covalently bound to hydrophobic insertions. INA has a significant affinity for the complementary DNA with stabilisation of up to 11 degrees for each modification. INA has greater specificity for a fully paired target over poorly paired targets than normal DNA. The fact that INAs have greater affinity for DNA makes it possible to use shorter probes and thereby even further increase specificity. Furthermore, an INA is a selective oligonucleotide analogue of DNA with a unique capacity to discriminate between DNA and RNA. Even though INAs have high affinities for complementary DNA, it has a lower affinity for a complementary INA sequence. Twisted intercalating nucleic acids are referred to as TINA.

Hexitol nucleic acids (HNA) are nucleotides made up of natural nucleobases and a phosphorylated 1,5-anhydrohexitol backbone. The molecular associations between HNA and RNA are more stable than between HNA and DNA and between natural nucleic acids (dsDNA, dsRNA, DNA/RNA). Other synthetically modified oligonucleotides comprise ANA (arabinonucleic acid), CNA (cyclohexane nucleic acids), CeNA (cyclohexenyl nucleic acid), and TNA (threosyl nucleic acid).

Morpholinos are synthetic molecules which are the product of redesigning the natural nucleic acid structure. Structurally, the difference between morpholinos and DNA or RNA is that while morpholinos have standard nucleobases, those bases are joined to 6-membered morpholine rings instead of to deoxyribose/ribose rings, and the non-ionic phosphorodiamidate bonds between the subunits replace the anionic phosphodiester bonds. Morpholinos are sometimes referred to as PMO (phosphorodiamidate morpholino oligonucleotide). The 6-membered morpholine ring has the chemical formula O-(CH2-CH2)2-NH

Gapmers or "gapped oligomeric compounds" are chimeric RNA-DNA-RNA oligonucleotide probes where DNA windows or 'gaps' are inserted into an otherwise normal or modified RNA oligonucleotide known as "wings". This modification increases the stability of the oligonucleotide *in vivo* and the avidity of the interaction of the probe with the target, such that shorter probes can be effectively used. Preferably, the wings are 2'-O-methyl (OMe) or 2'-O-methoxyethyl (MOE) modified oligonucleotides which protect the internal block from nuclease degradation. Furthermore, the nucleotides forming the gap or wings can be joined by phosphodiester bonds or phosphorothioate bonds, thereby making them resistant to degradation by RNase. Furthermore, the nucleotides forming the wings can also be modified by the incorporation of bases joined by 3'-methylphosphonate bonds.

In a particular embodiment, the antisense sequence of the nucleic acid of the invention is selected from the group consisting of SEQ ID NO. 6-10 and functionally equivalent sequences. In a preferred embodiment, the antisense sequence of the nucleic acid of the invention is selected from the group consisting of SEQ ID NO. 6-7 and functionally equivalent variants, more preferably SEQ ID NO. 6 or functionally equivalent variants.

In a particular embodiment, the definitions and particular embodiments indicated in the definition of "functionally equivalent sequences", as well as in the definition of "percentage of sequence identity", apply to sequences functionally equivalent to SEQ ID NO. 6-10, wherein the reference sequence is the antisense sequence as defined above; it is preferably selected from the group consisting of SEQ ID NO. 6-10, more preferably from the group consisting of SEQ ID NO. 6-7, even more preferably it is SEQ ID NO. 6.

Methods which allow measuring the sequence identity of a sequence have been indicated above in the definition of "identity", "identical", or "percentage of identity".

In a particular embodiment, the sequence functionally equivalent to the antisense sequence of the nucleic acid of the invention, the functionally equivalent sequence maintains the function of said sequence, preferably of the sequence selected from the group consisting of SEQ ID NO. 6-10, more preferably from the group consisting of SEQ ID NO. 6-7, even more preferably the sequence SEQ ID NO. 6, by at least 10%, 15%, 20%, 25%, 30%, 35%, 40%, 45%, 50%, 55%, 60%, 65%, 70%, 75%, 80%, 85%, 90%, 95%, 100%, 120%, 150%, 180%, 200%, 250%, 300%, 350%, 400%, or more. The function of the antisense sequence of the nucleic acid of the invention is to hybridise with the target sequence of the PIAS2β mRNA and inhibit the expression of the PIAS2β mRNA. Therefore, assays which allow identifying the sequences which maintain the function of the antisense sequences correspond to those indicated above for determining whether one sequence hybridises with a second sequence, in this case the target sequence in the corresponding PIAS2β mRNA (see the definition of "sequence capable of hybridising", "complementary sequence" above) and those indicated above for determining inhibition of the expression of the PIAS2β mRNA (see the definition of "inhibition of the expression of the PIAS2β mRNA"). In a particular embodiment, the function maintained by the functionally equivalent sequences of the corresponding antisense sequence is the hybridisation with the corresponding target sequence in the PIAS2β mRNA and/or inhibition of the expression of the PIAS2β mRNA, by at least 10%, 15%, 20%, 25%, 30%, 35%, 40%, 45%, 50%, 55%, 60%, 65%, 70%, 75%, 80%, 85%, 90%, 95%, a 100%, 120%, 150%, 180%, 200%, 250%, 300%, 350%, 400%, or more.

In a particular embodiment, the antisense sequence of the nucleic acid of the invention is a DNA sequence equivalent to the antisense RNA sequences defined in the preceding embodiments. In a particular embodiment, the antisense sequences of the nucleic acids of the invention are selected from the group of sequences consisting of SEQ ID NO. 16-20. In a particular embodiment, the complete nucleotide sequence of the nucleic acid of the invention is a DNA sequence. In a particular embodiment, the embodiments indicated above in relation to the antisense sequences selected from the group SEQ ID NO. 6-10 also apply to the antisense sequences selected from the group consisting of SEQ ID NO. 16-20, with references to SEQ ID NO. 6-10 in said embodiments being modified to SEQ ID NO. 16-20.

In another particular embodiment, the nucleic acid of the invention is a double-stranded RNA interference nucleic acid and further comprises a sense nucleotide sequence complementary to the antisense sequence. In a preferred embodiment, the antisense nucleotide sequence is selected from the group consisting of SEQ ID NO. 6-10 and functionally equivalent sequences, and the sense sequence is selected from the group consisting of SEQ ID NO. 11-15 and functionally equivalent sequences.

The expressions "double-stranded RNA interference nucleic acid", and "sense nucleotide sequence" and "sequences functionally equivalent to SEQ ID NO. 6-10" have been defined above.

In a particular embodiment, the definitions and particular embodiments indicated in the definitions of "functionally equivalent sequence" and of "percentage of sequence identity" apply to sequences functionally equivalent to SEQ ID NO. 11-15, wherein the reference sequence is selected from the group consisting of SEQ ID NO. 11-15, preferably from the group consisting of SEQ ID NO. 11-12, more preferably with SEQ ID NO. 11. Methods which allow measuring the sequence identity of a sequence have been indicated above in the definition of "identity", "identical", or "percentage of identity".

In a particular embodiment, the sequence functionally equivalent to the sense sequence of the nucleic acid of the invention, the functionally equivalent sequence maintains the function of said sense nucleotide sequence, preferably of the sequence selected from the group consisting of SEQ ID NO. 11-15, more preferably from the group consisting of SEQ ID NO. 11-12, even more preferably sequence SEQ ID NO. 11, by at least 10%, 15%, 20%, 25%, 30%, 35%, 40%, 45%, 50%, 55%, 60%, 65%, 70%, 75%, 80%, 85%, 90%, 95%, a 100%, 120%, 150%, 180%, 200%, 250%, 300%, 350%, 400%, or more. The function of the sense sequence of the nucleic acid of the invention is to hybridise with the antisense sequence of the nucleic acid and inhibit the expression of the PIAS2β mRNA. Therefore, assays which allow identifying the sequences which maintain the function of the sense sequences correspond to those indicated above for determining whether one sequence hybridises with a second sequence or is complementary to a second sequence which, in this case, would be the corresponding antisense sequence of the nucleic acid of the invention (see the definition of "sequence capable of hybridising", "complementary sequence" above) and those indicated above for determining inhibition of the expression of the PIAS2β mRNA (see the definition of "inhibition of the expression of the PIAS2β mRNA"). In a particular embodiment, the function maintained by the functionally equivalent sequences of the corresponding sense sequence is the hybridisation with the corresponding antisense sequence and/or the inhibition of the expression of the PIAS2β mRNA once they have hybridised with the corresponding antisense sequence, by at least 10%, 15%, 20%, 25%, 30%, 35%, 40%, 45%, 50%, 55%, 60%, 65%, 70%, 75%, 80%, 85%, 90%, 95%, a 100%, 120%, 150%, 180%, 200%, 250%, 300%, 350%, 400%, or more.

In a particular embodiment, the sense sequence of the nucleic acid of the invention is a DNA sequence equivalent to the sense RNA sequences defined in the preceding embodiments. In a particular embodiment, the sense sequences of the nucleic acids of the invention are selected from the group of sequences consisting of SEQ ID NO. 21-25. In a particular embodiment, the complete nucleotide sequence of the nucleic acid of the invention is a DNA sequence. In a particular embodiment, the embodiments indicated above in relation to the sense sequences selected from the group SEQ ID NO. 11-15 also apply to the sense sequences selected from the group consisting of SEQ ID NO. 21-25, with references to SEQ ID NO. 11-15 in said embodiments being modified to SEQ ID NO. 21-25.

In a preferred embodiment, the nucleotide sequence of the nucleic acid of the invention comprising the antisense sequence according to any of the preceding embodiments comprises a sequence with at least 1, 2, 3, 4, 5, 6, 7, 8, 9, 10, 15, 20 uracils, preferably at least 2, more preferably 2 uracils at the 3' end thereof. In a particular embodiment, the nucleotide sequence of the nucleic acid of the invention comprising the antisense sequence is DNA and the preceding embodiment applies to said nucleotide sequence, with "uracils" being replaced with "thymines".

In another preferred embodiment, the nucleotide sequence of the nucleic acid of the invention comprising the sense sequence according to any of the preceding embodiments comprises a sequence with at least 1, 2, 3, 4, 5, 6, 7, 8, 9, 10, 15, 20 uracils, preferably 20, more preferably at least 2, even more preferably 2 uracils at the 3' end thereof. In a particular embodiment, the nucleotide sequence of the nucleic acid of the invention comprising the sense sequence is DNA and the preceding embodiment applies to said nucleotide sequence, with "uracils" being replaced with "thymines".

In another preferred embodiment of the invention, the nucleic acid is a double-stranded nucleic acid, wherein both the sequence comprising the antisense sequence according to any of the preceding embodiments and the sequence comprising the sense sequence according to any of the preceding embodiments comprise a sequence with at least 1, 2, 3, 4, 5, 6, 7, 8, 9, 10, 15, 20 uracils, preferably 20, more preferably at least 2, even more preferably 2 uracils at the 3' end thereof. In a particular embodiment, the double-stranded nucleic acid is DNA and the preceding embodiment applies to said nucleic acid, with "uracils" being replaced with "thymines".

In a particular embodiment, the sequence of the nucleic acid of the invention comprising the antisense sequence and a sequence having 2 uracils at the 3' end thereof according to any of the preceding claims is selected from the group consisting of SEQ ID NO. 26-30 and functionally equivalent sequences, preferably from the group consisting of SEQ ID NO. 26-27 and functionally equivalent sequences, even more preferably from the group consisting of SEQ ID NO. 26 and functionally equivalent sequences. In the case where the sense sequence is DNA and all the preceding targeted embodiments apply, it is selected from the group consisting of SEQ ID NO. 36-40 and functionally equivalent sequences, preferably from the group consisting of SEQ ID NO. 36-37 and functionally equivalent sequences, more preferably from the group consisting of SEQ ID NO. 36 and functionally equivalent sequences.

In another particular embodiment, the sequence of the nucleic acid of the invention comprising the sense sequence which comprises two uracils at the 3' end thereof according to any of the preceding embodiments is selected from the group consisting of SEQ ID NO. 31-35 and functionally equivalent sequences, preferably from the group consisting of SEQ ID NO. 31-32 and functionally equivalent sequences, even more preferably from the group consisting of SEQ ID NO. 31 and functionally equivalent sequences. In the case where the sense sequence is DNA, it is selected from the group consisting of SEQ ID NO. 41-45 and functionally equivalent sequences, preferably from the group consisting of SEQ ID NO. 41-42 and functionally equivalent sequences, more preferably from the group consisting of SEQ ID NO. 41 and functionally equivalent sequences.

The expression "functionally equivalent sequences" in relation to variants of the antisense sequences has been defined above and the definition and particular embodiments indicated in relation to said variants of the antisense sequences apply to sequences selected from the group consisting of SEQ ID NO. 26-30 and to sequences selected from the group consisting of SEQ ID NO. 36-40.

The expression "functionally equivalent sequences" in relation to variants of the sense sequence has been defined above and the definition and particular embodiments indicated in relation to said variants of the sense sequences apply to sequences selected from the group consisting of SEQ ID NO. 31-35 and to sequences selected from the group consisting of SEQ ID NO. 41-45.

In a preferred embodiment, the target mRNA encodes the isoform PIAS2α. Therefore, all the preceding embodiments also apply to PIAS2α mRNA, with mention to PIAS2β being replaced with PIAS2α and mention to PIAS2α being replaced with PIAS2β. In a particular embodiment, the target sequence of the nucleic acid of the invention is located in exon 13 of the PIAS2α mRNA.

### 2- Vector of the invention

In a second aspect, the invention relates to a vector comprising a nucleic acid encoding the antisense sequence of the nucleic acid according to any of the preceding embodiments in relation to the first aspect of the invention. Preferably, the vector further comprises a nucleic acid encoding the sense sequence of the nucleic acid of the invention, according to any of the embodiments of the first aspect of the invention.

As used in the present invention, the term "vector" refers to a vehicle through which a polynucleotide or a DNA molecule can be manipulated or introduced into a cell. The vector can be a linear or circular polynucleotide or a polynucleotide having a large size or any other type of construct such as the DNA or RNA of a viral genome, a virion, or any other biological construct which allows the manipulation of the DNA or the introduction thereof in the cell. It is understood that the expressions "recombinant vector", "recombinant system" can be used interchangeably with the term vector. It will be apparent to one skilled in the art that there is no limitation in terms of the type of vector which can be used given that said vector can be a cloning vector suitable for propagation and for obtaining the suitable polynucleotides or gene constructs or expression vectors in different heterologous organisms suitable for conjugate purification. Therefore, suitable vectors according to the present invention include prokaryotic expression vectors such as pUC 18, pUC 19, Bluescript, and derivatives thereof, mp 1 8, mp 1 9, pBR322, pMB9, CoIEl, pCR1, RP4, phages, and "shuttle" vectors such as pSA3 and pAT28, yeast expression vectors such as vectors of the type of 2-micron plasmids, integration plasmids, YEP vectors, centromeric plasmids, and the like, insect cell expression vectors such as vectors from the pAC series and pVL series, plant expression vectors such as vectors from the pIBI, pEarleyGate, pAVA, pCAMBIA, pGSA, pGWB, pMDC, pMY, pORE series and the like, and higher eukaryotic cell expression vectors either based on viral vectors (adenovirus, adeno-associated virus, as well as retrovirus and lentivirus), as well as non-viral vectors such as pSilencer 4.1-CMV (Ambion), pcDNA3, pcDNA3.1/hyg pHCMV/Zeo, pCR3.1, pEFl/His, pIND/GS, pRc/HCMV2, pSV40/Zeo2, pTRACER-HCMV, pUB6/V5-His, pVAXl, pZeoSV2, pCI, pSVL and pKSV-10, pBPV-1, pML2d, and pTDTl.

The vector of the invention can be used to transform, transfect, or infect cells which can be transformed, transfected, or infected with said vector. Said cells can be prokaryotic or eukaryotic cells. By way of example, the vector in which said DNA sequence is introduced can be a plasmid or a vector which, when introduced in a host cell, integrates into the genome of said cell and replicates together with the chromosome (or chromosomes) into which it has integrated. Said vector can be obtained by means of conventional methods known by those skilled in the art (Joseph Sambrook, David W. Russel Eds. 2001, 3^{rd} ed. Cold Spring Harbor, New York).

In a preferred embodiment, the vector of the invention is a viral vector. In another preferred embodiment, the vector of the invention comprises the double expression of the luciferase and mCherry enzymes separated by an IRES (Internal Ribosomic Entry Site) element, such that the transcription of one luciferase molecule per each mCherry molecule is ensured. Viral infection of the population can thus be evaluated in a quick manner by means of conventional fluorescence microscopy and subsequent quantification of the tumour mass by means of luminescence can be ensured.

In a particular embodiment, all the definitions and embodiments indicated in relation to the first aspect of the invention apply to the second aspect of the invention.

### 3- Host cell of the host of the invention

In a third aspect, the invention relates to a host cell comprising the nucleic acid according to any of the embodiments of the first aspect of the invention or the vector according to any of the embodiments of the second aspect of the invention.

The host cell or hosts cells of the invention are capable of expressing or comprising the nucleic acids of the invention.

In the present invention, the term "host cell" refers to a cell comprising a nucleic acid or a vector of the invention, for which said cell could be transformed, transfected, or infected with a construct or vector provided in this invention. Transformed, transfected, or infected cells can be obtained by means of conventional methods known by those skilled in the art (Sambrook *et al.,* 2001, *ad supra,* Chenlo M. et al., EBioMedicine, 43:537-552, 2019; Garcia M. et al., Sci. Rep. 7:42937, 2017; Garcia-Rendueles A.R. et al., Oncogene, 36(5):652-666, 2017; Bravo S.B. et al., J Clin Endocrinol Metab. 98(6):2431-2441, 2013; Diaz-Rodriguez E. et al., Oncogene, 31(23):2824-35, 2012; Bravo SB et al., Anal Biochem., 400(2):219-28, 2010).

In a particular embodiment, said host cell is an animal cell transfected or infected with a suitable vector.

Suitable host cells for expression of the conjugates of the invention include, without limitation, mammalian, plant, insect, fungal, and bacterial cells. Bacterial cells include, without limitation, Gram-positive bacterial cells such as species from the genera *Bacillus, Streptomyces,* and *Staphylococcus,* and Gram-negative bacterial cells such as cells from genera *Escherichia* and *Pseudomonas.* Fungal cells preferably include yeast cells such as *Saccharomyces, Pichia pastoris,* and *Hansenula polymorpha.* Insect cells include, without limitation, Drosophila cells and Sf9 cells. Plant cells include, among others, cells from crop plants such as cereals, medicinal plants, ornamental plants, or bulb plants. Mammalian cells suitable for in the present invention include epithelial cell lines (porcine, etc.), osteosarcoma cell lines (human, etc.), neuroblastoma cell lines (human, etc.), epithelial carcinomas (human, etc.), glial cells (murine, etc.), hepatic cell lines (from monkey, etc.), CHO (Chinese Hamster Ovary) cells, COS cells, BHK cells, HeLa, 911, AT1080, A549, 293, or PER.C6 cells, human ECCs NTERA-2, D3 cells of the mESC line, human embryonic stem cells such as HS293 and BGVOl, SHEF1, SHEF2, and HS181, NIH3T3, 293T, REH, and MCF-7 cells and hMSCs cells. In a particular embodiment, the host cell of the invention is selected from the group of cells chosen from the *Cellosaurus ExpasY* database (https://web.expasy.org/cellosaurus/, version of 31 July 2019).

In a particular embodiment, all the definitions and embodiments indicated in relation to the first and second aspects of the invention apply to the third aspect of the invention.

### 4- Pharmaceutical composition of the invention

In a fourth aspect, the invention relates to a pharmaceutical composition comprising the nucleic acid of the first aspect of the invention or the vector of the second aspect of the invention and a pharmaceutically acceptable excipient.

As used herein, "pharmaceutical composition" refers to compositions and molecular entities which are physiologically tolerable and typically do not cause an allergic reaction or a similar unfavourable reaction such as gastric disorders, dizziness, and the like, when administered to a human or animal. Preferably, the expression "pharmaceutically acceptable" means that it is approved by a regulatory agency of a state or federal government or included in the US Pharmacopeia or another pharmacopeia generally recognised for use in animals, and more particularly in human beings.

The term "excipient" refers to a vehicle, diluent, or adjuvant which is administered with the active ingredient. Such pharmaceutical excipients can be sterile liquids, such as water and oils, including those of petroleum, animal, plant, or synthetic origin, such as peanut oil, soybean oil, mineral oil, sesame oil, and the like. Water or aqueous saline solutions and aqueous dextrose and glycerol solutions, particularly for injectable solutions, are preferably used as vehicles. Suitable pharmaceutical vehicles are described in "Remington Pharmaceutical Sciences" by E. W. Martin, 21st Edition, 2005; or "Handbook of Pharmaceutical Excipients", Rowe C. R.; Paul J. S.; Marian E. P., 6th edition.

Suitable pharmaceutically acceptable vehicles include, for example, water, saline solutions, alcohol, vegetable oils, polyethylene glycols, gelatine, lactose, amylose, magnesium stearate, talc, surface active agents, silicic acid, viscous paraffin, perfume oil, fatty acid monoglycerides and diglycerides, petroleum-reived fatty acid esters, hydroxymethylcellulose, polyvinylpyrrolidone and the like

In a particular embodiment, all the definitions and embodiments indicated in relation to the first and second aspects of the invention apply to the fourth aspect of the invention.

### 5- Medical uses of the invention

In a fifth aspect, the invention relates to an inhibitor of the PIAS2β protein for use in the treatment of cancer in a patient.

As used in the present invention, the term "inhibitor" refers to any compound capable of causing a decrease in the activity of the PIAS2β protein, particularly in sumoylation activity, compounds capable of reducing the expression of the PIAS2β gene, and compounds capable of reducing the levels of the PIAS2β protein. Compounds capable of reducing the expression of the PIAS2β gene include compounds having the ability to reduce the expression of the PIAS2β gene by reducing the transcription thereof, compounds having the ability to reduce or inhibit the expression of PIAS2 β mRNA, and compounds with the ability to inhibit the PIAS2 β protein.

In a particular embodiment, the inhibitor of the PIAS2β protein is a compound capable of inhibiting the expression of the PIAS2β mRNA. The definition and particular embodiments indicated in the first aspect of the invention in the definition of "inhibition of the expression of the PIAS2β mRNA" and in relation to inhibition of the expression of the PIAS2β mRNA by the nucleic acid of the invention, apply to the inhibitor as used in the present aspect of the invention, with the term "nucleic acid" being replaced by "inhibitor". In this sense, the reference value in relation to the present aspect of the invention is that one measured in the absence of the inhibitor of the invention.

In a particular embodiment, the inhibitor of the invention specifically inhibits the expression of the PIAS2β mRNA. The definition and particular embodiments indicated in the first aspect of the invention in the definition of "specifically inhibits the expression of the PIAS2β mRNA" apply to the present aspect of the invention, wherein the nucleic acid of the invention is replaced by the inhibitor of the invention.

Methods suitable for determining whether a PIAS2β inhibitor is capable of reducing the expression of the PIAS2β mRNA have been indicated in the first aspect of the invention.

Inhibitors inhibiting the PIAS2β protein are those capable of specifically reducing the amount of the PIAS2β protein by at least 5%, at least 10%, at least 25%, at least 50%, at least 75%, at least 90%, or by 100%, and all the ranges between 5% and 100% with respect to a reference value. In another particular embodiment, the inhibition of the PIAS2β protein consists of reducing the activity of the PIAS2β protein by at least 5%, at least 10%, at least 25%, at least 50%, at least 75%, at least 90%, or by 100%, and all the ranges between 5% and 100% with respect to a reference value.

In a particular embodiment, the reference value is that one measured in the absence of the inhibitor of the invention.

In a preferred embodiment, inhibition of the PIAS2β protein of the preceding embodiments refers to inhibition of the PIAS2β protein of a cell, group of cells, cell extract, tissue, tissue cell extract, preferably in which the cells or tissue are from the thyroid gland, more preferably from the follicular tissue of the thyroid gland, even more preferably from a tumour of the follicular tissue of the thyroid gland, still more preferably from a carcinoma of the follicular tissue of the thyroid gland, still further preferably from a carcinoma selected from the group consisting of an anaplastic carcinoma, poorly differentiated carcinoma, and a recurrent carcinoma of the thyroid gland. In an even more preferred embodiment, inhibition of the PIAS2β protein of the preceding embodiments refers to inhibition of the PIAS2β protein of a cell, group of cells, cell extract, tissue, tissue cell extract, preferably in which the cells or tissue are from an anaplastic or poorly differentiated carcinoma of the thyroid gland, preferably from an anaplastic carcinoma of the thyroid gland.

In a particular embodiment, inhibition of the PIAS2β protein of the preceding embodiments refers to inhibition of the PIAS2β protein extracted from the cell, group of cells, cell extract, tissue, tissue cell extract which are indicated in any of the preceding embodiments.

In a particular embodiment, the inhibitor of the invention specifically inhibits the PIAS2β protein. The expression "specifically inhibits the PIAS2β protein" or "specific inhibition of the PIAS2β protein" refers to inhibition of the PIAS2β protein as defined above and according to any of the particular and preferred embodiments indicated above, wherein the PIAS2β protein is furthermore inhibited without inhibiting a protein other than PIAS2B, such as a PIAS2 isoform other than PIAS2B, such as PIAS2α.

Suitable methods for determining whether an inhibitor acts by reducing the levels or the function of the PIAS2β protein include quantification by means of conventional methods, for example, using antibodies capable of binding specifically to the proteins encoded by the PIAS2β gene (or to fragments thereof containing antigenic determinants) and subsequent quantification of the resulting antibody-antigen complexes. There is a wide range of well-known assays that can be used in the present invention, which use non-labelled antibodies (primary antibody) and labelled antibodies (secondary antibodies); these techniques include, among others, Western blots or Western transfer, ELISA (enzyme-linked immunosorbent assay), RIA (radioimmunoassay), competitive EIA (enzyme immunoassay), DAS-ELISA (double antibody sandwich ELISA), immunocytochemical and immunohistochemical techniques, techniques based on the use of biochips or protein microarrays including specific antibodies or assays based on colloidal precipitation in formats such as reagent strips. Other ways of detecting and quantifying the levels of the protein of interest include affinity chromatography techniques, ligand binding assays, etc.

In another embodiment, the specific PIAS2β inhibitor acts by inhibiting the activity of PIAS2β. A specific PIAS2β inhibitor for use in the present invention can specifically inhibit the activity of PIAS2β by at least 5%, at least 10%, at least 25%, at least 50%, at least 75%, or at least 90%, and all the ranges between 5% and 100%. Given that PIAS2β is a Sumo E3 ligase, methods suitable for determining whether an inhibitor acts by reducing the activity PIAS2β include any method which allows the determination of sumoylation, such as by means of the determination of MDA5 sumoylation, using widely known assays such as those described in El Motiam A. et al, FASEB Journal, 2019, 33, 643-649.

In a particular embodiment, the PIAS2β inhibitor is cytotoxic in tumour cells, preferably in thyroid cancer cells, more preferably in cells selected from the group consisting of anaplastic cancer cells, poorly differentiated cancer cells, recurrent thyroid cancer cells, and combinations thereof. In a preferred embodiment, the PIAS2β inhibitor is cytotoxic in anaplastic thyroid cancer cells and in poorly differentiated thyroid cancer cells, preferably in anaplastic thyroid cancer cells.

In the present invention, the term "cytotoxic" or "cytotoxic effect" refers to the capacity of a compound, preferably the inhibitor of the invention, of being toxic for specific cells, i.e., of inducing the death of said cells, preferably tumour cells.

As used in the present invention, the expression "tumour cells" refers to cells originating from a specific tumour or cancer, either those present in a growing tumour in the body of a patient or cells growing in a cell culture and originating from the tumour of a patient. In a preferred embodiment, the tumour cells originate from a thyroid cancer selected from the group consisting of anaplastic cancer, poorly differentiated cancer, and recurrent thyroid cancer. More preferably, they originate from anaplastic cancer or poorly differentiated thyroid cancer, even more preferably from anaplastic thyroid cancer.

In a particular embodiment, the PIAS2β inhibitor is selected from the group consisting of the nucleic acids of the first aspect of the invention, an antibody specifically recognising PIAS2β, or an aptamer specifically recognising PIAS2β.

In the present invention, the expression "capable of specifically recognising PIAS2β" refers to the ability of a molecule for recognising PIAS2β but not another protein, such as another PIAS2 isoform, such as PIAS2α.

As used herein, the term "treatment" refers to any type of therapy the purpose of which is to end, improve, or reduce the susceptibility to suffering a clinical condition as described herein. Therefore, "treatment", "treat", and equivalent terms thereof refer to the attainment of a desired pharmacological or physiological effect, covering any treatment of a pathological condition or disorder in a mammal, including human beings. The effect can be prophylactic in terms of providing total or partial prevention of a disorder and/or an adverse effect which can be attributable to it. That is,, "treatment" includes (1) inhibiting the disease, for example, stopping its development, (2) interrupting or ending the disorder or at least the symptoms associated with it, so that the patient would no longer suffer the disease or its symptoms, for example, causing the regression of the disease or its symptoms by means of restoring or repairing a lost, missing, or defective function, or stimulating an inefficient process, or (3) slowing down, alleviating, or improving the disease or the symptoms associated with it, wherein slowing down is used in a broad sense to refer at least to a reduction in the magnitude of a parameter, such as inflammation, pain, or immune deficiency.

The term "patient", "subject", "individual" referred to in the present invention applies to a patient with cancer, wherein the patient is a member of a mammalian animal species and includes, but is not limited to, domestic animals, primates, and humans; the subject is preferably a male or female human being of any age or race.

For the use in the prevention and/or treatment according to the invention, the inhibitor is present in an effective amount.

The term "effective" amount or a "therapeutically effective amount" of a drug or pharmacologically active agent is understood as an amount of the drug or agent that is nontoxic but sufficient to provide the desired effect. In the combination therapy of the present invention, an "effective amount" of a component of the combination is the amount of that compound which is effective in providing the desired effect when used in combination with the other components of the combination.

Although individual needs vary, determination of the optimal ranges for the effective amounts of the agent of the invention falls within the common experience of those skilled in the art. Generally, the dose needed to provide an effective amount of said compound, which can be adjusted by one skilled in the art, will vary depending on age, health, physical condition, sex, diet, weight, treatment frequency, and the nature and degree of deterioration or disease, medical condition of the patient, route of administration, pharmacological considerations such as the activity, efficacy, pharmacokinetic and toxicology profile of the particular compound used, whether a drug delivery of the system is used, and whether the compound is administered as part of a combination of drugs.

The term "cancer" or "tumour" used herein refers to a disease characterised by uncontrolled cell division (or by increased survival or resistance against apoptosis) and by the ability of said cells to invade other neighbouring tissues (invasion) and spread to other areas of the body where the cells are not normally located (metastasis) through lymphatic and blood vessels, circulating through the bloodstream, and then invade normal tissues in other parts of the body. Tumours are classified as benign or malignant depending on whether or not they can spread by way of invasion and metastasis: benign tumours are those which cannot spread by invasion or metastasis, for example, those that can only grow locally; whereas malignant tumours are those capable of spreading by invasion and metastasis.

In a particular embodiment, the cancer is selected from the group consisting of an anaplastic or undifferentiated carcinoma of the thyroid, a poorly differentiated carcinoma of the thyroid, recurrent carcinoma of the thyroid, differentiated carcinoma of the thyroid including papillary carcinoma and follicular carcinoma of the thyroid. In a preferred embodiment, the cancer is selected from the group consisting of anaplastic carcinoma of the thyroid, poorly differentiated carcinoma of the thyroid, and recurring undifferentiated carcinoma of the thyroid. In an even more preferred embodiment, the cancer is anaplastic carcinoma of the thyroid.

"Anaplastic carcinoma (AC) of the thyroid" referred to in the present invention is a malignant tumour of the thyroid gland made up of highly undifferentiated follicular thyroid cells. AC, also referred to as undifferentiated carcinoma of the thyroid, is the most aggressive malignant primary thyroid tumour with a mean survival of only 10-20% in the first year. Although the number of patients with a life expectancy of more than one year is increasing, despite aggressive therapies, mean survival of AC has not significantly improved in the last 20 years. Patients are often older, between 60-70 years of age, and the tumour tends to manifest clinically as a mass in the neck which grows quickly and infiltrates the structures of the neck causing pain, hoarseness, dyspnoea, and/or dysphagia. About 35% of patients present distant metastasis at the time of diagnosis.

Macroscopically, the tumours are large infiltrating fleshy tumours with haemorrhagic and necrotic areas. Histologically, the morphology is variable, although they can be grouped into 3 main morphological patterns (alone or in combination): sarcomatoid pattern, giant cells pattern, and epithelial pattern. Sarcomatoid AC is formed by fusiform cells with characteristics similar to those observed in high-grade pleomorphic sarcoma. Giant-cell AC contains malignant pleomorphic cells which are sometimes multinucleated. Epithelial AC manifests with cohesive nests of squamous or squamoid cells and occasional keratinisation spots. Necrosis, a high mitotic activity, a highly infiltrating growth, and vascular invasion are common in all cases. The presence of acute inflammatory infiltrate, tumour-infiltrating macrophages, the presence of heterologous elements (for example, cartilage and/or neoplastic bone), and/or osteoclastic-type multinucleated giant cells are also common. Unlike AC, squamous carcinoma of the thyroid exhibits squamous differentiation in all the cells thereof. There are also rare paucicellular-, angiomatoid-, rhabdoid-, lymphoepithelioma-, and small cell-type AC variants. In the immunohistochemistry study, unlike what occurs in differentiated carcinomas (papillary and follicular) and in poorly differentiated carcinoma, tumour cells are negative for thyroglobulin and TTF1, while usually being positive for PAX8 and FoxA1 in 50% of the cases of AC. Although most cases of AC are unresectable at the time of manifestation, some patients may benefit from aggressive surgery with radiotherapy and/or adjuvant chemotherapy. Given that AC found incidentally in thyroidectomy specimens of differentiated carcinoma may have a better clinical course, the percentage of AC must always be indicated in the anatomopathological report. There are also numerous ongoing trials with new targeted therapies and/or immunotherapy for AC.

"Poorly differentiated carcinoma (PDC) of the thyroid" referred to in the present invention is a follicular cell neoplasia which exhibits limited evidence of follicular differentiation and constitutes an intermediate between differentiated carcinomas (papillary and follicular) and anaplastic carcinoma, both from the morphological and from the biological behaviour viewpoint. The histopathological criteria for the diagnosis of PDC are the so-called Turin consensus criteria. This consensus includes a diagnostic algorithm based on the following criteria for the diagnosis of PDC: 1) presence of solid/trabecular/insular growth pattern, 2) absence of nuclei with conventional characteristics of papillary carcinoma of the thyroid, and 3) presence of at least one of the following data: convoluted nuclei, 3 or more mitotic activities per 10 fields at 400 magnifications, and tumour necrosis. These criteria have been validated in more recent series. It has been proven that the presence of 10% of areas with PDC criteria in a well differentiated carcinoma is already associated with a worse prognosis. Furthermore, PDC criteria are also applicable to Hürthle cell carcinomas and to the cribriform-morular variant of papillary carcinoma. PDC-associated molecular alterations include early "driver" events typically associated with well differentiated carcinomas (for example, RAS or BRAF mutations), as well as other alterations that occur such as final events associated with dedifferentiation (for example, TP53, TERT, CTNNB1, and AKT1 mutations). The mutational load of PDC is intermediate between the well differentiated papillary carcinoma and the anaplastic carcinoma. The 5-year overall survival for PDC ranges between 60 and 70%. Recurrences tend to occur within the first 3 years. The mean survival time for PDC is 5 years after diagnosis. PDC generally exhibits a poor response to treatment with radioactive iodine.

"Recurrent carcinoma of the thyroid" referred to in the present invention is a tumour originating or resulting from the regrowth of a thyroid cancer which has already been treated or extirpated, more specifically from the cells of a thyroid cancer or tumour which has already been treated or extirpated. Generally, it originates from a differentiated or poorly differentiated thyroid cancer. Recurrent carcinoma of the thyroid may have regrown in the same area of the body in which the thyroid tumour from which it originates was located, or it may grow in an area of the body, tissue, or organ different from the thyroid cancer in the gland from which it originates. In this sense, recurrent thyroid cancer can be classified as local or metastatic. Recurrent thyroid cancer can originate from a differentiated or poorly differentiated cancer and can also be a differentiated or poorly differentiated cancer. It is often detected by the presence of specific proteins of the thyroid gland in blood, generally the thyroglobulin protein. Specifically, prior thyroid gland extirpation was performed on patients having tumours of this type as treatment for the original thyroid cancer. In this sense, given that these patients, in principle, lack cells of the thyroid gland, they should not be able to synthesise or exhibit any specific proteins of the thyroid gland in blood. Therefore, the presence of specific proteins of the thyroid gland in blood is an indication that cells of the original thyroid cancer have proliferated such that the original thyroid cancer has regrown.

"Papillary carcinoma (PC) of the thyroid" referred to in the present invention is a malignant epithelial tumour with evidence of follicular differentiation and a series of nuclear characteristics. PC is generally invasive. Its diagnosis requires the existence of papillae, invasion, or cytological traits of PC. It is the predominant type of thyroid cancer and the most increasing thyroid cancer around the world. The incidence of PC in women is three times that in men. About 20% of PC cases are multifocal. Unlike follicular carcinoma, PC tends to disseminate by the lymphatic route, metastasising to regional lymph nodes. Metastases to lateral lymph nodes of the neck and to those of the median line (central compartment and levels VI and VII) are common. Haematogenous dissemination is less common and tends to affect the lungs. As a group, PC is usually biologically indifferent, with an excellent prognosis, 5-year survival rates of 95%, and a 20-year survival rate of more than 90%.

"Follicular carcinoma (FC) of the thyroid" referred to in the present invention is a malignant tumour of follicular cells in which there are no nuclear characteristics of papillary carcinoma of the thyroid. FC is generally an encapsulated tumour that exhibits an invasive growth. Follicular cell tumours with an oncocytic change are called Hürthle cell tumours and classified separately from FC. FC includes three histological subtypes which are associated with progressive clinical aggressiveness: a) minimally invasive FC, characterised by the exclusive presence of capsular invasion, b) angioinvasive encapsulated FC, and c) highly invasive FC. FCs with only capsular invasion have an excellent prognosis, but the larger the number of vessels invaded by the tumour, the worse the prognosis becomes. The most common metastatic sites are bone, lungs, brain, and liver, although skin dermis metastases are not uncommon. Mutation of the TERT promoter has become an independent marker indicating a higher risk of recurrence of the disease and of mortality associated with differentiated (papillary and follicular) thyroid cancer. Given that FCs are well differentiated tumours, they usually respond to treatment with radioactive iodine and have a long survival.

In another particular embodiment of the invention, the cancer is selected from the group consisting of an undifferentiated or anaplastic carcinoma of the chest, heart, lung, small intestine, colon, spleen, liver, bladder, head, neck, ovary, prostate, brain, rectum, pancreas, skin, bone, bone marrow, blood, thymus, uterus, testicle, liver, or hepatobiliary; poorly differentiated carcinoma of the chest, heart, lung, small intestine, colon, spleen, liver, bladder, head, neck, ovary, prostate, brain, rectum, pancreas, skin, bone, bone marrow, blood, thymus, uterus, testicle, liver, or hepatobiliary; sarcomatoid carcinoma of the thyroid, chest, heart, lung, small intestine, colon, spleen, liver, bladder, head, neck, ovary, prostate, brain, rectum, pancreas, skin, bone, bone marrow, blood, thymus, uterus, testicle, liver, or hepatobiliary; pleomorphic carcinoma of the thyroid, chest, heart, lung, small intestine, colon, spleen, liver, bladder, head, neck, ovary, prostate, brain, rectum, pancreas, skin, bone, bone marrow, blood, thymus, uterus, testicle, liver, or hepatobiliary; fusiform-cell or giant-cell carcinoma of the chest, heart, lung, small intestine, colon, spleen, liver, bladder, head, neck, ovary, prostate, brain, rectum, pancreas, skin, bone, bone marrow, blood, thymus, uterus, testicle, and liver or hepatobiliary carcinoma.

In a particular embodiment, the cancer is an anaplastic carcinoma.

As it is used herein, the expression "anaplastic carcinoma" indicates a malignant neoplasm resulting from the uncontrolled proliferation of transformed cells of epithelial origin or showing certain epithelial characteristics, but not cytological characteristics associated with more differentiated tumour types differentiated, such as the formation of glands or the presence of cell junctions characteristic of adenocarcinomas and squamous cell carcinomas.

In a particular embodiment, the cancer or anaplastic carcinoma is an anaplastic astrocytoma, an anaplastic large-cell lymphoma, or an anaplastic meningioma.

In a particular embodiment, the cancer is anaplastic or undifferentiated pancreatic ductal adenocarcinoma.

In a particular embodiment, the cancer is anaplastic large-cell lung carcinoma or anaplastic giant-cell lung carcinoma.

In a particular embodiment, the cancer is undifferentiated gastric carcinoma.

In a particular embodiment, the cancer is a cancer characterised by exhibiting a high degree of aneuploidy.

The term "aneuploidy" herein refers to a genetic material imbalance caused by losing or gaining a complete chromosome or part of a chromosome. It is understood that cancer has an elevated degree of aneuploidy when the percentage value of aneuploid cells measured by means of FISH is at least 3%, at least 4%, at least 5%, at least 6%, at least 7%, at least 8%, at least 9%, at least 10% of the total cells in the tumour.

In a particular embodiment, the cancer is a primary or metastatic cancer.

In a particular embodiment, the inhibitor for use of the invention is a nucleic acid of the invention according to any of the embodiments of the first aspect of the invention, a vector according to any of the embodiments of the second aspect of the invention, or combinations thereof. In another preferred embodiment, the inhibitor for use of the invention is a nucleic acid according to any of the embodiments of the first aspect of the invention, or combinations thereof. In a particular embodiment, the inhibitor for use of the invention is the nucleic acid of the invention in which the antisense nucleotide sequence is selected from the group consisting of SEQ ID NO. 6, SEQ ID NO. 7, and functionally equivalent sequences, and the sense nucleotide sequence is selected from the group consisting of SEQ ID NO.11, SEQ ID NO. 12, and functionally equivalent sequences.

In a preferred embodiment, the inhibitor for use of the invention is the nucleic acid of the invention, wherein the antisense nucleotide sequence is SEQ ID NO. 6 or a functionally equivalent sequence and the sense nucleotide sequence is SEQ ID NO. 11 or a functionally equivalent sequence.

In a particular embodiment, the inhibitor for use of the invention is the nucleic acid of the invention, wherein the antisense nucleotide sequence comprises a sequence of at least two uracils at the 3' end.

In a particular embodiment, the inhibitor for use of the invention is the nucleic acid of the invention, wherein the sense nucleotide sequence comprises a sequence of at least two uracils at the 3' end.

In another particular embodiment, the inhibitor for use of the invention is the nucleic acid of the invention in which the sense and antisense sequences comprise a sequence of at least two uracils at their 3' ends.

In a particular embodiment, the inhibitor of the invention is the nucleic acid of the invention comprising the antisense sequence SEQ ID NO. 26 or functionally equivalent sequences and the sense sequence SEQ ID NO. 31 or functionally equivalent sequences.

In a particular embodiment, the inhibitor of the invention comprises a combination of nucleic acids of the invention, preferably the combination of any of the nucleic acids mentioned in the preceding embodiments in this fifth aspect of the invention.

In a preferred embodiment, the invention relates to the pharmaceutical composition of the invention for use in the treatment of cancer in a patient, selected from the group consisting of anaplastic carcinoma of the thyroid, poorly differentiated carcinoma of the thyroid, or recurring undifferentiated carcinoma of the thyroid, preferably from the group consisting of anaplastic carcinoma of the thyroid and poorly differentiated carcinoma of the thyroid, more preferably anaplastic carcinoma of the thyroid.

In a particular embodiment, the cancer is any of the cancers indicated above in relation to the medical use of the inhibitor of the invention in this fifth aspect of the invention.

In a preferred embodiment, the pharmaceutical composition of the invention comprises the inhibitor for use of the invention, as defined in any of the embodiments of this fifth aspect of the invention.

In a particular embodiment, the inhibitor or the pharmaceutical composition of the invention is administered to the patient at least one time, at least 2, 3, 4, 5, 6, 7, 8, 9, 10, 11, 12, 13, 14 times a week.

In another particular embodiment, the inhibitor or the pharmaceutical composition is administered by means of the most suitable route according to its composition, for example, by means of the systemic (for example, intravenous, subcutaneous, intramuscular), oral, parenteral, or topical routes, for which the pharmaceutically acceptable excipients necessary for the formulation of the desired method of administration will be included. Furthermore, the inhibitor or the pharmaceutical composition can be also administered intranasally or sublingually, and thus allows systemic administration by means of a non-aggressive mode of administration. Furthermore, intraventricular administration may be suitable. A preferred route of administration is the intravenous route.

In a particular embodiment, all the definitions embodiments indicated in relation to the first, second, third, and fourth aspects apply to the fifth aspect of the invention.

In a particular embodiment, the inhibitor for use of the invention specifically inhibits the expression of the PIAS2α protein, such that in the embodiments described in this fifth aspect of the invention, the term PIAS2β is replaced with PIAS2α, and the term PIAS2α is replaced with PIAS2β.

### 6- Method and kit for the differential detection of the nucleic acid sequence of isoform β or of isoform α of the PIAS2 gene in a sample and method for the differential detection of a differentiated thyroid carcinoma of an advanced carcinoma, a poorly differentiated carcinoma, or an anaplastic carcinoma.

In another aspect, the invention relates to a polymerase chain reaction (PCR) amplification method for detecting, in a differential manner, the nucleic acid sequence of isoform β or of isoform α of the PIAS2 gene in a sample, said process comprises:
(a) providing the PCR assay containing said sample with a labelled oligonucleotide containing a sequence complementary to a region of the target nucleic acid, wherein said labelled oligonucleotide hybridises with the target nucleic acid sequence, binding thereto through the primers of step (b), and wherein said labelled oligonucleotide has the sequence SEQ ID NO: 87 when the detection of nucleic acid sequence of isoform α of the PIAS2 gene is desired or SEQ ID NO: 88 when the detection of nucleic acid sequence of isoform β of the PIAS2 gene is desired;
(b) providing a pair of primers, wherein the first primer contains a sequence complementary to a region of a chain of the target nucleic acid sequence and initiates the synthesis of an extension product and a second primer containing a sequence complementary to the other chain of the target nucleic acid sequence or complementary to a region in the extension product of the first primer and initiates the synthesis of a complementary DNA chain; and wherein each primer is selected so that it hybridises with its complementary template upstream of any labelled oligonucleotide hybridised with the same nucleic acid chain, wherein the pair of primers is formed by the primers of sequence SEQ ID NO: 89 and SEQ ID NO: 90 when the detection of the nucleic acid sequence of isoform α of the PIAS2 gene is desired or by the primers of sequence SEQ ID NO: 91 and SEQ ID NO: 92 when the detection of the nucleic acid sequence of isoform β of the PIAS2 gene is desired,
(c) amplifying the target nucleic acid sequence using a nucleic acid polymerase with 5' to 3' nuclease activity as a template-dependent polymerising agent under conditions permissive for PCR cycling steps of (i) hybridising the primers and the labelled oligonucleotide with a template nucleic acid sequence contained within the target sequence, and (ii) extending the primer, wherein said nucleic acid polymerase synthesises a primer extension product while the 5' to 3' nuclease activity of the nucleic acid polymerase simultaneously releases labelled fragments of the hybridised double chains comprising labelled oligonucleotides and their complementary template nucleic acid sequences, thereby creating detectable labelled fragments and
(d) detecting and/or measuring the signal generated by the hydrolysis of the labelled oligonucleotide in order to determine the presence or absence of the target sequence in the sample.

The terms used for defining the method of the invention have been described in detail, among others, in European patent EP919565-B1 belonging to F. Hoffmann - La Roche which is incorporated in its entirety to the present description by reference. Likewise, preferred embodiments of the method are also described in detail in patent EP919565-B1. In a preferred embodiment, the labelled oligonucleotide comprises a pair of interactive signal-generating markers suitably positioned in the oligonucleotide to shield the generation of a detectable signal, said markers are separated by a site within the oligonucleotide which is susceptible to nuclease cleavage, thereby allowing, during primer extension, the 5' to 3' nuclease activity of the nucleic acid polymerase to separate the first interactive signal-generating marker from the second interactive signal-generating marker by cleaving at the susceptible site, thereby producing a detectable signal. In an even more preferred embodiment, said first marker is a chemiluminescent substrate and said second substrate is a fluorophore which interacts with same.

In another aspect, the invention relates to a kit for the differential detection the nucleic acid sequence of isoform β or of isoform α of the PIAS2 gene in a sample comprising:
(a) at least one labelled oligonucleotide containing a sequence complementary to a region of the target nucleic acid, wherein said labelled oligonucleotide has the sequence SEQ ID NO: 87 when the detection of the nucleic acid sequence of isoform α of the PIAS2 gene is desired or SEQ ID NO: 88 when the detection of the nucleic acid sequence of isoform β of the PIAS2 gene is desired, and wherein the 3' end of the labelled oligonucleotide is blocked to prevent extension by a nucleic acid polymerase having 5' to 3' activity and the labelled oligonucleotide contains first and second markers, the first marker being separated from the second marker by a site susceptible to cleavage by a nuclease, and wherein the markers of the labelled oligonucleotide constitute a pair of interactive signal-generating markers located on the oligonucleotide to shield the generation of a detectable signal and
(b) at least one pair of primers, formed by the primers of sequence SEQ ID NO: 89 and SEQ ID NO: 90 when the detection of the nucleic acid sequence of isoform α of the PIAS2 gene is desired or by the primers of sequence SEQ ID NO: 91 and SEQ ID NO: 92 when the detection of the nucleic acid sequence of isoform β of the PIAS2 gene is desired.

The terms used for defining the kit of the invention have been described in detail, among others, in European patent EP919565-B1 belonging to F. Hoffmann - La Roche which is incorporated in its entirety to the present description by reference. Likewise, preferred embodiments of the kit of the invention are also described in detail in patent EP919565-B1. In a preferred embodiment, the kit further comprises a nucleic acid polymerase with 5' to 3' nuclease activity. In another even more preferred embodiment, said nucleic acid polymerase is a thermostable enzyme, preferably a DNA polymerase from the *Thermus* species.

The invention is described below by means of the following merely illustrative examples that do not limit the scope of the invention.

### EXAMPLES

### Materials and methods

### Cell cultures

### A- Primary follicular cell cultures:

Culturing is performed with a specific medium, h7H, designed for thyroid cultures as described previously (Bravo et al., J Clin Endocrinol Metab. 2013 98(6):2431-2441; Garcia-Rendueles et al., Oncogene, 2017, 36(5): 652-666). This is a humanised medium with seven additive components, modified Coon's medium (5% FCS, 5% NCS, penicillin, and glutamine) with hormones, transferrin, iodine, trace elements, antioxidants, metabolites, and ethanol.

The primary tissue culturing system has a strict protocol to prevent bacterial, fundamentally mycoplasma, contaminations. Sealed sterile aliquots of the culture medium h7H were used to introduce excess fragments selected from a surgical specimen, fragments within the tumour (T), or normal tissue (N) under a horizontal hood. Tissue dispersion and culture establishment (passage 1) was performed as described (Bravo et al., J Clin Endocrinol Metab. 2013 98(6):2431-2441; Garcia-Rendueles et al., Oncogene, 2017, 36(5): 652-666). Those methods described for the culture passage of 1:2 plates with trypsin when they were filled with cells were also used (Bravo et al., J Clin Endocrinol Metab. 2013 98(6):2431-2441; Garcia-Rendueles et al., Oncogene, 2017, 36(5): 652-666).

The cultures are characterised for the expression of phenotype markers which are maintained after several passages by means of RT-PCR, qRT-PCR, and in the case of protein by means of Western blot and immunofluorescence. Genotyping is also performed by means of STR identification to ensure matching with the tissue of origin. Both technologies are described in Bravo et al., J Clin Endocrinol Metab. 2013 98(6):2431-2441; Garcia-Rendueles et al., Oncogene, 2017, 36(5): 652-666)

### B- Commercial cell line culture

The low incidence of anaplastic carcinomas means that very little primary cultures are available for this pathology, and this has led to the performance of the experiments in commercial cell lines. The lines were selected from several databases, among others (Cellosaurus, https://web.expasy.org/cellosaurus/), and are certified by the International Cell Line Authentication Committee (ICLAC) and confirmed as original human thyroid cells that are not contaminated with other lines or recorded in the Register of Misidentified Cell Lines (v9) (https://iclac.org/databases/cross-contaminations/).

The STRs thereof were characterised in the laboratory of the present applicant to ensure that the genetic profile, such as the most characteristics known mutations, coincides with that found in the databases (Bravo et al., J Clin Endocrinol Metab. 2013 98(6):2431-2441; Garcia-Rendueles et al., Oncogene, 2017, 36(5): 652-666)

Six commercial cell lines were compared: Anaplastic carcinoma of the thyroid: MB-1, BHT-101, CAL-62 (DSMZ Germany); 8305C (ECACC, Sigma). Poorly differentiated carcinoma: BCPAP: (DSMZ), FTC-238 (ECACC).

The cell lines were grown in the h7H medium as described above (Bravo et al., J Clin Endocrinol Metab. 2013 98(6):2431-2441; Garcia-Rendueles et al., Oncogene, 2017, 36(5): 652-666). Routine methods for the passage of cells every week at 1:10 dilution after incubation with trypsin have also been described previously (Bravo et al., J Clin Endocrinol Metab. 2013 98(6):2431-2441; Garcia-Rendueles et al., Oncogene, 2017, 36(5): 652-666).

### dsRNAi design and synthesis

The bioinformatic programme BlockiTTM siRNA Designer, Ambion, Life Technologies and recommendations found in the Silencer^{®} siRNA Construction Kit manual, Ambion (Silencer^{®} siRNA construction kit manual, Life Technologies Corporation, Thermo Fisher Scientific) were used for the design. The following conditions were followed to select definitive sequences from among the sequences of the PIAS2b isoform which may be potentially susceptible to be a target: 1) target sequence starting with AA: therefore, the siRNA sequence ending in UU, 3' UU hanging end (UU in the oligonucleotide for transcription). 2) a G/C content between >25% and 50%

A sense and antisense oligonucleotide sequence complementary to the dsRNAi is obtained for each sequence of the dsRNAi. To each of the target sequences with 21 nucleotides were added 8 nucleotides complementary to the first nucleotide which contains the consensus binding site for bacterial RNA polymerase T7 at promoters (5'-CCTGTCTC-3'), and this was performed both in the antisense chain and in the sense chain. For negative control, the same nucleotide composition as the experimental dsRNAi was used, but without significant sequence homology with the human genome. Furthermore, the 8 nucleotides complementary to the consensus of T7 in promoters were also added. To choose the best sequences and eliminate off targets, the sequences were examined with the BLAST programme (https://blast.ncbi.nlm.nih.gov/Blast.cgi). The sequences were aligned against Ensembl. All sequences which comply with the preceding requirements, but do not return any results in Emsembl, were also excluded.

Five dsRNAi sequences (PIAS2b dsRNAi 1, PIAS2b dsRNAi 2, PIAS2b dsRNAi 3, PIAS2b dsRNAi 4, and PIAS2b dsRNAi 5) and a control sequence ns-dsRNAi were obtained (see Table 4).

To synthesise the dsRNAi sequences, a commercial dsRNAi Silencer^{®} siRNA Construction Kit (AM1620M) (Ambion, later absorbed by Invitrogen, and now Thermo) was used following the manufacturer's instructions. In separate reactions, the two oligonucleotides of each sequence are hybridised with the first of the T7 promoter (included in the kit). Each complementary sense and antisense sequence is then purified and hybridised following the instructions on the kit. They are kept in aliquots once quantified. The dsRNAi were transfected into the cells with various protocols (Bravo et al., J Clin Endocrinol Metab. 2013 98(6):2431-2441; Garcia-Rendueles et al., Oncogene, 2017, 36(5): 652-666).

### Production of lentivirus with inducible shRNAs and calculation of the concentration of doxycycline. Viral transduction.

Lentiviruses were produced by means of transfection with TRIPZ Inducible Lentiviral Non-silencing shRNA Control or TRIPZ Human PIAS2 shRNA (Thermo), together with pHelper virus, and psPAX2 (Addgene). Packaging cells HEK293T were transfected with CaCl₂ using conventional protocols. 20 µg TRIPZ vector, 15 µg of pHelper virus, 6 µg of psPAX2 (lentivirus) were transfected for each 90 mm plate. The medium was collected for two days, then filtered, and viral particles were concentrated by means of ultracentrifugation in a swinging rotor SW32Ti (369694, Beckman Coulter) at 19,500 rpm for 2 hours at 4°C in an Optima L-100XP ultracentrifuge (392052, Beckman Coulter). The pellet is resuspended in 60 µL of Coom's medium h7H and is wet incubated on ice for 10 minutes. The supernatant is split into 50 µL aliquots which are kept at -80°C. The viruses are titrated by means of qPCR for calculating concentration (Barczak et al., Molecular Biotechnology. 2015 57(2):195-200). A second functional titration is performed thereafter by means of the infection of HEK293T with serial dilutions for calculating multiplicity of infection (MOI).

To determine the concentration of doxycycline which best induces the expression of shRNAs, concentrations of 0 µg/mL (control), 0.5 µg/mL, 1 µg/mL, and 2 µg/mL were tested in the CAL-62 cell line. The best concentration was 2 µg/mL. TurboRFP- or mCherry-positive cell colonies in each well corresponding to each factor of dilution are counted. Each colony is formed by a small group of cells and it should be considered as a transduced cell due to the cell divisions that occurred during the last 72 hours.

After obtaining the lentiviruses T-Scr-sh and T-PIAS2-sh, anaplastic thyroid cell lines were infected. The cells are placed in 1 mL of serum-free medium and 8.9 µg/mL of polybrene the day after seeding. Once the amount of necessary viral particles has been added, the multiplates are incubated until the next day. A complete culture medium is added and incubation is carried out for 48 hours. Positive cells were selected with puromycin, an antibiotic for the selection of infected cells, generating stable regulated lines T-PIAS2-sh and T-Scr-sh.

Negative (non-red) populations of each line were selected thereafter in the sorter. Subsequently, cells from these same populations which had, however, been induced with doxycycline overnight were passed through the sorter in order to sort them. Red populations (HI) and extremely red populations (SU) were selected. The selected populations were seeded in the absence of doxycycline so that they lose the red colour, become negative, and grow without differences. Four regulated populations were thus generated: T-PIAS2-sh _HI, T-PIAS2-sh_SU, T-Scr-sh _HI, T-Scr-sh_SU.

### Functional studies in cells

### A- Cell proliferation study

Assays for quantifying the number of cells and proliferation were performed by means of a colourimetric method (MTT) as described in Garcia-Rendueles et al., Oncogene, 2017, 36(5): 652-666).

### B- Cell cycle study

Mitosis study: Videomicroscopy (time-lapse):
For videomicroscopy or time-lapse experiments, 96-well multiplates and Leica microscope with a CO₂ and humidified cell chamber (OKI) using SiR-DNA (Spirochrome) as a nuclear marker are used as described in Lukinavičius et al, Nature Communication 2015;6:8497.

The microscope has an automatic slide which allows using several treatment conditions and preparing several replicates for each experiment. Usually, at least 2 fields are prepared per replicate and at least 6 replicates per condition. The photos were analysed with the Leica LASX programme v 3.30, with the help of manual supervision for counting. Videos were also created with the programme.

### DNA study

### STR profile

To prepare the genetic profile of the primary cultures and their tissues, the AmpFlSTR^{®} Identifier^{®} Plus PCR Amplification Kit is used as described in (Bravo et al., J Clin Endocrinol Metab. 2013 98(6):2431-2441).

### RNA study

### Quantitative PCR

Once the RNA has been extracted and reverse transcribed into a cDNA following conventional methods, quantitative PCR was performed either by means of the TaqMan method (more specific but less sensitive) or else by means of the SybrGreen method (more sensitive, less specific), following conventional protocols (described in Chenlo et al. EBioMedicine. 2019 43:537-552; Bravo et al., J Clin Endocrinol Metab. 2013 98(6):2431-2441) and using the following primers (Table 1).

**Table 1: Primers used for measuring the expression of mRNA**

| | **Gene** | **Sequence** | **Amplification size** | **Annealing T^{a}** |
|---|---|---|---|---|
| **TAQMAN** | **TBP** | Fw: 5'- GCCCGAAACGCCGAATAT -3' | 67 bp | 60°C |
| | | Rv: 5'- TTCGTGGCTCTCTTATCCTCATG -3' | | |
| | | Pb: 5'- TCCCAAGCGGTTTGCTGCGGTA -3' | | |
| | **PIASa** | Fw: 5'- TTGGCTCAAACCACTGTGTC -3' | 171 bp | 60°C |
| | | Rv: 5'- GGGTCTCGCTATGTTGCCTA -3' | | |
| | | Pb: 5'- CAAGCTGGGTGCGGTGGCTT -3' | | |
| | **PIASb** | Fw: 5'- ATTTGAGGCTTGCCTTCTCA -3' | 155 bp | 60°C |
| | | Rv: 5'- TACCACCTCCCAAAATCTGC -3' | | |
| | | Pb: 5'- TGGGGTACGGTGTGACTGGAATG -3' | | |
| **SYBR GREEN** | **PIASa** | Fw: 5'- CGGTTGATCCTGCTGCTATT -3' | 144 bp | 60°C |
| | | Rv: 5'- AAGATGTTCCAAGCTTCAGTTCA -3' | | |
| | **PIASb** | Fw: 5'- CGGTTGATCCTGCTGCTATT -3' | 182 bp | 60°C |
| | | Rv: 5'- GCTTGCTGTTAAGGGTGAGG -3' | | |
| | **Global PIAS2** | Fw: 5'- GCGGATTTCGAAGAGTTGAG-3' | 142 bp | 60°C |
| | | Rv: 5'- AGCCGCTCTTCAATAAATGC -3' | 142 bp | 60°C |
| | **TBP (similar to above)** | Fw: 5'- GCCCGAAACGCCGAATAT -3' | 67 bp | 60°C |
| | | Rv: 5'- TTCGTGGCTCTCTTATCCTCATG -3' | | |

Each 96-well multiplate is used for only one reaction and the samples in each are measured in duplicate together with positive and negative controls of the technique (all the reagents except MMLV) and blanks (all the reagents except cDNA). TBP is used as an expression control for quantitative mRNA assays since its expression is stable both in tissue and in cell lines (Radonic et al., Biochem Biophys Res Commun. 2004 313(4):856-62).

The cycle threshold difference or ΔCt method (ΔCt =Ct_{gene} - Ct_{TBP}) is used to express the results of each gene.

### Protein studies

### A- Global 2D proteomics analysis

2D gels separate the proteins present in the samples based on their isoelectric point, IP (first dimension) and their molecular weight, MW (second dimension). To perform these studies, soluble proteins from human thyrocyte samples were suspended in different lysis buffers described in the literature. The best lysis buffer was: 7M Urea (17-1319-01, GE Healthcare), 2M Thiourea (RPN630, GE Healthcare), 4% CHAPS (C3023, Sigma), 40 mM DTT (D9779, Sigma). The 2D separation methodology, spot quantification, and mass spectrometry identification are described in Bravo et al., International Journal of Peptides, 2011, 2011:969818, an earlier literature published by the inventors.

### B- Western blot

Methods of detecting proteins by means of Western blot and immunofluorescence are extensively described in an earlier literature published by the inventors (Bravo et al., J Clin Endocrinol Metab. 2013 98(6):2431-2441; Garcia-Rendueles et al., Oncogene, 2017, 36(5): 652-666; Chenlo et al. EBioMedicine. 2019 43:537-552).

### In vivo studies

### A- Generation of cell populations

For *in vivo* studies, a population was generated with transduction of the retrovirus MI-Luciferase-IRES-mCherry in primary anaplastic cultures (T-UC1-MI-Luciferase-IRES-mCherry). The population expresses RFP (red fluorescent protein) as a cell infection control and luciferase which allows tracking tumour growths in mice later on.

### B- Orthotopic xenoimplant model of anaplastic carcinoma thyroid cells from patients in NOD-SCID mouse (oPDX)

Female immunosuppressed NOD-SCID mice (NOD.CB-17-Prkdc scid/Rj, Janvier Labs) (Hidalgo *et al,* 2014; Byrne *et al,* 2017) (5 controls and 4 treated) 7 weeks of age at the time of inoculation were used.

The cells were orthotopically implanted in the right lobe of the thyroid gland by means of neck surgery with a Hamilton syringe having a 3 cm long blunt needle (100 µL/30 mm/PST3 (1710N, Hamilton). For the surgical implantation technique, a decision was made to standardise an in-house methodology. The group Sewell et al, Journal Visualized Experiments 2013, 74: 50097, standardised a technique for the implantation of human thyroid cell line xenografts, which included sectioning laryngeal muscles to access the thyroid. The present group decided that this technique was too aggressive for obtaining a relevant human thyroid carcinoma model because it disrupted the muscle structures surrounding the thyroid, caused inflammation, and may alter the endocrine physiology of the receiving animal.

The step-by-step protocol is as follows:
- Two days before implantation in the mouse, cells of the population were trypsinised with trypsin IX, counted in a Neubauer chamber, and reseeded with a 1:2 dilution. This is performed to maintain their active state and to estimate the number of available cells.
- On the day of implantation, the cell pellet is resuspended in the residual volume after centrifugation by gentle tapping, and an approximate volume of Matrigel (E1270, Sigma) is added, the mixture is kept in ice (4°C)
- The mice were anaesthetised with a volume of ketamine/xylazine (100 µL for every 10 g). The surgical field was shaved using shaving cream (Veet^{®} shaving cream with aloe vera), cleaning the area with gauze and warm water to remove traces of cream and to disinfect the area with Betadine^{®} before surgery. The four extremities were fixed with an adhesive tape. A vertical incision of about 1 centimetre is made in the mid-region of the neck with a scalpel. With the help of a pair of forceps, submaxillary glands are separated until the trachea with its muscles and fascias are visible. The sternothyroid muscle is lifted. With the help of a Hamilton syringe which is kept cold to prevent the contents thereof from solidifying, 25 µL of the cells mixture is inoculated, taking care not to inject same in the trachea or to run with the needle through the gland. Upon inoculation, there is a need to wait a few seconds before removing the needle so as to favour the solidification of the Matrigel and thereby prevent the diffusion of the mixture to adjacent tissues. The incision is closed with a 4-0 suture thread (Mersil Soie, Ethicon) with two-three independent stitches. Disinfection is performed and analgesia applied.

### C- In vivo bioluminescence assay measurement

Measurement is performed by means of the conventional protocol recommended by IVIS (Perkin Elmer) by injecting 300 mg/kg of luciferin (122799, Perkin Elmer) into the anaesthetised animal with isoflurane. The signal is picked up at 5, 10, 15, 20, 25, and 30 minutes. Signal quantification is performed, representing the maximum signal value in photons/second with PerkinElmer's Living Image 3.2 programme (Inoue et al.,. Int J Biomed Imaging. 2010; 2010:471408; Stewart et al., Nature. 2017 549(7670):96-100). Tumour growth was tracked on a weekly basis for 5 weeks before starting treatment to check the development of the tumour in the animal.

### D- In vivo dsRNAi treatments

After injecting the cell suspension, the animals are distributed into groups of 4 or 5 animals per box. The animals are kept in this manner up to week 7 in which treatment starts. Tumours were generated in all the animals. Based on the treatment, the animals were randomly mixed at the time of treatment and they were kept in this manner until the end of the experiment.

For the dsRNAi to have a sufficient half-life, they were mixed with a vehicle, atelocollagen (AteloGene koken, Cosmobio) which protects nucleic acids from nuclease degradation (Ochiya et al., Current Gene Therapy 2001 1: 31-52; Takei et al., Cancer Research 2004, 64: 3365-3370; Takeshita et al., Proceedings of the National Academy of Science of the USA 2005, 102 (34): 12177-12182; Takahashi et al., PLoS One 2013, 8(8):e73214).

When the tumours in the neck have grown into large masses (5 weeks), they are treated intravenously with a dose of 200 µL of 40 µM per injection/mouse. This concentration was based on previously published papers (Azuma et al., Biochemical Biophysics Research Communication 2010, 391 (1): 1075-1079; Sasaki et al., Biochemical Biophysics Research Communication 2010, 399(1): 79-83).

All the values from bioluminescence measurements for each animal are time-adjusted immediately before the start of treatment (100%) and recalculated in a corresponding manner. Longitudinal curves are depicted by connecting the measurements of individual mice and the regression of the data sets was calculated by means of LOWESS non-linear regression. LOWESS (LOcally WEighted Scatter-plot Smoother) is a non-parametric, locally weighted regression method for generating a smooth curve between two data groups (Jacoby WG, Electoral Studies. 2000; 19(4):577-613). Graphpad Prism 8 software was used with the specific algorithm of (Chambers et al., Graphical methods for data analysis, wadsworth & Brooke/Cole publ. Comp., Pacific Grove, California. 1983).

### E- Histological analysis of the oPDX

The specimens are fixed with formalin and included in paraffin according to a conventional method (FFPE). The pathologist trims the specimens according to a conventional method until reaching the middle part. From there, the specimens are sectioned upwards and downwards. The sections are stained with haematoxylin-eosin (H-E) or immunohistochemistry. Immunohistochemistry was performed with antibodies clinically validated for the diagnosis of anaplastic carcinoma of the thyroid: CK8+CK18, CK AE1AE3, PAX8, TTF1, Ki67, TG, and p53.

In addition to general microscopic observation, the H-E sections were used to count the number of dead cells of the carcinoma (deep pink background and black central condensed nucleus) by comparing the control group to the treated group. Counting was performed with the FiJi programme (ImageJ 1.52) (https://fiji.sc/).

### Statistical analysis

The Prism 8 programme (Graphpad Software Inc.) was used for statistical analysis.

All the results are represented using mean ± SEM. Samples demonstrating evident errors for the methodology used were excluded (technical exclusion due to alteration of the protocol; a rare event in this thesis).

The *in vitro* experiments are performed at last 3 times with between 3 and 6 replicates per condition. In each result group, a normality test was first performed using the Kolgomorov-Smirnof test for n between 5 and 7, the Shapiro-Wilk test for samples with n equal to or greater than 3, and the D'Agostino & Pearson test for n equal to or greater than 10.

All the comparisons between data groups that follow a normal distribution were performed with the T-test to compare two groups, or with an ANOVA to compare more than 2 groups. In the case of non-normal distributions, the Mann-Whitney test was used to compare two groups and the Kruskal-Wallis test was used to compare more than 2 groups, respectively.

Data with p<0.05 was considered statistically significant (^{∗}, p<0.05; ^{∗∗}, p < 0.01; ^{∗∗∗}, p < 0.001; ^{∗∗∗∗}, p < 0.0001).

### Example 1.

### 2D-proteomics differential expression study

The present group established an *in vitro* culturing system for human follicular thyroid cells, h7H, in which each step of the culturing method is looked after in order to achieve 1) cell growth; 2) maintenance of the phenotype which the cells exhibit *in vivo* (Bravo et al., J Clin Endocrinol Metab. 2013 98(6):2431-2441). One of the essential elements is the culture medium which contains seven groups of elements considered essential and in which concentrations are strictly maintained in range with those found in the serum of healthy adult humans. These seven groups include: 1) ions and osmolarity; 2) hormones; 3) trace elements; 4) antioxidants and vitamins; 5) nutrients and metabolites; 6) iodine, 7) ethanol.

As a result of this culturing method, normal thyroid cells can be grown for at least twenty *in vitro* passages using simply surgical residues in acute cultures, something that no one had been able to do until then. The cultures of the present invention were enriched in follicular epithelial cells by >95% (Bravo et al., J Clin Endocrinol Metab. 2013 98(6):2431-2441). Since normal and cancerous follicular epithelium can be grown under the same conditions, specific differential mechanisms can be compared and identified, without distortions derived from proliferation "*per se*" given that, in a tissue, cancerous epithelium is dividing, but normal epithelium is not. This was proven in the study conducted by the present inventors on the mechanism of action of TGF beta, one of the most abundant factors in normal and cancerous epithelium, which resets the anti-proliferative/pro-apoptotic mechanism thereof in normal follicular epithelium to pro-proliferative/anti-apoptotic in the follicular epithelium of differentiated carcinoma (Garcia-Rendueles et al., Oncogene, 2017, 36(5): 652-666.).

Next, the inventors seek to look for new proteins that explain the mechanisms of thyroid cancer and serve for diagnosis, prognosis, or treatment. Therefore, the present inventors started a differential proteomics study. Proteins were extracted from two normal cultures T-NT1 and T-NT2; and to emphasise how different "cancer" is, the decision was made to include a culture of diffuse goitre caused by Pendred disease, T-PS2 as "normal". Goitre refers to a growth of "normal" follicular epithelium brought about by any cause; in this case, pendrin (SLC26A4) mutation leads to an impaired thyroid hormone synthesis, and the pituitary gland stimulates a compensatory growth of follicular epithelium with more TSH.

With respect to same, proteins were extracted from three papillary carcinomas, two T-PC2 and T-PC3. Table 2 describes the key characteristics of the original specimen of each culture.

**Table 2: Characteristics of the original tissues of the cultures used in the 2D-proteomics study**

| **Normal** | **Characteristics** | **Differentiated carcinoma** | **Characteristics** |
|---|---|---|---|
| T-NT2 | Normal thyroid Female, 24 years old Prior minimally invasive follicular carcinoma, contralateral lobe | T-PC2 | Classic-variant papillary carcinoma. Male, 34 years old |
| T-NT3 | Normal thyroid Female, 44 years old Contralateral follicular-variant papillary carcinoma | T-PC3 | Tall cell-variant papillary carcinoma Male, 73 years old |
| T-PS2 | Pendred syndrome nodule, confirmed c.279delT mutation, c.416-1G>A Male, 24 years old | | |

Proteins were extracted in the first passages of growth in culture, except for passage 5, by means of extraction with urea/thiourea/Chaps. The proteins were measured and 50 micrograms were loaded in an isoelectric focusing strip with a pH range of: 4-7. After this first step, the strip was run in the second dimension in a 15% PAGE gel, and subsequently stained with silver staining. The four isoelectric focusing strips and the four gels were run and stained together to avoid variables due to any of these steps. (Figure 1-A).

The gels were scanned and analysed with the PDQuest programme for a first analysis (Figure 1-B), and the analysis was repeated with the SameSpots programme (Figure 1-C). Spots that were differentially expressed, in a quantitatively relevant manner and in a statistically significant manner, in carcinomas in comparison to normal cultures in both analyses were selected.

As can be observed in Figure 1-B, the three spots meeting those characteristics are spot 2502 which increases 1.7 times in cancer, spot 7003 exclusive of cancer, and spot 8002 which doubles in cancer. The result was confirmed upon repeating the analysis (shown for spot 8002, Figures 1-C).

Thereafter, the spots were minced and identified by means of mass spectrometry (Figures 1-D). Spot 2502 is vimentin, a cytoskeleton protein which is abundantly expressed in thyroid tissue (Human Protein Atlas, www.proteinatlas.org). For spot 7003, it was not possible to obtain peptides with sufficient score to be identified. Spots 4304 and 5303 were both identified as beta-actin. Spot 8002 was identified as the PIAS2 protein, a protein that is unknown up until known in relation to the thyroid.

### The PIAS2 gene and its isoforms

A detailed *in silico* study of the PIAS2 protein and its gene in humans was performed (Figure 2). Sixteen mRNA transcripts are described in the ENSEMBL database, of which only 8 can be translated and 8 are non-coding transcripts. Of the coding transcripts, 5 of those transcripts only encode small peptides of between 80-282 amino acids the functional relevance of which is unknown at this time. The 3 transcripts which yield a functional protein are PIAS2-204 that gives rise to the PIAS2 beta protein (PIAS2b), PIAS2-201 that gives rise to the PIAS2 alpha protein (PIAS2a), and PIAS2-215 that gives rise to a small protein referred to as PIAS2 isoform 8 or PIAS2 NY. If a search is performed in the NCBI database, 29 recorded transcripts can be found, 22 being coding transcripts NM_ and 7 being non-coding transcripts NR PIAS2 was not studied in the thyroid and the proteomics analysis result of the present invention indicated that it could be a protein with differential expression. A decision was made to start the detailed study thereof following the initial objective.

### PIAS2 proteins: characterisation and validation of two antibodies

PIAS2b and PIAS2a are two identical proteins in the first 551 amino acids. Several functional domains are recognised in this sequence (Uniprot, https://www.uniprot.org/uniprot/O75928):
- -the N-terminal SAP domain (amino acids 11-45) formed by two sets of hydrophobic residues alternating with highly conserved polar residues separated by a glycine. Its action would include DNA binding and chromatin organisation. It includes therein an LXXLL subdomain found to be important for interaction with other transcription factors in other proteins.
- -the PINIT domain (amino acids 331-408) formed by two anti-parallel beta sheets.

It serves as a substrate anchoring point to promote lysine sumoylation.
- -the SP-RING (amino acids 331-408): a domain common to the Siz and PIAS proteins, hence its name. It differs from the conventional "RING fingers", characteristics of E3 ubiquitin ligases, in which it only contain one Zn2+ ion instead of two. It therefore functions as an E3 SUMO ligase, but not ubiquitin ligase. The PIAS proteins bind through SP-RING to Ubc9 (UBE2I) which is an e2 sumo-ligase;
- -the consensus SUMO binding site (amino acids 467-473)
- -the nuclear localization sequence, NLS (amino acids 484-492).
- -From amino acid 551, the isoform PIAS2a has a small tail until its terminal amino acid 572. However, PIAS2b has another important domain, the serine-rich carboxyl-terminal domain (ser-rich domain), amino acids 571-612, having therein a subdomain with a series of seven continuous serines (poly-S).

Since it was unknown which antibodies were validated and for what techniques , up to seven different antibodies have been tested during this study (see methods, and data is not shown). From these antibodies, only two were characterised because the rest did not seem to truly recognise PIAS2 in the positive controls overexpressing the two isoforms separately.

The epitope of the mouse anti-PIAS2 monoclonal antibody (mPIAS2) was located between amino acids 518-540, and therefore was common to PIAS2b and PIAS2a. The epitope of the rabbit anti-PIAS2beta polyclonal antibody (rPIAS2) was located in the serine-rich region, exclusive of the C-terminal end of PIAS2b. rPIAS2 had been validated for immunohistochemistry by the Atlas Consortium (Human Protein Atlas, https://www.proteinatlas.org), but not for other techniques.

Human proteins overexpressed with an N-terminal FLAG epitope (FLAG-hPIAS2b and FLAG-hPIAS2a) are used as controls (Arora et al., J. Biol. Chem. 2003, 278(24):21327-30). In addition to being validated by complete sequencing in the Addgene plasmid bank, both constructs were entirely sequenced by the present inventors. Another control overexpressing the chimeric protein EGFP-hPIAS2b was also used (Galanti et al., Nature, 2009, 462(7275):935-9).

### Expression of PIAS2 in the thyroid tissues of patients

Once the antibodies have been validated, it was also of interest to be able to quantify RNA expression in tissues. TaqMan assays which would allow quantitatively measuring the mRNA expression of all the coding RNA isoforms as a whole (global PIAS2) and of each of isoforms PIAS2b and PIAS2a are designed. In the present invention, the series of tissues from the TIROCHUS collection (in collaboration for anaplastic cases with the group of Dr. Laura Fugazzola, Endocrine and Metabolic Diseases Department, Universidad degli Studi di Milano) included 29 normal tissues (NT), 33 multinodular goitres (benign proliferative disease, MNG), 23 differentiated papillary-type carcinomas (PTC) of various histological variants, 9 poorly differentiated carcinomas (PDTC), and 15 anaplastic or undifferentiated carcinomas (ATC) (Figure 3-A). As a technical control of the multiple PCRs, a commercial mRNA sample from a normal thyroid tissue which was included in all the reaction plates is used.

Global PIAS demonstrated an abundant expression of the gene in all the tissue groups except in ATC, where it was quantitatively expressed to a lesser extent with a statistical difference of p=0.09.

Meanwhile, NT and MNG expressed the same levels of PIAS2b, PTCs expressed significantly more PIAS2b. Moreover, PDTC and ATC expressed significantly much less PIAS2b (Figure 3-A, bottom left).

PIAS2a was expressed in a smaller amount compared to PIAS2b, but it was again expressed in a significantly larger amount in PTC compared to any other group. Since it was hardly expressed in the other groups, significant differences were not observed in PDTC or ATC (Figure 3-A, right).

When comparing 8 sets of NT/PTC tissue originating from the same patient, it is found that PIAS2b is increased in all the cases, but it was not so with PIAS2a. (Fig. 3-B).

### Validation of the commercial thyroid cancer cell line model

To start the functional study of the present invention, thyroid carcinoma cell lines of different tumour origins and different genetic background are selected as the first model. The lines were validated following ICLAC's indications (https://iclac.org/) but with the actual system of the present inventors previously used based on 16 STRs. The data matched the data described by the provider companies (DSMZ, ECACC) and by the Cellosaurus consortium (https://web.expasy.org/cellosaurus/).

The lines were chosen such that they originated from patients and were not genetically modified, and such that they represented the key pathological categories of thyroid carcinoma. Therefore, FTC-238s represent differentiated follicular thyroid carcinoma (FTC) of a 42-year old male patient, although they originate from a lung metastasis which is characteristic of these cancers (Goretzki et al., Karger Publisher, 1989, 18:56-80; Goretzki et al. Recent Results Cancer Res., 1990, 118:48-63). BCPAPs originate from a metastatic poorly differentiated carcinoma (PDTC) of a 76-year old woman that, however, presented a papillary component (PTC) (Paulin et al., C. R. Acad. Sci. III, 1992, 315(12):493-8). Moreover, four anaplastic carcinoma lines (ATC) were selected as they represent different genetic backgrounds: 8305Cs originate from an 85-year old Japanese woman (Ito et al., INt. J. Oncol., 1994, 4(3):583-6), CAL-62s originate from a 70-year Caucasian French woman (Gioanni et al., Bull Cancer, 1991, 78(11):1053-62), BHT-101s originate from a 63-year old Caucasian Hungarian woman (Palyi et al., Virchows Arch B Cel Pathol. INcl. Mol. Pathol. 1993, 63(4): 263-9), and MB-1s originate from a 57-year old Caucasian Swiss man (Stenner et al., Cancer Sci. 2008, 99(9): 1847:52).
Table 3 show the known mutations most characteristic of these commercial lines (Cellosaurus, https://web.expasy.org/cellosaurus/).

**Table 3: Commercial cell lines used and their characteristics**

| **Line** | **Proven genetic status** | **Patient of origin** | **Pathology of origin** |
|---|---|---|---|
| **FTC-238** | TP53 R273H; TERT G228A Het | 42-year old Caucasian German man | FTC, lung metastasis |
| **BCPAP** | BRAF V600E Hom; TERT G228A Het | 76-year old Caucasian French woman | PDTC/ PTC |
| **8305C** | BRAF V600E Het; TP53 R273C; TERT G250A Het | 85-year old Asian Japanese woman | ATC |
| **CAL-62** | KRAS G12R; TP53 A161D; TERT promoter wt | 70-year old Caucasian French woman | ATC, right thyroid lobe |
| **BHT-101** | BRAF V600E Het; TERT G228A Het | 63-year old Caucasian Hungarian woman | ATC, lymphatic metastasis (prior PTC) |
| **MB-1** | wtBRAF; TP53 A161D; TERT promoter under study | 57-year old Caucasian Swiss man | ATC, retrosternal mass |

The lines were cultured with the h7H medium of the present invention even though they came from other types of medium because the present inventors consider that, on one hand, it is more suitable for functional studies as it closely resembles internal human medium, and on the other hand, there was a need to balance the growth conditions in commercial cell lines with the primary cultures of the present invention. The lines conserved certain expected characteristics such as, for example, giant cells in the anaplastic lines, a characteristic observed in the tissue samples from patients.

Transfection efficiency with the two best anaplastic line transfection methods which were standardised from a set of lipids or nucleofection to achieve maximum efficiency (data not shown). The lipids tested were Fugene 6, Fugene 6HD, K2, Turbofect, and Viafect. Only the latter two allowed the degree of efficiency required for the purposes of the present invention to be achieved.

### Effect of the suppression of PIAS2b with dsRNAi in thyroid cancer cell lines and primary cultures

A decision was made to start a study on the function of PIAS2 in human thyroid cells. First, PIAS2 RNA expression is measured for all its isoforms (global), and specifically for isoforms PIAS2a and PIAS2b, by means of quantitative TaqMan qRT-PCR, comparing lines with the primary culture of normal human thyroid T-NT2, which grows for many passages (Bravo *et al.,* 2012; Garcia-Rendueles et al., Oncogene, 2017, 36(5):652-666) (Figure 4-A). As can be observed, global expression does not always coincide with that of the protein isoforms. Except for MB-1 and 8305C which have both isoforms PIAS2b and PIAS2a in abundance, thyroid cancer lines express less PIAS2b and PIAS2a compared to the normal cell culture T-NT2. Generally, PIAS2a is expressed to a lesser extent compared to PIAS2b, a fact that coincides with the studies, characterising the antibodies shown in Figure 5, where a PIAS2a band was not detected in the expected weight.

The most direct way to learn about the function of a protein is to inhibit its expression and to observe whether there is an effect on proliferation or phenotype. To inhibit expression, interference with the mRNA (RNAi) is required, but in the case of PIAS2, it must be isoform specific, since it was already known that interference with PIAS2b would be performed given that it was differentially expressed in tissues (Figure 3). Since all commercial RNAi inhibited many PIAS2 isoforms, the inventors decide to make their own RNAi based on the essential data required for an efficient RNAi sequence: 21 nucleotides, starting with AA (ending with UU in the RNA, 3' UU hanging end), not having an excessively high % of GC, and not having more than three identical continuous nucleotides. As the different region of PIAS2b is the final region, the sequences of the present invention were concentrated in the 3' UTR, located in exon 14 of PIAS2b (Figure 4-B).

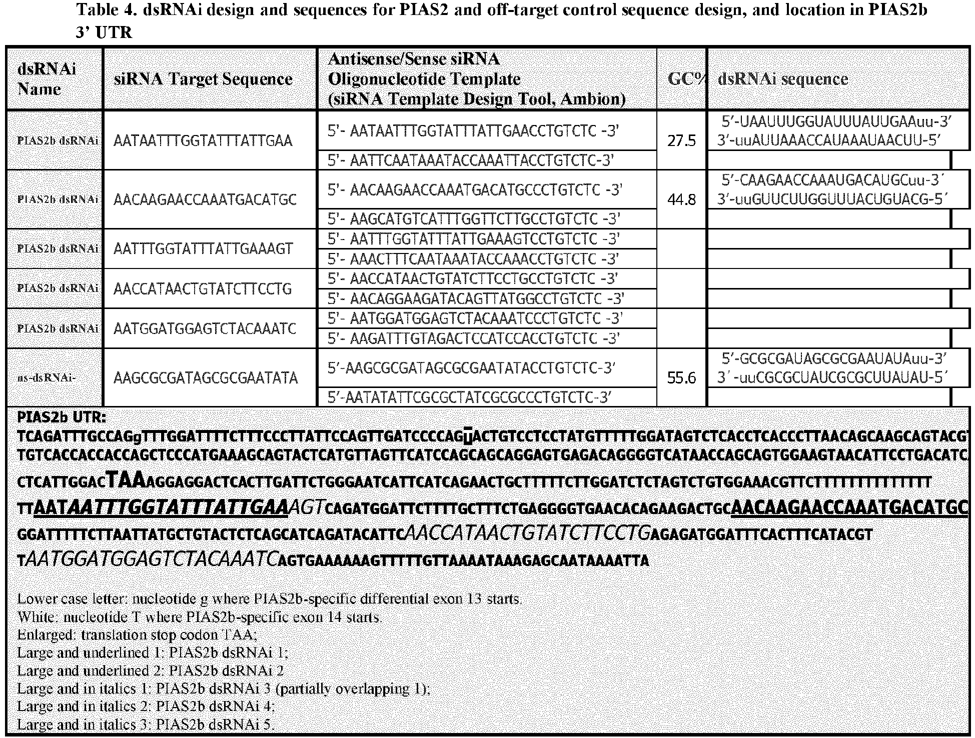

Ambion's siRNA Template Tool (http://www.ambion.com/techlib/misc/silencer_siRNA_template.html) is used to make the design. Its *in vitro* transcription kit is used to synthesise dsRNAi based on T7 polymerase. Five sequences with the conditions of the present invention were found in the web application (Table 4).

Sequences with 19 nucleotides were blasted in order to confirm that the only recognised transcript thereof with a total score >31 and an E-value <1 was PIAS2b (data not shown). As can be observed in Figure 5, the sequences only recognise PIAS2-204 (PIAS2b) and PIAS-202, one of the non-coding isoforms (only those used in the present invention are shown).

No other human mRNA surpassed the criteria of the present invention in any of the 5 sequences (data not shown). The control sequence ns-dsRNAi did not surpass any target with a total score >24 and an E-value <111.

The first two sequences PIAS2b dsRNAi 1 and 2 were empirically selected for transfection into cells on the basis that sequence 1 partially overlapped sequence 3, so the latter was ruled out; and sequences 4 and 5 were much more terminal and there could be a secondary mRNA chain structure at this level preventing the binding thereof (Table 4).

It was measured whether the transfection of the sequence PIAS2b dsRNAi 1 compared with the control sequence ns-dsRNAi suppressed the expression of PIAS2b or PIAS2a by means of TaqMan, but the number of undetectable values in the PIAS2b dsRNAi 1 samples was so numerous that it prevented calculating the statistics (data not shown). A Sybr-based quantitative qRT-PCR system which is less specific but more sensitive was then used (Figure 4-C). PIAS2b dsRNAi 1 efficiently, significantly, and specifically reduced the expression of PIAS2b mRNA, but not PIAS2a mRNA, in the five cell lines (Figure 4-C). The same result was obtained when measuring expression of the protein, both with mPIAS2 and with rPIAS2, by means of Western blot (Figure 4-D). When quantifying the Western blots, it was observed that p75 mPIAS2 was suppressed in all the cell lines after treatment with PIAS2 dsRNAi 1, although suppression was significantly lower in the BCPAP line compared to the other lines both one day and two days after the addition of dsRNAi.

Transfection efficiency was greater than 75% in FTC-238, BCPAP, 8305C, and CAL-62, and greater than 50% in BHT-101 and MB-1 (Figure 4-E). However, the functional effect of the dsRNAi of the present invention on cell growth varied depending on the sequence and on the origin of the lines.

In differentiated follicular carcinoma line FTC-238, no sequence had an effect (Figure 4-E). In poorly differentiated carcinoma line BCPAP, sequence 1 had no effect, but sequence 2 or the sum of 1+2 had a significant antiproliferative effect (Figure 4-F). In the four anaplastic carcinoma lines, 8305C, CAL-62, BHT-101, and MB-1, the two sequences had a pronounced antiproliferative effect (Figure 8-G).

Although there were hardly any differences in the pronounced antiproliferative effect between the three treatments in the anaplastic lines, the sequence which had the most potent, long-lasting, and consistent effect out of the four anaplastic lines was PIAS2b dsRNAi 1, so continuous characterisation of the effect thereof is performed in primary cultures from the TIROCHUS collection.

Primary cultures are kept in humanised conditions (h7H medium) and standardised in the same manner as the cell lines. Furthermore, when available, normal or benign tissue and cancerous tissue originating from the same patient are grown in parallel. Several examples of anaplastic carcinoma cases can be observed in Figure 6. The genetic profile of some cultures can be observed in Table 5, comparing the normal culture with the cancerous culture, and in turn with the original tissue thereof from which they were grown. It can be seen that the conservation of the genetic profile is absolute, although small, expected variations are observed in anaplastic cancers due to certain tumour heterogeneity in such an aggressive cancer. However, mass sequencing studies have found that in thyroid cancer, and the data probably refers to differentiated and poorly differentiated carcinoma, the mutation load per tumour (mutational burden) is not too high (Williams et al., Nat. Genet. 2016, 48(39): 238-244; Martincorena et al., Cell, 2018, 173(7): 1823). In a study which compares the global mutation load in cancers occurring in many organs, thyroid carcinoma was among the lowest with about 1.5 mutations/10⁻⁷ bases/division compared, for example, to lung carcinoma with almost 1 mutation/10⁻⁶ bases/division (Williams et al., Nat. Genet. 2016, 48(39: 238-244). In another subsequent study which studied substitutions exclusively, it was found that thyroid cancer has <1 substitution/tumour in "driver" oncogenes compared, for example, to colon or endometrial cancer with 4 substitutions/tumour in "driver" oncogenes, and focusing exclusively on the coding region, thyroid cancer exhibited <1 coding substitution/tumour in "driver" oncogenes compared to >10 coding substitutions/tumour in "driver" oncogenes in colon carcinoma, endometrial carcinoma, or melanoma (Martincorena et al., Cell, 2018, 173(7):1823).

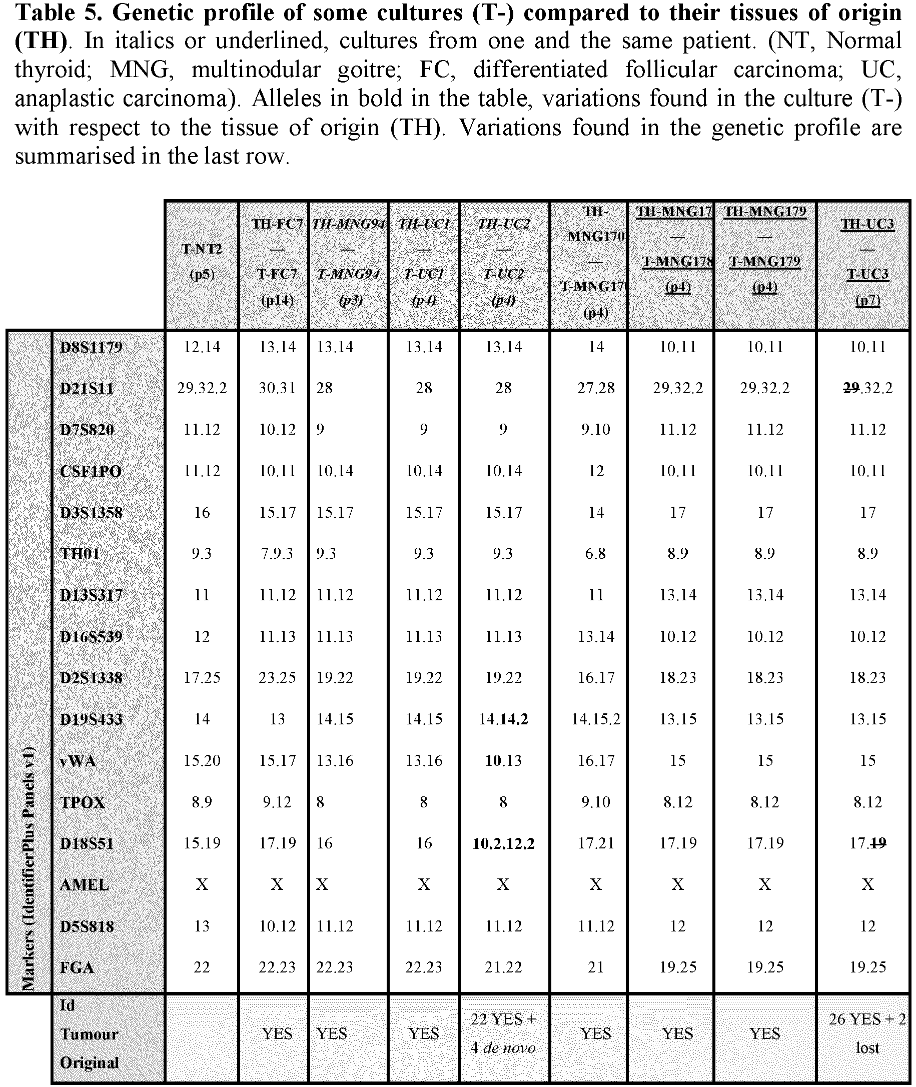

As performed previously with the cell lines, RNA expression of global PIAS2, and of isoforms PIAS2b and PIAS2a, was first evaluated in cultures of different origin which were cultured at that time (Figure 7-A). This measurement indicated that several coding and non-coding isoforms should be expressed since global PIAS2 does not coincide with the specific TaqMan for each of the coding isoforms. Again, it was observed that more PIAS2b is expressed compared to PIAS2a, but anaplastic cultures express less PIAS2 compared to cultures of other origins such as a normal tissue (T-NT), multinodular goitre (T-MNG), or differentiated papillary carcinoma (T-PC). To confirm this datum, measurements were performed on groups of established cultures of one and the same tissue origin such as normal thyroid tissue (T-NT, n=15), multinodular goitre (T-MNG, n=27), papillary carcinoma (T-PC, n=24), and anaplastic carcinoma (T-UC, n=3 with replicates in different passages up to 24 samples). The group of anaplastic cultures exhibited significantly lower global expression of PIAS2, and of PIAS2b and PIAS2a, compared to the other two groups (Figure 7-B). The group of T-PC exhibited significantly higher PIAS2b (Figure 7-B).

The effect of the sequence of the present invention was thentested. PIAS2b dsRNAi 1 transfection was capable of effectively suppressing the expression of the protein in cultures of different pathological origin (normal thyroid tissue, multinodular goitre, differentiated papillary carcinoma, and anaplastic carcinoma), whereas the control sequence nd-dsRNAi did not exhibit any effect (Figures 7-C-F). When verification was performed at the mRNA level, PIAS2b dsRNAi 1-induced suppression was specific for isoform beta, without altering PIAS2a (Figure 7-G).

The biological effect on culture growth was measured six days after transfection, controlling the % of transfected cells such that it was greater than 50% in all the cases, and close to 100% in some cases (Figure 11-H). It could be observed that there was no effect or differences between PIAS2b dsRNAi 1 and nd-dsRNAi in cultures originating from normal thyroid (T-NT), autoimmune Graves-Basedow hyperplasia (T-GD), multinodular goitre (T-MNG), differentiated papillary carcinoma (T-PC) even though it was metastatic (T-M), differentiated follicular carcinoma (T-FC) (Figure 7-H). However, in the three anaplastic cultures established from independent surgical fragments of two affected patients, PIAS2b dsRNAi 1 exhibited a significant mass effect, inhibiting cell growth by more than 90% six days after transfection (Figure 7-H). The experiment was repeated with some cultures with measurements being taken over days after transfection, and it could be verified that cells transfected with ns-dsRNAi grew over days, as did those transfected with PIAS2b dsRNAi 1 in T-NT2 or T-MNG94, but not the three anaplastic cultures T-UC1, T-UC2, and T-UC3 the number of cells of which started to decrease from day 1 (Figures 7-1-J).

One of the questions the present inventors asked themselves was how long the effect of a single PIAS2 dsRNAi 1 transfection on an anaplastic cell line would last and when anaplastic cells would start to recover taking into account that these anaplastic cells proliferate in a quick and uncontrolled manner. An experiment is performed in CAL-62, comparing daily cell growth (data not shown). The effect lasts for days, although a slow growth recovery is observed from day 5. This suggests that it would be possible to control these cells by treating the cells once a week, as performed in several chemotherapies.

In order to learn about what happened to cells treated with PIAS2b dsRNAi, video-microscopy experiments were performed in which cells were observed for several days growing under controlled humidity and temperature conditions and in the presence of a small amount of a non-toxic colorant, SiRDNA, which becomes more intense every time a cell duplicates or a DNA condenses during mitosis, to reduce the intensity thereof after mitosis. Each cell can thus be individually tracked. Figure 8 shows the results. In anaplastic cells from patients T-UC1, T-UC2, and cell lines 8305C and CAL-62, the PIAS2 dsRNAi 1 sequence is cytotoxic because it causes mitotic catastrophe or death during mitosis (Figures 8A-B-C-D, graphs in the top panels, black bars). In contrast, cells treated with the control sequence ns-dsRNAi successfully divided without any problem (Figures 8A-B-C-D, graphs in the top panels, white bars). When the cells were analysed, it could be demonstrated that the cells died at the start of mitosis, just as they entered prophase (Figures 8A-B-C-D, graphs in the bottom panels). Similar results were obtained in the BCPAP line but with the PIAS2 dsRNAi 2 sequence (Figure 8-E).

The conclusion reached by the present inventors is that the expression of PIAS2 in anaplastic thyroid cells is highly restricted and the reduction (dsRNAi) or increase (overexpression) in PIAS2 causes specific mitotic death in cancer cells of this type without affecting differentiated cancer cells, benign proliferation, or normal thyroid.

### Effectiveness of other commercial methods for suppressing the expression of PIAS2b

The isoform-specific PIAS2 dsRNAi sequence of the present invention exhibited a potent, anaplastic cell-specific anti-cancer effect. The next step was to consider whether it was important for the sequence to be directed to isoform PIAS2b and whether the form in which it was produced, such as double-stranded RNAi transcribed *in vitro,* was important. Furthermore, this system is hard to produce at a large scale.

Other commercial systems which were technically compatible and functional in very fast growing cells such as anaplastic cells were sought. After evaluating several systems, suppression of PIAS2b after viral infection and induced expression of a shRNA were selected.

The TRIPZ-human shRNA inducible system (Dharmacon, see bibliography) allows the generation of lentiviruses which infect cells. After the administration of a dose of doxycycline compatible with cell growth, the expression of shRNA (*Tet-on*) is induced at the same time at which the expression of the red fluorescent protein (RFP) is induced. The system is based on a lentiviral vector containing a "tetracycline"-regulated promoter (*Tre promoter*) which directs the expression of fusion transcript RFP-shRNA, with the shRNA acting as a 3'UTR (Naughton et al., 2011, Anal. Biochem. 417(1):162-4). The vector has two BspQ1 sites between the 5'mir30 region and the 3'mir30 region into which double-stranded oligonucleotides with shRNA sequences are cloned with relative ease. The vector has a second ubiquitin C promoter (*UBC promoter*) which directs the expression of doxycycline/tetracycline-regulated transactivator rtTA3 associated with IRES and puromycin resistance gene (*puromycin N-acetyl-transferase, PAC*).

The two plasmids T-PIAS2_shRNA and T-Scr_shRNA containing the recommended off-target control sequence *Scramble (*Scr, *off-target*) were then purchased. By means of complementary Addgene vectors, viral particles having a high titre, measured by means of quantitative PCR and functional assays in HEK293T, were prepared in the laboratory of the present inventors. Meanwhile, by means of a dose-response experiment with doxycycline in anaplastic thyroid cancer cells of the present invention (CAL-62), concentrations that would not affect cell growth (1 and 2 mg/ml) were sought.

In a first experiment, the CAL-62 cells are infected and selected with puromycin. Resistant clones were mixed in populations which, in the next passage, were compared in the absence/presence of doxycycline. Unfortunately, variability in the expression of RFP was very high with very intense cells and absolutely negative cells, despite selection with puromycin.

A decision to use sorter selection after flow cytometry was then made. After adding doxycycline (or not for fluorescence controls) overnight, two populations having a high RFP intensity (T-PIAS2_shRNA_{HI} and a second population with an even higher intensity T-PIAS2_shRNA_{SU}) were selected the next day. The same was done with the control population T-Scr_shRNA; however, no subsequent difference whatsoever was obtained between them in terms of growth over time, so only one was used for the experiments (data not shown).

Figure 9 shows the results obtained from the subsequent experiments with these populations. The addition of doxycycline had a significant inhibitory effect on the growth of the two populations expressing PIAS2_shRNA, but not on those expressing Scr_shRNA (Figure 9-A). The maximum effect is 75% inhibition after 7 days with the maximum dose of doxycycline.

The PIAS2_shRNA sequence is directed to the coding portion common to all the protein isoforms of the PIAS2 RNA; in a search performed in BLAST, it would be capable of interacting with 5 of the 16 PIAS2 isoforms, all of them being coding isoforms PIAS2-204 (PIAS2b), PIAS2-201 (PIAS2a), PIAS2-215 (PIAS-NY), PIAS2-203 (fragment of 282 aa), and PIAS2-202 (fragment of 182, *nonsense-mediating decay*). In fact, by measuring the expression of isoform-specific RNA by means of TaqMan qRT-PCR it can be seen that doxycycline suppresses the expression of PIAS2b and PIAS2a in the population T-PIAS2_{HI}_sh, but not in the population T-Scr_sh (Fig. 9-B). By means of Western blot, it was observed that the PIAS2b protein was suppressed both with the mPIAS2 antibody and with rPIAS2 (Fig. 9-C).

The inventors wanted to observe them in experiments that run through a longer period of time, since it is assumed that the population is continuously expressing the shRNA which interferes with PIAS2. The effect is maintained for 7 days after the addition of doxycycline, however, the effect is lost after 9 days (Fig. 9-D).

Upon observing that PIAS2b dsRNAi 1 was the most efficient form for suppressing PIAS2b with a significant functional anti-proliferative effect, the rest of the studies continued with this formulation.

### PIAS2 dsRNAi 1 exhibits anti-tumour effect on oPDX, an in vivo preclinical model

dsRNAi PIAS2 exhibits a specific anti-tumour behaviour in anaplastic thyroid cells *in vitro.* A decision was then made so as to develop a clinically relevant *in vivo* model.

Anaplastic cancer cells are poorly infected with virus following conventional protocols. A viral vector with the double expression of the luciferase enzyme and mCherry separated by an IRES (*Internal Ribosomic Entry Site*) element which ensures that about one luciferase molecule will be transcribed for each mCherry molecule was developed. Viral infection of the population can therefore be evaluated in a quick manner by means of conventional fluorescence microscopy and, in turn, subsequent quantification of the tumour mass by means of luminescence ensured.

Figure 10 summarises the development of the model. With the most efficient infection protocol, most of the cells express the fluorescent protein mCherry vs. those uninfected cells (Fig. 10-A). These very cells emit light after the addition of luciferin since they express the luciferase protein combined in the same virus and can be observed in the IVIS (Fig. 10-B) and subsequently quantified (Fig. 10-C).

The cells are carefully introduced parallel to the trachea below the laryngeal muscles in female NOD-SCID mice by means of surgery. The mice are allowed to recover for at least two weeks. After said period, tumour growth evaluation in the IVIS starts following quick gas anaesthesia and *i.p.* luciferin injection (Fig. 10-D). It can be observed that the model is an orthotopically located reproducible model which acquires the maximum measurement point between 20-30 minutes after injection of the luciferin substrate.

Once the animal model was validated, the *in vivo* anti-cancer action of PIAS2 dsRNAi 1 was studied. To that end, 10⁶ cells from the anaplastic primary culture T-UC1 infected with the MI-luciferase-IRES-mcherry virus were injected into 9 female NOD-SCID mice, generating patient-derived orthotopic xenografts (oPDX).

Tumour growth was tracked once a week by measuring bioluminescence using the IVIS apparatus (Perkin Elmer). The tumours were left to grow for up to 5 weeks when the mice exhibited large masses in the neck and treatment was started at that time. The mice were randomised to be treated by systematic route 2 times a week for 3 weeks with 40 uM dsRNAi (ns-dsRNAi or PIAS2b dsRNAi 1) mixed with the vehicle Atelocollagen (Azuma K et al., Biochem. BIophys. Res. Commun. 2010, 391(1): 1075-9; Sasaki T et al., Biochem. Biophys. Res. Commun. 2010, 399(1):79-83) (AteloGene koken, Cosmobio). (Figure 11-A).

This type of vehicle allows dsRNAi to be stable *in vivo.* The complexes that they form with dsRNAi protect nucleic acids from degradation by nucleases. Four mice were treated with the PIAS2b dsRNAi and Atelocollagen mixture and 5 mice with the ns-dsRNAi and atelocollagen mixture. Once the treatment was finished, it was waited for a week and the mice were sacrificed and samples collected (Fig. 11-A).

The individual bioluminescence evolution curves obtained on a weekly basis by means of IVIS were fitted to 100% on week 5 at which the treatment started. The statistical analysis of the data by ANOVA with multiple measurements shows a significant difference at the end of treatment (p=0.01) (Fig. 11-B).

The RECIST criteria were used for final response evaluation (PD= Progressive Disease; SD= Stable Disease; PR= Partial response) (Fig. 11-B). While tumours continued to grow progressively (PD) in the control group, ns-dsRNAi, in the group treated with PIAS2b dsRNAi, 3 mice were capable of stabilising their tumour growth (SD) and the fourth mouse was able to partially reduce tumour volume (PR) (Fig. 11-B). Figure 11-C shows two cases, one treated with ns-dsRNAi in which the signal increases over time, and one treated with PIAS2 dsRNAi 1 in which it is observed that the signal decreases with treatment.

Non-linear regression group curves have also been obtained for ns-dsRNAi and PIAS2b dsRNAi using the LOWESS regression method (Figure 11-B, thick lines). The LOWESS curves mathematically analyse the trend in data groups. It can be seen how all the tumours have grown in the samemanner up to week 5 (thick grey and black lines are not separated), but after treatment, the two curves start to move away from each other, with the grey line (ns-dsRNAi) increasing in intensity and the black line (PIAS2 dsRNAi 1) stabilising and being increasingly separated from the grey line.

By depicting the initial intensity before treatment and the final one, at the end of treatment, in an initial-final data analysis (Mann-Whitney test), there is a statistically significant difference between the control group of mice ns-dsRNAi and PIAS2b dsRNAi 1 (Fig. 11-D).

Autopsy and tumour extirpation are performed at the end point. A first *in situ* assessment is performed to determine whether the tumour growth affects other regions. An aggressive local growth which invades and destructs anatomical structures of the neck representative of human anaplastic carcinomas is observed in control mice. An *ex vivo* examination of the dimensions of the tumour is performed, in which differences are observed with the naked eye. It is observed that the tumours of the control animals, ns-dsRNAi, are larger compared to mice treated with PIAS2 dsRNAi 1 (Fig. 11-E). A macroscopic examination of the organs most susceptible to metastasis such as lungs, brain, heart, and liver, is also performed. No apparent metastases were seen in any of the groups.

The tumours were fixed and sectioned for subsequent histological analysis. Their three-dimensional volume was calculated from the sections. Tumours from mice treated with ns-dsRNAi are larger compared to mice treated with PIAS2b dsRNAi 1, although the differences in tumour volumes do not reach statistically significant difference (p=0.1) (Fig. 11-F).

In the histological analysis by means of haematoxylin-eosin, human tumour invasion of the normal thyroid of the mouse, both as vascular and as muscle invasion, reproducing the characteristics of human anaplastic thyroid carcinomas, is observed (Fig. 11-G). Histological characteristics have also been compared by means of immunohistochemistry using clinically validated antibodies for the diagnosis of anaplastic thyroid carcinomas. The tumours show a positivity for global cytokeratins (A1A3) and CK8/CK18, characteristic of anaplastic carcinoma. The carcinoma is negative for thyroid markers (thyroglobulin, TG; NKX2.1, TTF1) but highly positive for Ki67, p53, and PAX8 (Fig. 11-H). The tumour pathology showed similarities with the characteristics of human anaplastic carcinomas.

In Figure 11-I, carcinomas treated with ns-dsRNAi and PIAS2b dsRNAi 1 were compared by counting the carcinoma cells with signs of death, similar to mitotic catastrophe: cells with a bright pink background and condensed haematoxylin (black dots and arrows) compared to total cells (grey dots). Significant differences were verified in carcinomas treated with PIAS2b dsRNAi 1, which exhibit more dead cells.

### Effect of the suppression of PIAS2b with dsRNAi in cell lines and primary cultures of cancers other than thyroid cancer

Lastly, the effectiveness of PIAS2b dsRNAi 1 as a growth inhibitor for other types of cancer not originating from the thyroid gland was tested. To that end, new experiments were prepared.

This was based on the hypothesis that there are three characteristics of anaplastic thyroid carcinomas that are absent in differentiated or poorly differentiated thyroid carcinomas and these characteristics are: 1) undifferentiation, shown by the absence of the expression of proteins or other phenotype markers characteristic of that tissue; 2) severe cell atypia with characteristic microscopic (pleomorphic) events referred to as "anaplasia", and 3) genetic alterations known as "aneuploidy" (containing >2n) in all the cells, with some exhibiting polyploidy (n^{∗}2n) in "anaplastic giant" cells. Both characteristics may or may not be associated.

There are other non-thyroid human cancers which exhibit these three characteristics at the clinical pathology level. Similar to (undifferentiated) anaplastic thyroid carcinoma, there are other undifferentiated, non-neuroendocrine epithelial neoplasias with a high degree of malignancy and an anaplastic (pleomorphic) morphology in different anatomical locations and they tend to have in common: a high average age of manifestation (50-60 years old), highly aggressive clinical course, sarcomatoid (mesenchymal-like) appearance, coexistence with residual areas of well-differentiated carcinoma, and alterations in TP53 coexisting with molecular alterations in other genes. They include undifferentiated lung carcinoma (also referred to as large-cell, pleomorphic, fusocellular, or giant-cell carcinoma), undifferentiated carcinoma of the pancreas, stomach, liver, ovary, uterus, or prostate.

For these studies, an *in silico* search was first performed for commercial cell lines originating from undifferentiated carcinoma of the type sought (non-thyroid) but conserving the same pathological characteristics of anaplastic thyroid carcinoma and maintaining said characteristics in the line cultured from the tissue of the initial patient. Three lines were selected (see Table 6): PANC-1 derived from undifferentiated/ carcinoma anaplastic of the pancreas, COR-L23 derived from undifferentiated/anaplastic large- or giant-cell lung carcinoma, HGC-27 derived from undifferentiated gastric carcinoma. Like anaplastic thyroid cells, after being established, these lines were described with a highly altered karyotype with respect to the 46 chromosomes of the cells.

The next step was the standardisation of dsRNAi transfection to achieve the maximum transfection efficiency exactly as it was done for all anaplastic thyroid carcinoma cells. Two types of lipid complexes, found most frequently to be effective in cell lines, from two different companies (Viafect, Promega; and Turbofect, Thermo) were tested, seeking at least > than 70% of cells transfected with control ns-dsRNAi mixed with a GFP-expressing plasmid, which allows counting cells with green fluorescence and calculating the percentage of transfection.

The most effective reagent was Turbofect for all the lines (Table 6). Once standardised, the action of dsRNAi on cell growth in these lines was compared. Transfection of ns-dsRNAi was compared to transfection of PIAS2-dsRNAi 1. Figure 12 shows how PIAS2-dsRNAi 1 exhibits a specific inhibitory effect on the growth of three undifferentiated/anaplastic cancer lines tested. The maximum effect reached is 62% growth inhibition in undifferentiated pancreatic carcinoma line PANC-1 (Figure 12 A), 80% in undifferentiated lung carcinoma line COR-L23 (Figure 12 B), and 97%% in gastric carcinoma line HGC-23 (Figure 12 C).

**Table 6: Human non-thyroid cancer lines selected due to their cancer origins similar to anaplastic thyroid carcinoma**

| **Line** | **Organ of origin** | **Type of cancer and/or pathological diagnosis** | **Original patient** | **Known mutations** | **Reference** | **Transfection efficiency (Turbofect)** |
|---|---|---|---|---|---|---|
| PANC-1 | Pancreas (Pancreas head) | Undifferentiated/an aplastic pancreatic ductal adenocarcinoma | 56-year old Caucasian man | CDKN2A deletion, homozygous KRAS G12D, heterozygou s TP53 R273H, homozygous | Lieber M.M., et al., & Todaro G.J. Establishment of a continuous tumour-cell line (PANC-1) from a human carcinoma of the exocrine pancreas. Int. J. Cancer 15:741-747(1975) | 74% |
| COR-L23 | Lung (Pleural Effusion) | Undifferentiated (large-cell)/anaplastic lung carcinoma (NSCLC, giant-cell anaplastic lung carcinoma) | 62-year old Caucasian man | KRAS G12V, homozygous | Baillie-Johnson H., et al., & Bleehen N.M. Establishment and characterisation of cell lines from patients with lung cancer (predominantly small cell carcinoma). Br. J. Cancer 52:495-504(1985) | 73% |
| HGC-27 | Stomach (metastatic lymph node from a patient with a gastric cancer) | Undifferentiated gastric carcinoma | Asian, sex and age unknown (suspected to be a woman) | ATR G2316fs*9, heterozygouu us PIK3CA E542L, heterozygou s TP53 W153fs*28 | Akagi T., Kimoto T. Human cell line (HGC-27) derived from the metastatic lymph node of gastric cancer. Acta Med. Okayama 30:215-219 (1976) | 80% |

## Claims

1. A nucleic acid comprising an antisense nucleotide sequence complementary to a target region of the PIAS2β mRNA, wherein the binding of the nucleic acid to its target sequence causes inhibition of the expression of the PIAS2β mRNA.

2. The nucleic acid according to claim 1, wherein the target sequence is located in exon 14 of the PIAS2β mRNA.

3. The nucleic acid according to claim 1, wherein the target sequence is selected from the group consisting of sequences SEQ ID NO. 1-5 and functionally equivalent sequences.

4. The nucleic acid according to any of claims 1-3, wherein the nucleic acid is selected from the group consisting of a double-stranded RNA interference nucleic acid, an antisense oligonucleotide, a gapmer, a PNA, a LNA, an INA, a HNA, a morpholino, a ribozyme, and an aptamer.

5. The nucleic acid according to claim 4, wherein the nucleic acid is a double-stranded RNA interference nucleic acid selected from the group consisting of small interfering RNA (siRNA), short hairpin RNA (shRNA), and a microRNA (miRNA).

6. The nucleic acid according to claim 5, wherein the nucleic acid is a small interfering RNA.

7. The nucleic acid according to any of claims 1-6, wherein the antisense nucleotide sequence is selected from the group consisting of SEQ ID NO. 6-10 and functionally equivalent sequences.

8. The nucleic acid according to claim 7, wherein the antisense nucleotide sequence is selected from the group consisting of SEQ ID NO. 6, SEQ ID NO. 7, and functionally equivalent sequences.

9. The nucleic acid according to claim 8, wherein the antisense nucleotide sequence is SEQ ID NO. 6 or a functionally equivalent sequence.

10. The nucleic acid according to any of claims 1-9, wherein the nucleic acid is a double-stranded RNA interference nucleic acid and further comprises a sense nucleotide sequence complementary to the antisense sequence.

11. The nucleic acid according to claim 10, wherein the antisense nucleotide sequence is selected from the group consisting of SEQ ID NO. 6-10 and functionally equivalent sequences, and the sense sequence is selected from the group consisting of SEQ ID NO. 11-15 and functionally equivalent sequences.

12. The nucleic acid according to claim 11, wherein the antisense nucleotide sequence is selected from the group consisting of SEQ ID NO. 6, SEQ ID NO. 7, and functionally equivalent sequences, and the sense nucleotide sequence is selected from the group consisting of SEQ ID NO. 11, SEQ ID NO. 12, and functionally equivalent sequences.

13. The nucleic acid according to claim 12, wherein the antisense nucleotide sequence is SEQ ID NO. 6 or a functionally equivalent sequence, and the sense nucleotide sequence is SEQ ID NO. 11 or a functionally equivalent sequence.

14. The nucleic acid according to any of claims 1-13, wherein the antisense nucleotide sequence comprises a sequence of at least two uracils at the 3' end.

15. The nucleic acid according to any of claims 1-13, wherein the sense nucleotide sequence comprises a sequence of at least two uracils at the 3' end.

16. The nucleic acid according to claim 13, wherein the sense and antisense sequences comprise a sequence of at least two uracils at their 3' ends.

17. A vector comprising a nucleic acid encoding the antisense sequence of the nucleic acid of any of claims 1-14.

18. The vector according to claim 17, comprising a nucleic acid encoding the sense sequence of the nucleic acid of any of claims 1-16.

19. A host cell comprising the nucleic acid according to any of claims 1-16 or the vector according to any of claims 17-18.

20. A pharmaceutical composition comprising the nucleic acid according to any of claims 1-16 or the vector according to any of claims 17-18, and a pharmaceutically acceptable excipient.

21. An inhibitor of the PIAS2β protein for use in the treatment of cancer in a patient.

22. The inhibitor for use according to claim 21, wherein the cancer is an anaplastic carcinoma or a cancer having a high degree of aneuploidy.

23. The inhibitor for use according to claim 22, wherein the anaplastic carcinoma is selected from the group consisting of anaplastic or undifferentiated pancreatic ductal adenocarcinoma, anaplastic large-cell lung carcinoma or anaplastic giant-cell lung carcinoma, and undifferentiated gastric carcinoma

24. The inhibitor for use according to claim 21 or 22, wherein the anaplastic carcinoma or cancer is selected from the group consisting of anaplastic carcinoma of the thyroid, poorly differentiated carcinoma of the thyroid, or recurring undifferentiated carcinoma of the thyroid.

25. The inhibitor for use according to claim 24, wherein the cancer is an anaplastic carcinoma of the thyroid.

26. The inhibitor for use according to any of claims 21-25, wherein the inhibitor is a nucleic acid according to any of claims 1-16, a vector according to any of claims 17-18, or combinations thereof.

27. The inhibitor for use according to claim 26, wherein the inhibitor is a nucleic acid according to any of claims 1-16 or combinations thereof.

28. The inhibitor for use according to claim 27, wherein the inhibitor is the nucleic acid according to any of claims 12-16 or combinations thereof.

29. The inhibitor for use according to claim 28, wherein the inhibitor is the nucleic acid of claim 16.

30. The pharmaceutical composition according to claim 20 for use in the treatment of cancer in a patient, selected from the group consisting of anaplastic carcinoma of the thyroid, poorly differentiated carcinoma of the thyroid, or recurring undifferentiated carcinoma of the thyroid.

31. The inhibitor for use according to claims 21-29 or the pharmaceutical composition for use according to claim 30, wherein the inhibitor or the pharmaceutical composition is administered to the patient at least two times a week.

32. The inhibitor for use according to claims 21-29 or the pharmaceutical composition for use according to claim 30, wherein the inhibitor or the pharmaceutical composition is administered to the patient intravenously.

33. A polymerase chain reaction (PCR) amplification method for detecting, in a differential manner, the nucleic acid sequence of isoform β or of isoform α of the PIAS2 gene in a sample, said process comprises:
(a) providing the PCR assay containing said sample with a labelled oligonucleotide containing a sequence complementary to a region of the target nucleic acid, wherein said labelled oligonucleotide hybridises with the target nucleic acid sequence, binding thereto through the primers of step (b), and wherein said labelled oligonucleotide has the sequence SEQ ID NO: 87 when the detection of the nucleic acid sequence of isoform α of the PIAS2 gene is desired or SEQ ID NO: 88 when the detection of the nucleic acid sequence of isoform β of the PIAS2 gene is desired;
(b) providing a pair of primers, wherein the first primer contains a sequence complementary to a region of a chain of the target nucleic acid sequence and initiates the synthesis of an extension product and a second primer containing a sequence complementary to the other chain of the target nucleic acid sequence or complementary to a region in the extension product of the first primer and initiates the synthesis of a complementary DNA chain; and wherein each primer is selected so that it hybridises with its complementary template upstream of any labelled oligonucleotide hybridised with the same nucleic acid chain, wherein the pair of primers is formed by the primers of sequence SEQ ID NO: 89 and SEQ ID NO: 90 when the detection of the nucleic acid sequence of isoform α of the PIAS2 gene is desired or by the primers of sequence SEQ ID NO: 91 and SEQ ID NO: 92 when the detection of the nucleic acid sequence of isoform β of the PIAS2 gene is desired,
(c) amplifying the target nucleic acid sequence using a nucleic acid polymerase with 5' to 3' nuclease activity as a template-dependent polymerising agent under conditions permissive for the PCR cycling steps of (i) hybridising the primers and the labelled oligonucleotide with a template nucleic acid sequence contained within the target sequence, and (ii) extending the primer, wherein said nucleic acid polymerase synthesises a primer extension product while the 5' to 3' nuclease activity of the nucleic acid polymerase simultaneously releases labelled fragments of the hybridised double chains comprising labelled oligonucleotides and their complementary template nucleic acid sequences, thereby creating detectable labelled fragments, and
(d) detecting and/or measuring the signal generated by the hydrolysis of the labelled oligonucleotide for determining the presence or absence of the target sequence in the sample.

34. The method according to claim 33, wherein the labelled oligonucleotide comprises a pair of interactive signal-generating markers suitably positioned in the oligonucleotide to shield the generation of a detectable signal, said markers being separated by a site within the oligonucleotide which is susceptible to cleavage by nuclease, thereby allowing, during primer extension, the 5' to 3' nuclease activity of the nucleic acid polymerase to separate the first interactive signal-generating marker from the second interactive signal-generating marker by cleaving at the susceptible site, thereby producing a detectable signal.

35. The method according to claim 34, wherein said first marker is a chemiluminescent substrate and said second substrate is a fluorophore which interacts with the same.

36. A kit for the differential detection of the nucleic acid sequence of isoform β or of isoform α of the PIAS2 gene in a sample, comprising:
(a) at least one labelled oligonucleotide containing a sequence complementary to a region of the target nucleic acid, wherein said labelled oligonucleotide has the sequence SEQ ID NO: 87 when the detection of the nucleic acid sequence of isoform α of the PIAS2 gene is desired or SEQ ID NO: 88 when the detection of the nucleic acid sequence of isoform β of the PIAS2 gene is desired, and wherein the 3' end of the labelled oligonucleotide is blocked to prevent extension by a nucleic acid polymerase having 5' to 3' activity and the labelled oligonucleotide contains first and second markers, the first marker being separated from the second marker by a site susceptible to cleavage by a nuclease, and wherein the markers of the labelled oligonucleotide constitute a pair of interactive signal-generating markers located on the oligonucleotide to shield the generation of a detectable signal, and
(b) at least one pair of primers, formed by the primers of sequence SEQ ID NO: 89 and SEQ ID NO: 90 when the detection of the nucleic acid sequence of isoform α of the PIAS2 gene is desired or by the primers of sequence SEQ ID NO: 91 and SEQ ID NO: 92 when the detection of the nucleic acid sequence of isoform β of the PIAS2 gene is desired.

37. The kit according to claim 36, further comprising a nucleic acid polymerase with 5' to 3' nuclease activity.

38. The kit according to claim 37, wherein said nucleic acid polymerase is a thermostable enzyme, preferably a DNA polymerase from the *Thermus* species.
